# EUROPEAN PATENT SPECIFICATION

(11) **EP 1 912 636 B1**
(45) Date of publication and mention of the grant of the patent: **25.06.2014**
(21) Application number: 06788078.1
(22) Date of filing: 21.07.2006
(51) Int. Cl.: A61K 31/44, A61K 31/195, C07D 207/36, C07D 231/18, C07D 233/84, C07D 261/10, C07D 277/36, C07D 277/54, C07D 307/64, C07D 333/34, C07D 333/38, C07D 417/04, C07C 311/08, C07C 311/09, C07C 311/10

(54) **N-(ARYLAMINO)-SULFONAMIDE INHIBITORS OF MEK**
N-(ARYLAMINO)-SULFONAMID-MEK-INHIBITOREN
INHIBITEURS N-(ARYLAMINO)-SULFONAMIDE DE MEK

(30) Priority: 21.07.2005 US 701814 P; 08.08.2005 US 706719 P; 28.10.2005 US 731633 P
(43) Date of publication of application: 23.04.2008
(73) Proprietor: Ardea Biosciences, Inc., Costa Mesa, CA 92626 (US)
(72) Inventor: MADERNA, Andreas, Stony Point, NY 10980 (US); VERNIER, Jean-Michel, Laguna Niguel, CA 92677 (US); BARAWKAR, Dinesh A., Dhanakawadi Pune, MAHARASTRA 411043 (IN); CHAMAKURA, Varaprasad, Irvine, CA 92614 (US); ABDELLAOUI, Hassan, El, Jamestown, NC 27282 (US); HONG, Zhi, Chapel Hill, NC 27517 (US)
(74) Representative: Williams, Paul Howard
(86) International application number: PCT/US2006/028326
(87) International publication number: WO 2007/014011

(56) References cited:
- WO-A-2005/121142
- US-A1- 2003 092 748
- US-A1- 2004 171 632
- DATABASE WPI Thomson Scientific, London, GB; AN 2004-718750 XP002549056 "New sulfamide derivatives or their salts for preventing and treating cancer, transplant rejection, osteoarthritis, cystic fibrosis, diabetes, hepatomegaly, seizure, cardiac failure, asthma and Alzheimer's disease" & WO 2004/083167 A (SANKYO CO [JP] SHIBATA TOMOYUKI [JP]; IWADARE HAYATO [JP]; KIGA MASAK) 30 September 2004 (2004-09-30)
- LEE J.T. ET AL: 'The Raf/MEK/ERK Signal Transduction Cascade as a Target for Chemotherapeutic Intervention in Leukemia.' LEUKEMIA vol. 16, no. 4, April 2002, pages 486 - 507, XP008124990
- COBB M.H. ET AL: 'How MAP Kinases are Regulated.' JOURNAL OF BIOLOGICAL CHEMISTRY vol. 270, no. 25, 23 June 1995, pages 14843 - 14846, XP008124980

## Description

### Cross Reference to Related Applications

This application claims priority to U.S. Provisional Application Ser. No. 601701,814, filed July 21, 2005; to U.S. Provisional Application Ser. No. 60/706,719, filed August 8, 2005; and to U.S. Provisional Application Ser. No. 60/731,633, filed October 28, 2005.

### Field of the Invention

This invention concerns N-(2-arylamino) aryl sulfonamides, which are inhibitors of MEK. Such compounds are useful in the treatment of cancer and other hyperproliferative diseases.

### Background of the invention

Oncogenes - genes that contribute to the production of cancers -- are generally mutated forms of certain normal cellular genes ("proto-oncogenes"). Oncogenes often encode abnormal versions of signal pathway components, such as receptor tyrosine kinases, serine-threonine kinases, or downstream signaling molecules. The central downstream signaling molecules are the Ras proteins, which are anchored on the inner surfaces of cytoplasmic membranes, and which hydrolyze bound guanosine triphosphate (GTP) to guanosine diphosphate (GDP). When activated by a growth factor, growth factor receptors initiate a chain of reactions that leads to the activation of guanine nucleotide exchange activity on Ras. Ras alternates between an active "on" state with a bound GTP (hereafter "Ras.GTP") and an inactive "off" state with a bound GDP. The active "on" state, Ras.GTP, binds to and activates proteins that control the growth and differentiation of cells.

For example, in the "*m*itogen-*a*ctivated *p*rotein kinase (MAP kinase) cascade," Ras.GTP leads to the activation of a cascade of serine/threonine kinases. One of several groups of kinases known to require a Ras.GTP for their own activation is the Raf family. The Raf proteins activate "MEK1" and "MEK2," abbreviations for *m*itogen-activated *E*RK-activating *k*inases (where ERK is extracellular signal-regulated protein kinase, another designation for MAPK). MEK1 and MEK2 are dual-function serine/threonine and tyrosine protein kinases and are also known as MAP kinase kinases. Thus, Ras.GTP activates Raf, which activates MEK1 and MEK2, which activate MAP kinase (MAPK). Activation of MAP kinase by mitogens appears to be essential for proliferation, and constitutive activation of this kinase is sufficient to induce cellular transformation. Blockade of downstream Ras signaling, as by use of a dominant negative Raf-1 protein, can completely inhibit mitogenesis, whether induced from cell surface receptors or from oncogenic Ras mutants.

The interaction of Raf and Ras is a key regulatory step in the control of cell proliferation. To date, no substrates of MEK other than MAPK have been identified; however, recent reports indicate that MEK may also be activated by other upstream signal proteins such as MEK kinase or MEK1 and PKC. Activated MAPK translocates and accumulates in the nucleus, where it can phosphorylate and activate transcription factors such as Elk-1 and Sapla, leading to the enhanced expression of genes such as that for c-fos.

Once activated, Raf and other kinases phosphorylate MEK on two neighboring serine residues, S²¹⁸ and S²²² in the case of MEK-1. These phosphorylations are required for activation of MEK as a kinase. In turn, MEK phosphorylates MAP kinase on two residues separated by a single amino acid: a tyrosine, Y¹⁸⁵ and a threonine, T¹⁸³. MEK appears to associate strongly with MAP kinase prior to phosphorylating it, suggesting that phosphorylation of MAP kinase by MEK may require a prior strong interaction between the two proteins. Two factors - MEK's unusual specificity and its requirement for a strong interaction with MAP kinase prior to phosphorylation -- suggest that MEK's mechanism of action may differ sufficiently from the mechanisms of other protein kinases as to allow for selective inhibitors of MEK. Possibly, such inhibitors would operate through allosteric mechanisms rather than through the more usual mechanism involving blockage of an ATP binding site.

Thus, MEK1 and MEK2 are validated and accepted targets for anti-proliferative therapies, even when the oncogenic mutation does not affect MEK structure or expression. See, *e.g.,* U.S. Patent Publications 2003/0149015 by Barrett et al. and 2004/0029898 by Boyle et al.

Several examples of 1-substituted-2(*p*-substituted-phenylamino)-aryl inhibitors of MEK have been reported. U.S. Patent Nos. 6,440,966 and 6,750,217 and corresponding publication WO 00/42003 described carboxylic and hydroxamic acid esters and N-substituted amide derivatives of sulfonamide-substituted-2(4-iodophenylamino)-benzoic acid esters and N-substituted benzamides as functioning as MEK inhibitors. The sulfonamide may also be N-substituted.

U.S. Patent 6,545,030 and corresponding publication WO 00/42029 describe MEK inhibitors that are 1-heterocyclyl-2(4-iodophenylamino)-benzene, where the heterocycle is a five-membered nitrogen-containing ring such as pyrazole, triazole, oxazole, isoxazole, and isoxazolinone. The more recent U.S. Patent Publication 2005/004186 describes related compounds in which the 4-iodo substituent of the '030 patent is replaced by a very broad genus of moieties including alkyl, alkoxy, acyloxy, alkenyl, carbamoyl, carbamoylalkyl, carboxyl, carboxylalkyl, N-acylsulfonamido, and others.

U.S. Patent 6,469,004 and corresponding publication WO 00/42022 describe carboxylic and hydroxamic acid esters of a group of heterocyclo-condensed phenylene compounds, *i.e.,* benzimidazoles, benzooxazoles, benzothiazoles, benzothiadiazoles, quinazolines, *etc..* The heterocycles are 7-F-6-(4-iodo-phenylamino)-5-carboxylic acid esters, carboxylic acid amides or hydroxamic acid esters. More recent publication U.S. 2005/0026970 described similar compounds in which the 4-iodo substituent was replaced by a very broad genus of structures. Related compounds are described in patent publications WO 03/077855, WO 03/77914 and US 2005/0554701. Further examples of 2-(4-iodophenylamino)-phenylhydroxamic acid esters which are reported to be useful as MEK inhibitors can be found in WO 2005/028426.

Patent Publication WO 02/06213 and corresponding U.S. Application Ser. No. 10/333,399 (U.S. 2004/0054172) describe hydroxy-substituted acid esters of 1-oxamic acid-2(4-halophenylamino)-3,4-difluorobenzene. U.S. Patent No. 6,891,066 and corresponding publication WO 03/62191 describe similar compounds wherein the 4-halo substituent is replaced by a very broad genus of structures. Among the substituents in the 4-position were methyl, ethyl, ethynyl, and 2-hydroxyethyl. Specific related compounds are described in U.S. Patent No. 6,770,778.

Patent Publication WO 04/083167, published September 30, 2004, (in Japanese) discloses more than two thousand - but provides NMR data for only 400 - 1-(N-substituted sulfonyl urea)-2(2,4-dihalophenylamino)-3,4-difluorobenzenes and asserts that they useful as MEK inhibitors. Data indicating inhibition of MEK were presented for a subgroup of just twelve. In addition to a secondary or tertiary amine, these twelve compounds all contained one of the following groups: an N, N-disubstituted sulfonyl urea, N-piperazinesulfonamide, N-piperidinesulfonamide or N-pyrrolidinesulfonamide.

The MEK cascade has also been implicated in inflammatory diseases and disorders. U.S. Application Publication No. 2006/0030610 to Koch et al.*,* U.S. Application Publication No. 2006/0140872 to Furue et al. This includes both acute and chronic inflammation disorders. Examples of such disorders are allergic contact dermatitis, rheumatoid arthritis, osteoarthritis, inflammatory bowel diseases, chronic obstructive pulmonary disorder, psoriasis, multiple sclerosis, asthma, diseases and disorders related to diabetic complications, and inflammatory complications of the cardiovascular system such as acute coronary syndrome. Among inflammatory bowel diseases are Crohn's disease and ulcerative colitis.

An article in Leukemia, 2002, 16(4), 486-507 relates to the Raf/MEK/ERK signal transduction cascade as a target for chemotherapeutic intervention in leukemia.

US 2003/0092748 relates to benzenesulfonamide derivatives and their use as MEK inhibitors.

An article in Journal of Biological Chemistry, 1995, 270(25), 14843-14846 addresses how MAP kinases are regulated.

WO 2005/121142 (Japan Tobacco Inc.) refers to 5-amino-2,4,7-trioxo-3,4,7,8-tetrahydro-2H-pyrido-[4,3-d]-pyrimidine derivatives and related compounds for the treatment of cancer.

US 2004/171632 relates to mitotic inhibitors such as paclitaxel which have inproved antitumour activity when used in combination with a selective MEK inhibitor, especially a phenyl amine compound as described therein.

### Brief Description of the Invention

This invention provides compounds of formula I 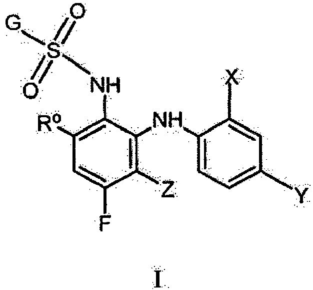 where G is R₁ₐ, R_{1b}, R_{1c}, R_{1d}, or R₁ₑ; R^{o} is H, halogen, CH₃NH-, (CH₃)₂N-, C₁-C₆ alkyl, C₁-C₄ alkoxy, C₃-C₆ cycloalkyl, C₂-C₆ alkenyl, C₂-C₆ alkynyl, said alkyl, alkoxy, cycloalkyl, alkenyl, and alkynyl groups optionally substituted with 1-3 substituents selected independently from halogen, OH, CN, cyanomethyl, nitro, phenyl, and trifluoromethyl, and said C₁-C₆ alkyl and C₁-C₄ alkoxy groups also optionally substituted with OCH₃ or OCH₂CH₃; X is F, Cl, or methyl; Y is I, Br, Cl, CF₃, C₁-C₃ alkyl, C₂-C₃ alkenyl, C₂-C₃ alkynyl, cyclopropyl, phenyl, pyridyl, pyrazolyl, OMe, OEt, or SMe, where all said methyl, ethyl, C₁-C₃ alkyl, and cyclopropyl groups of X and Y are optionally substituted with OH, where all said phenyl, pyridyl, pyrazolyl groups of Y are optionally substituted with halogen, acetyl, methyl, and trifluoromethyl, and where all said methyl groups of X and Y are optionally substituted with one, two, or three F atoms; and Z is H or F;
where R₁ₐ is methyl, optionally substituted with 1-3 fluorine atoms or 1-3 chlorine atoms, or with OH, cyclopropoxy, or C₁-C₄ alkoxy, where said cyclopropoxy group or the C₁-C₄ alkyl moieties of said C₁-C₄ alkoxy groups are optionally substituted with one hydroxy or methoxy group, and where all C₂-C₄ alkyl groups within said C₁-C₄ alkoxy are optionally further substituted with a second OH group;
R_{1b} is CH(CH₃)-C₁₋₃ alkyl or C₃-C₆ cycloalkyl, said methyl, alkyl and cycloalkyl groups optionally substituted with 1-3 substituents selected independently from F, Cl, Br, I, OH, OCH₃, and CN;
R_{1c} is (CH₂)ₙOₘR', where m is 0 or 1; where, when m is 1, n is 2 or 3, and when m is 0, n is 1 or 2; and where R' is C₁-C₆ alkyl, optionally substituted with 1-3 substituents selected independently from F, Cl, OH, OCH₃, OCH₂CH₃, and C₃-C₆ cycloalkyl;
R_{1d} is C(A)(A')(B)- were B, A, and A' are, independently, H or C₁₋₄ alkyl, optionally substituted with one or two OH groups or halogen atoms, or A and A', together with the carbon atom to which they are attached, form a 3- to 6- member saturated ring, said ring optionally containing one or two heteroatoms selected, independently, from O, N, and S and optionally substituted with one or two groups selected independently from methyl, ethyl, and halo; and
R₁ₑ is benzyl or 2-phenyl ethyl, in which the phenyl group is optionally substituted, as shown below: 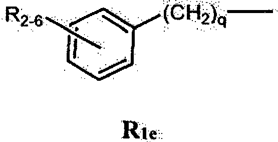 where q is 1 or 2, R₂, R₃ and R₄ are, independently, H, F, Cl, Br, CH₃, CH₂F, CHF₂, CF₃, OCH₃, OCH₂F, OCHF₂, OCF₃, ethyl, *n*-propyl, isopropyl, cyclopropyl, isobutyl, *sec-*butyl, *tert*-butyl, and methylsulfonyl, and R₄ may also be nitro, acetamido, amidinyl, cyano, carbamoyl, methylcarbamoyl, dimethylcarbamoyl, 1,3,4-oxadiazol-2-yl, 5-methyl-1,3,4-oxadiazol, 1,3,4-thiadiazol, 5-methyl-1,3,4-thiadiazol 1H-tetrazolyl, N-morpholyl carbonyl amino, N-morpholylsulfonyl and N-pyrrolidinylcarbonylamino; R₅ and R₆ are independently H, F, Cl or methyl.

Such compounds are inhibitors of MEK and are useful in treatment of cancer and other hyperproliferative diseases.

This invention is also directed to pharmaceutical compositions comprising pharmaceutically effective amounts of a compound of formula I or a pharmaceutically acceptable salt, ester, solvate, or hydrate thereof, and a pharmaceutically acceptable carrier. Such compositions may contain adjuvants, excipients, preservatives, agents for delaying absorption, fillers, binders, adsorbents, buffers, disintegrating agents, solubilizing agents, other carriers, and other inert ingredients. Methods of formulation of such compositions are well-known in the art.

The invention is also directed to a use of a compound of formula as defined in the claims herein, or a pharmaceutically acceptable salt, salt, ester, or hydrate thereof, for the manufacture of a medicament for treating a hyperproliferative disorder in a mammal, including a human.

The invention is also directed to a use of a compound of formula I as defined in the claims herein, or a pharmaceutically acceptable salt, salt, ester, or hydrate thereof, for the manufacture of a medicament for treating a disorder or condition ehich is modulated by the MEK cascade in a mammal, including a human.

The appropriate dosage for a particular patient can be determined, according to known methods, by those skilled in the art.

In accordance with an embodiment, the present invention covers compounds of general formula (I), *supra,* as defined in claim 1 herein, where :
X is F, Cl, or CH₃: Y is I, Br, Cl, CF₃, or C₁-C₃ alkyl; and Z is H or F.

In accordance with an embodiment, the present invention covers compounds of general formula (I), *supra,* as defined in claim 1 herein, where:
R^{o} is F, Cl, C₁-C₄ alkyl or C₁-C₄ alkoxy, said C₁-C₄ alkyl group and the C₁-C₄ alkyl moiety of said C₁-C₄ alkoxy group optionally substituted with F, Cl, OCH₃, or OCH₂CH₃.

In accordance with an embodiment, the present invention covers compounds of general formula (I), *supra,* as defined in claim 2 herein, where R^{o} is H, F, Cl, C₁-C₄ alkyl, methoxy, ethoxy, or 2-methoxy-ethoxy.

In accordance with an embodiment the present invention covers compounds of general formula (I), *supra,* as defined in claim 4 herein, where G is R₁ₐ and Z is F.

In accordance with an embodiment, the present invention covers compounds of general formula (I), *supra,* as defined in claim 5 herein, where G is CH₃; R^{o} is H; and Y is Br, I, CF₃, or CH₃.

In accordance with an embodiment, the present invention covers compounds of general formula (I), *supra,* as defined in any of claims 1 to 4 herein, where G is R_{1b}.

In accordance with an embodiment, the present invention covers compounds of general formula (I), *supra*, as defined in claim 4 herein, where G is R_{1b} and Z is F.

In accordance with an embodiment, the present invention covers compounds of general formula (I), *supra,* as defined in claim 8 herein, where R^{o} is H, F, or OCH₃, X is F or CH₃, and Y is Br, I, or CH₃.

In accordance with an embodiment, the present invention covers compounds of general formula (I), *supra*, as defined in claim 9 herein, where G is unsubstituted C₃-C₆ cycloalkyl.

In accordance with an embodiment, the present invention covers compounds of general formula (1), *supra,* as defined in claim 10 herein, where R^{o} is H.

In accordance with an embodiment, the present invention covers compounds of general formula (I), *supra,* as defined in claim 9 herein, where G is isopropyl or cyclopropyl.

In accordance with an embodiment, the present invention covers compounds of general formula (I), *supra,* as defined in any of claims 1 to 4 herein, where G is R_{1c}.

In accordance with an embodiment, the present invention covers compounds of general formula (I), *supra*, as defined in claim 4 herein, where G is R_{1c}, Y is I, Br, CH₃, or CF₃; and Z is F.

In accordance with an embodiment, the present invention covers compounds of general formula (I), *supra,* as defined in claim 14 herein, where m is zero.

In accordance with an embodiment, the present invention covers compounds of general formula (I), *supra,* as defined in any of claims 1 to 4 herein, where G is R_{1d}.

In accordance with an embodiment, the present invention covers compounds of general formula (I), *supra,* as defined in claim 16 herein, where R^{o} is fluoro, chloro, methyl, ethyl, propyl, isopropyl, sec-butyl, iso-butyl, tert-butyl, cyclopropyl, cyclobutyl, fluoromethyl, methoxy, fluoromethoxy, methylamino or dimethylamino; X is F, Cl, CH₃, or mono-, di- or tri- fluoromethyl; Y is I, Br, Cl, or mono-, di- or tri- fluoromethyl; and Z is H or F.

In accordance with an embodiment, the present invention covers compounds of general formula (I), *supra,* as defined in claim 16 herein, where R^{o} is F, Cl, methyl, ethyl, methoxy, ethoxy, or 2-methoxy-ethoxy; X is F, Cl, or CH₃; Y is I, Br, Cl, or mono-, di- or tri-fluoromethyl; and Z is H or F.

In accordance with an embodiment, the present invention covers compounds of general formula (I), *supra,* as defined in claim 16 herein, where R^{o} is H; X is F, Cl, CH₃, or mono-, di- or tri- fluoromethyl; Y is I, Br, Cl, or mono-, di- or tri- fluoromethyl; and Z is H or F.

In accordance with an embodiment, the present invention covers compounds of general formula (I), *supra,* as defined in any of claims 17-19 herein, where C(A)(A') is C₁-C₆ cycloalkyl.

In accordance with an embodiment, the present invention covers compounds of general formula (I), *supra,* as defined in claim 20 herein, where B is H.

In accordance with an embodiment, the present invention covers compounds of general formula (I), *supra,* as defined in claim 21 herein, where C(A)(A') is cyclopropyl.

In accordance with an embodiment, the present invention covers compounds of general formula (I), *supra,* as defined in claim 20 herein, where B is methyl, optionally substituted with one OH group, or C₂-C₄ alkyl, optionally substituted with one or two OH groups.

In accordance with an embodiment, the present invention covers compounds of general formula (I), *supra*, as defined in claim 23 herein, where C(A)(A') is cyclopropyl.

In accordance with an embodiment, the present invention covers compounds of general formula (I), *supra,* as defined in claim 24 herein, where B is methyl, ethyl, 2-hydroxyethyl, *n-*propyl, 3-hydiroxypropyl, 2,3-dihydroxypropyl, 3,4-dihydroxybutyl, isopropyl, 1-methyl-2-hydroxy ethyl, n-butyl, sec-butyl, isobutyl, or 2-hydroxymethyl-3-hydroxy propyl.

In accordance with an embodiment, the present invention covers compounds of general formula (I), *supra,* as defined in claim 25 herein, where B is 2,3-dihydroxypropyl or 3,4-dihydroxybutyl.

In accordance with an embodiment, the present invention covers compounds of general formula (I), *supra,* as defined in claim 26 herein, in which the chiral carbon in B is in the R configuration.

In accordance with an embodiment, the present invention covers compositions comprising a compound of general formula (I), *supra*, as defined in claim 27 herein, which is substantially free of the S isomer.

In accordance with an embodiment, the present invention covers compounds of general formula (I), *supra,* as defined in any of claims 1 to 4 herein, where G is R₁ₑ.

In accordance with an embodiment, the present invention covers compounds of general formula (I), *supra*, as defined in claim 29 herein, where q is 1.

In accordance with an embodiment, the present invention covers compounds of general formula (I), *supra,* as defined in claim 30 herein, where R^{o} is H, R₄₋₆ are H; R₂ and R₃ are, independently, H, F, Cl, Br, CH₃, CH₂F, CHF₂, CF₃, OCH₃, OCH₂F, OCHF₂, OCF₃, ethyl, *n-*propyl, isopropyl, cyclopropyl, isobutyl, *sec*-butyl, *tert-*butyl, and methylsulfonyl; X is F and Y is I.

In one subgeneric embodiment, this invention provides a compound of formula IA-1, 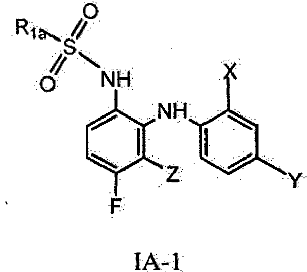 where R₁ₐ is defined as above.

In another embodiment the invention provides a compound of formula IA-2, where R₁ₐ is defined as above and where R^{o}' is R^{o}, defined above, other than H.

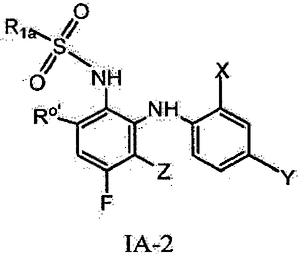

In another embodiment, the invention provides a compound formula IB-1, 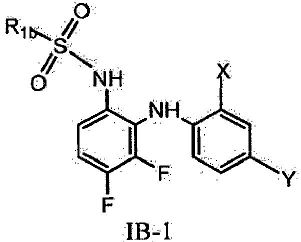 where X and Y are defined as for formula I, and where R_{1b} is defined as above for formula I.

In another embodiment, the invention provides a compound of formula IB-2, 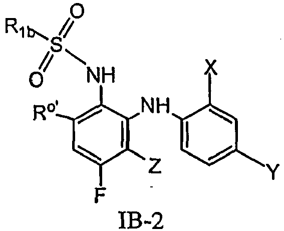 where R_{1b} is defined as above for formula I, and where R^{o}' is R^{o}, defined above, other than H.

In a third embodiment, this invention provides a compound of formula IC-1, 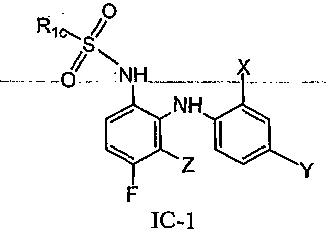 where R_{1c} is (CH₂)ₙOₘR', where m is 0 or 1, n is 2 or 3 when m is 1, and n is 1 or 2 when m is 0, and R' is C₁-C₆ alkyl, optionally substituted with 1-3 substituents selected independently from F, Cl, OH, OCH₃, OCH₂CH₃, and C₃-C₆ cycloalkyl.

In another embodiment, this invention provides a compound of formula IC-2, 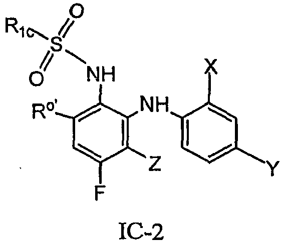 where R_{1c} is defined as above and where R^{o}' is R^{o}, defined above, other than H.

In another embodiment, this invention provides a compound of formula ID-1 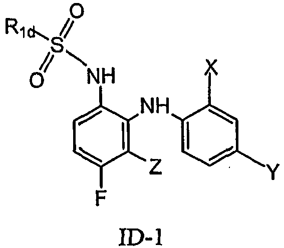 where R_{1d} is C(A)(A')(B)- where B, A, and A' are, independently, H or C₁₋₄ alkyl, optionally substituted with one or two OH groups or halogen atoms, or A and A', together with the carbon atom to which they are attached, form a 3- to 6- member saturated ring, said ring optionally containing one or two heteroatoms selected, independently, from O, N, and S and optionally substituted with one or two groups selected independently from methyl, ethyl, and halo

In another embodiment, the invention provides a compound of formula ID-2, 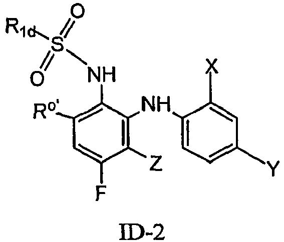 where R_{1d} is defined as above and where R^{o}' is R^{o}, defined above, other than H.

In another embodiment, this invention provides a compound of formula IE-1. 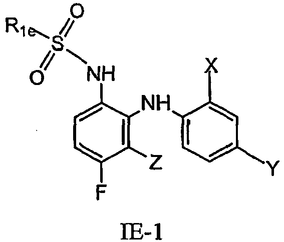 where R₁ₑ is defined as above.

In another embodiment, this invention provides a compound of formula IE-2, 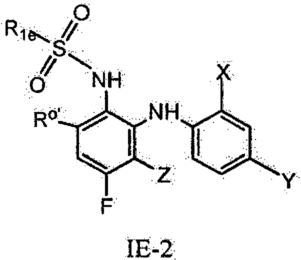 where R^{o}' is R^{o}, defined above, other than H.

In additional subgeneric embodiments, this invention provides compounds of formulas IA-1, IA-2, IB-1, IB-2, IC-1, IC-2, ID-1 and ID2, where X is F, Cl, or CH₃: Y is I, Br, Cl, CF₃, or C₁-C₃ alkyl, and Z is H or F.

In additional subgeneric embodiments, this invention provides compounds of formulas IA-2, IB-2, IC-2 and ID-2, where X is F, Cl, or CH₃: Y is I, Br, Cl, CF₃, or C₁-C₃ alkyl, Z is H or F, and R^{o} is halogen, C₁-C₆ alkyl, monohalo C₁-C₆ alkyl, C₃-C₆ cycloalkyl, C₂-C₆ alkenyl, C₂-C₆ alkynyl, phenyl, monosubstituted phenyl, OR₃, O-C(=O)R₄, or C(=O)OR₅.

In additional subgeneric embodiments, this invention provides or contemplates compounds of formulas IA-2, IB-2, IC-2 and ID-2, where X is F, Cl, or CH₃: Y is I, Br, Cl, CF₃, or C₁-C₃ alkyl, Z is H or F, and R^{o} is furyl, thienyl, thiazolyl, isothiazolyl, oxazolyl, isoxazolyl, pyrrolyl, or pyrazolyl.

In additional subgeneric embodiments, this invention provides compounds of formulas IA-2, IB-2, IC-2 and ID-2, where X is F, Cl, or CH₃: Y is I, Br, Cl, CF₃, or C₁-C₃ alkyl, is H or F, and R^{o} is F, Cl, C₁-C₄ alkyl, C₁-C₃ alkoxy, trifluoromethoxy, or 2-methoxy-ethoxy.

In a more specific subgeneric embodiment, this invention provides a compound of formula IA-1, where R₁ₐ is methyl, monohalomethyl, C₁-C₃ alkoxymethyl, or cyclopropoxymethyl.

In another more specific subgeneric embodiment, this invention provides a compound of formula IA-2, where R₁ₐ is methyl, monohalomethyl, C₁-C₃ alkoxymethyl, or cyclopropoxy methyl and where R^{o}' is F, Cl, C₁-C₃ alkyl, monochloro C₁-C₃ alkyl, C₁-C₃ alkoxy, trifluoro methoxy, or 2-methoxy-ethoxy.

In a more specific subgeneric embodiment of formula IB-1, this invention provides a compound of formula IB-1, where R_{1b} is isopropyl, 2-butyl, 2-pentyl, cyclopropyl, cyclobutyl, cyclopentyl, or cyclohexyl, all optionally substituted with 1 or 2 substituents selected independently from F, Cl, OH, and OCH₃; Y is Br, I, methyl, or trifluoromethyl.

In a more specific subgeneric embodiment of formula IB-2, this invention provides a compound of formula IB-2, where R_{1b} is isopropyl, 2-butyl, 2-pentyl, cyclopropyl, cyclobutyl, cyclopentyl, or cyclohexyl, optionally substituted with 1 or 2 substituents selected independently from F, Cl, OH, and OCH₃; Y is Br, I, methyl, or trifluoromethyl; and R^{o}' is F, Cl, C₁-C₃ alkyl, monochloro C₁-C₃ alkyl, C₁-C₃ alkoxy, trifluoromethoxy, or 2-methoxy-ethoxy.

In a still more specific subgeneric embodiment of formula IB-1, this invention provides a compound of formula IB-1, where R_{1b} is isopropyl, 2-butyl, 2-pentyl, cyclopropyl, cyclobutyl, cyclopentyl, or cyclohexyl, all optionally substituted with one Cl, with one or with 1 or 2 OH groups; and Y is Br, I, methyl, or trifluoromethyl.

In a more specific subgeneric embodiment of formula IB-2, this invention provides a compound of formula IB-2, where R_{1b} is isopropyl, 2-butyl, 2-pentyl, cyclopropyl, cyclobutyl, cyclopentyl, or cyclohexyl, all optionally substituted with one Cl or with 1 or 2 OH groups; Y is Br I, methyl, or trifluoromethyl; and R^{o}' is F, Cl, C₁-C₃ alkyl, monochloro C₁-C₃ alkyl, C₁-C₃ alkoxy, trifluoromethoxy, or 2-methoxy-ethoxy.

In another more specific subgeneric embodiment, this invention provides a compound of formula IC-1 or IC-2, where m is zero, n is 1 or 2, and R' is C₁-C₄ alkyl, optionally substituted as described above.

In another more specific subgeneric embodiment, this invention provides a compound of formula IC-1 or IC-2, where m is 1, n is 2 or 3, and R' is C₁-C₄ alkyl, optionally substituted as described above.

In a still more specific subgeneric embodiment, this invention provides a compound of formula IC-1 or IC-2, where m is zero, n is 1 or 2, and R' is-C₁-C₄ alkyl, optionally substituted with 1 -3 groups selected from OH, OCH₃, Cl, and cyclopropyl.

In another more specific subgeneric embodiment, this invention provides a compound of formula ID-1, where R_{1d} is cycloalkyl or 1-alkyl-cycloalkyl, in which the 1-alkyl group is optionally substituted with one or two OH groups or with one or two halogen atoms.

In another more specific subgeneric embodiment, this invention provides a compound of formula ID-2, where R^{o}' is halogen, C₁-C₆ alkyl, monohalo C₁-C₆ alkyl, C₃-C₆ cycloalkyl, C₂-C₆ alkenyl, C₂-C₆ alkynyl, phenyl, monosubstituted phenyl, OR₃, O-C(=O)R₄, or C(=O)OR₅; and R_{1d} is cycloalkyl or 1-alkyl-cycloalkyl, in which the 1-alkyl group is optionally substituted with one or two OH groups or with one or two halogen atoms.

In another more specific subgeneric embodiment, this invention provides a compound of formula ID-2, where R^{o}' is furyl, thienyl, thiazolyl, isothiazolyl, oxazolyl, isoxazolyl, pyrrolyl, or pyrazolyl; and R_{1d} is cycloalkyl or 1-alkyl-cycloalkyl, in which the 1-alkyl group is optionally substituted with one or two OH groups or one or two halogen atoms.

In another more specific subgeneric embodiment, this invention provides a compound of formula ID-1, where R_{1d} is cycloalkyl or 1-alkyl-cycloalkyl, in which the 1-alkyl group is optionally substituted with one or two OH groups, and where Y is Br, I, methyl, or trifluoromethyl.

In another more specific subgeneric embodiment, this invention provides a compound of formula ID-1, where R_{1d} is cycloalkyl or 1-alkyl-cycloalkyl, in which the 1-alkyl group is optionally substituted with one or two fluorine or chlorine atoms, and where Y is Br, I, methyl, or trifluoromethyl.

In another more specific subgeneric embodiment, this invention provides a compound of formula ID-2, where R_{1d} is cycloalkyl or (1-alkyl)-cycloalkyl, in which the 1-alkyl group is optionally substituted with one or two OH groups, and where R^{o}' is F, Cl, C₁-C₃ alkyl, monochloro C₁-C₃ alkyl, C₁-C₃ alkoxy, trifluoromethoxy, or 2-methoxy-ethoxy.

In another more specific subgeneric embodiment, this invention provides a compound of formula ID-1, where R_{1d} is tetrahydrofuryl, tetrahydrothienyl, pyrrolidyl, piperidyl, piperazinyl, or morpholyl, each optionally substituted as described above, and where Y is Br, I, methyl, or trifluoromethyl.

In another more specific subgeneric embodiment, this invention provides a compound of formula ID-1, where R_{1d} is oxazolidinyl, thiazolidinyl, isoxazolidinyl, isothiazolidinyl, tetrahydrofuryl, tetrahydrothienyl, pyrrolidyl, piperidyl, piperazinyl, or morpholyl, each optionally substituted as described above, and where Y is Br, I, methyl, or trifluoromethyl.

In another more specific subgeneric embodiment, this invention provides a compound of formula ID-2, where R_{1d} is cyclopropyl or 1-alkyl-cyclopropyl, in which the 1-alkyl group is optionally substituted with one or two OH groups, and where R^{o}' is F, Cl, methyl, ethyl, chloromethyl, C₁-C₂ alkoxy, trifluoromethoxy, or 2-methoxy-ethoxy.

In an even more specific embodiment, the invention provides a compound of formula ID-1 in which R_{1d} is 1-(monohydroxyalkyl) cycloalkyl.

In another more specific embodiment, the invention provides a compound of formula ID-2 in which R_{1d} is 1-(monohydroxyalkyl) cycloalkyl, where R^{o}' is F, Cl, methyl, ethyl, chloromethyl, C₁-C₂ alkoxy, trifluoromethoxy, or 2-methoxy-ethoxy.

In an even more specific embodiment, the invention provides a compound of formula ID-1 in which R_{1d} is 1-(dihydroxyalkyl) cycloalkyl.

In another more specific embodiment, the invention provides a compound of formula ID-2 in which R_{1d} is I-(dihydroxyalkyl) cycloalkyl, where R^{o}' is F, Cl, methyl, ethyl, chloromethyl, C₁-C₂ alkoxy, trifluoromethoxy, or 2-methoxy-ethoxy.

In a more specific subgeneric embodiment, this invention provides a compound of formula I, where A is An, where Ar₁ is phenyl or monosubstitutedphenyl, R^{o} is F, Cl, C₁-C₃ alkyl, C₁-C₃ alkoxy, 2-methoxyethoxy, C₂-C₃ alkenyl, C₂-C₃alkynyl, trifluoromethyl, phenyl, furyl, or thienyl. thiazolyl, isothiazolyl, oxazolyl, isoxazolyl, pyrrolyl, or pyrazolyl; X is F, Cl, or methyl; Y is I, Br, Cl, CF₃, or C₁-C₃alkyl; and Z is F.

### Detailed description of the invention

As used herein a "pharmaceutically acceptable salt" includes salts that retain the biological effectiveness of the free acids and bases of the specified compound and that are not biologically or otherwise undesirable. A compound of this invent ion may possess acidic or basic groups and therefore may react with any of a number of inorganic or organic bases, and inorganic and organic acids, to form a pharmaceutically acceptable salt. Examples of pharmaceutically acceptable salts include those salts prepared by reaction of the compounds of this invention with a mineral or organic acid or an inorganic base, such salts including sulfates, pyrosulfates, bisulfates, sulfites, bisulfites, phosphates, monohydrogenphosphates, dihydrogenphosphates, metaphosphates, pyrophosphates, chlorides, bromides, iodides, acetates, propionates, decanoates, caprylates, acrylates, formates, isobutyrates, caproates, heptanoates, propiolates, oxalates, malonates, succinates, suberates, sebacates, fumarates, maleates, butyn-1,4-dioates, hexyne-1,6-dioates, benzoates, chlorobenzoates, methylbenzoates, dinitrobenzoates, hydroxybenzoates, methoxybenzoates, phthalates, sulfonates, xylenesulfonates, phenylacetates, phenylpropionates, phenylbutyrates, citrates, lactates, .γ-hydroxybutyrates, glycollates, tartrates, methanesulfonates, propanesulfonates, naphthalene-1-sulfonates, naphthalene-2-sulfonates, and mandelates.

As used herein, a "prodrug" is a compound that may be converted under physiological conditions or by solvolysis to the specified compound or to a pharmaceutically acceptable salt of such compound. Prodrugs include compounds wherein an amino acid residue, or a polypeptide chain of two or more amino acid residues, is covalently joined through an amide or ester bond to a free amino, hydroxy, or carboxylic acid group of compounds of Formulas I. The amino acid residues contemplated include but are not limited to the 20 naturally-occurring amino acids. Other suitable amino acids include 4-hydroxyproline, hydroxylysine, demosine, isodemosine, 3-methyl histidine, norvaline, β-alanine, γ-aminobutyric acid, cirtulline, homocysteine, homoserine, ornithine and methionine sulfone. Additional types of prodrugs are well known in the art.

The tables below show the individual compounds provided or contemplated by this invention.

Table I shows embodiments of this invention which are compounds of formula IA-1.

**Table 1: Embodiments According to Formula IA-1**

| **R₁ₐ** | **X** | **Y** | **Z** |
|---|---|---|---|
| | | | |
| CH₃ | F | I | F |
| CH₃ | Cl | I | F |
| CH₃ | F | Br | F |
| CH₃ | Cl | Br | F |
| CH₃ | F | CH₃ | F |
| CH₃ | Cl | CH₃ | F |
| CH₃ | F | CF₃ | F |
| CH₃ | Cl | CF₃ | F |
| CH₃ | F | C≡CH | F |
| CH₃ | Cl | C=CH | F |
| CH₃ | F | SCH₃ | F |
| CH₃ | Cl | SCH₃ | F |
| CH₃ | F | (CH₂)₂CH₃ | F |
| CH₃ | Cl | (CH₂)₂CH₃ | F |
| CH₃ | F | CH₂CH₃ | F |
| CH₃ | Cl | CH₂CH₃ | F |
| CH₃ | F | CH₂OH | F |
| CH₃ | Cl | CH₂OH | F |
| CH₃ | F | 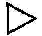 | F |
| CH₃ | Cl | 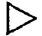 | F |
| CH₃ | CH₃ | CH=CH₂ | F |
| CH₃ | CH₃ | C=CH | F |
| CH₃ | CH₃ | SCH₃ | F |
| | | | |
| CH₂F | F | I | F |
| CH₂F | Cl | I | F |
| CH₂F | F | Br | F |
| CH₂F | Cl | Br | F |
| CH₂F | F | CH₃ | F |
| CH₂F | Cl | CH₃ | F |
| CH₂F | F | CF₃ | F |
| CH₂F | Cl | CF₃ | F |
| | | | |
| CF₃ | F | I | F |
| CF₃ | Cl | I | F |
| CF₃ | F | Br | F |
| CF₃ | Cl | Br | F |
| CF₃ | F | CH₃ | F |
| CF₃ | Cl | CH₃ | F |
| CF₃ | F | CF₃ | F |
| CF₃ | Cl | CF₃ | F |
| CH₂Cl | F | I | F |
| CH₂Cl | Cl | I | F |
| CH₂Cl | F | Br | F |
| CH₂Cl | Cl | Br | F |
| CH₂Cl | F | CH₃ | F |
| CH₂Cl | Cl | CH₃ | F |
| CH₂Cl | F | CF₃ | F |
| CH₂Cl | Cl | CF₃ | F |
| | | | |
| CHCl₂ | F | I | F |
| CHCl₂ | Cl | I | F |
| CHCl₂ | F | Br | F |
| CHCl₂ | Cl | Br | F |
| CHCl₂ | F | CH₃ | F |
| CHCl₂ | Cl | CH₃ | F |
| CHCl₂ | F | CF₃ | F |
| CHCl₂ | Cl | CF₃ | F |
| | | | |
| CCl₃ | F | I | F |
| CCl₃ | Cl | I | F |
| CCl₃ | F | Br | F |
| CCl₃ | Cl | Br | F |
| CCl₃ | F | CH₃ | F |
| CCl₃ | Cl | CH₃ | F |
| CCl₃ | F | CF₃ | F |
| CCl₃ | Cl | CF₃ | F |
| | | | |
| CH₂OH | F | I | F |
| CH₂OH | Cl | I | F |
| CH₂OH | F | Br | F |
| CH₂OH | Cl | Br | F |
| CH₂OH | F | CH₃ | F |
| CH₂OH | Cl | CH₃ | F |
| CH₂OH | F | CF₃ | F |
| CH₂OH | Cl | CF₃ | F |
| | | | |
| CH₂OMe | F | I | F |
| CH₂OMe | Cl | I | F |
| CH₂OMe | F | Br | F |
| CH₂OMe | Cl | Br | F |
| CH₂OMe | F | CH₃ | F |
| CH₂OMe | Cl | CH₃ | F |
| CH₂OMe | F | CF₃ | F |
| CH₂OMe | Cl | CF₃ | F |
| CH₂OMe | F | C≡CH | F |
| CH₂OMe | Cl | SCH₃ | F |
| CH₂OMe | CH₃ | CF₃ | F |
| CH₂OMe | CH₃ | C≡CH | F |
| | | | |
| CH₂OEt | F | I | F |
| CH₂OEt | Cl | I | F |
| CH₂OEt | F | Br | F |
| CH₂OEt | Cl | Br | F |
| CH₂OEt | F | CH₃ | F |
| CH₂OEt | Cl | CH₃ | F |
| CH₂OEt | F | CF₃ | F |
| CH₂OEt | Cl | CF₃ | F |
| | | | |
| 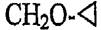 | F | I | F |
| 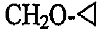 | Cl | I | F |
| 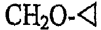 | F | Br | F |
| 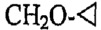 | Cl | Br | F |
| 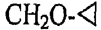 | F | CH₃ | F |
| 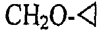 | Cl | CH₃ | F |
| 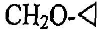 | F | CF₃ | F |
| 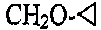 | Cl | CF₃ | F |
| | | | |
| 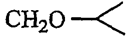 | Cl | I | F |
| 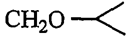 | F | Br | F |
| 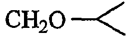 | Cl | Br | F |
| 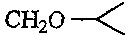 | F | CH₃ | F |
| 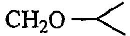 | Cl | CH₃ | F |
| 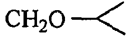 | F | CF₃ | F |
| 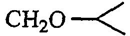 | Cl | CF₃ | F |
| | | | F |
| 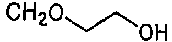 | F | I | F |
| 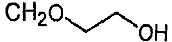 | Cl | I | F |
| 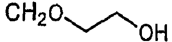 | F | Br | F |
| 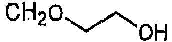 | Cl | Br | F |
| 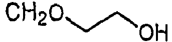 | F | CH₃ | F |
| 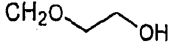 | Cl | CH₃ | F |
| 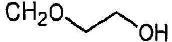 | F | CF₃ | F |
| 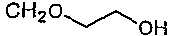 | Cl | CF₃ | F |
| 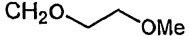 | F | I | F |
| 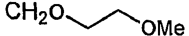 | Cl | I | F |
| 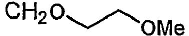 | F | Br | F |
| 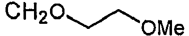 | Cl | Br | F |
| 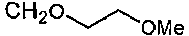 | F | CH₃ | F |
| 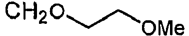 | Cl | CH₃ | F |
| 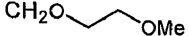 | F | CF₃ | F |
| 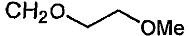 | Cl | CF₃ | F |
| 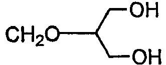 | F | I | F |
| 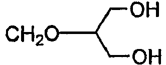 | Cl | I | F |
| 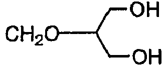 | F | Br | F |
| 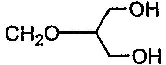 | Cl | Br | F |
| 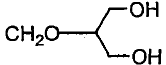 | F | CH₃ | F |
| 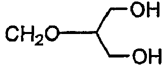 | Cl | CH₃ | F |
| 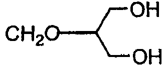 | F | CF₃ | F |
| 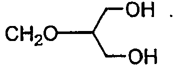 | Cl | CF₃ | F |

**Table 2: Embodiments According to Formula IB**

| **R_{1b}** | **X** | **Y** | **Z** |
|---|---|---|---|
| 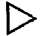 | F | I | F |
| 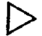 | Cl | I | F |
| 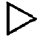 | F | Br | F |
| 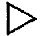 | Cl | Br | F |
| 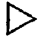 | F | CH₃ | F |
| 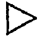 | Cl | CH₃ | F |
| 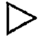 | F | CF₃ | F |
| 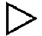 | Cl | CF₃ | F |
| 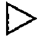 | F | C=CH | F |
| 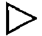 | Cl | C≡CH | F |
| 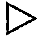 | F | SCH₃ | F |
| 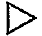 | Cl | SCH₃ | F |
| 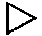 | F | CH₂OH | F |
| 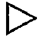 | Cl | CH₂OH | F |
| 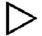 | F | (CH₂)₃OH | F |
| 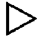 | Cl | (CH₂)₃OH | F |
| 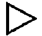 | F | (CH₂)₂CH₃ | F |
| 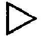 | Cl | (CH₂)₂CH₃ | F |
| 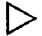 | F | CH₂CH₃ | F |
| 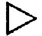 | Cl | CH₂CH₃ | F |
| 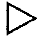 | F | (CH₂)₂CH₃ | F |
| 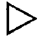 | Cl | (CH₂)₂CH₃ | F |
| 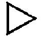 | CH₃ | I | F |
| 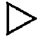 | CH₃ | Br | F |
| 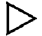 | CH₃ | CH₃ | F |
| 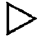 | CH₃ | CF₃ | F |
| 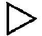 | CH₃ | CH₂CH₃ | F |
| 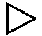 | CH₃ | (CH₂)₂CH₃ | F |
| 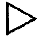 | CH₃ | C=CH | F |
| 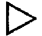 | CH₃ | SCH₃ | F |
| 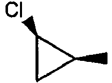 | Cl | (CH₂)₂CH₃ | F |
| 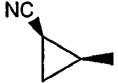 | CH₃ | I | F |
| 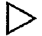 | F | CH=CH₂ | F |
| 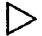 | Cl | CH=CH₂ | F |
| 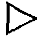 | CH₃ | CH=CH₂ | F |
| 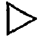 | F | 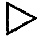 | F |
| 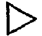 | F | OCH₃ | F |
| 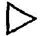 | Cl | (CH₂)₂CH₂OH | F |
| 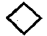 | F | I | F |
| | | | |
| 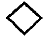 | Cl | I | F |
| 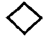 | F | Br | F |
| 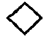 | Cl | Br | F |
| 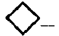 | F | CH₃ | F |
| 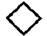 | Cl | CH₃ | F |
| 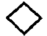 | F | CF₃ | F |
| 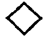 | Cl | CF₃ | F |
| | | | |
| 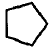 | F | I | F |
| 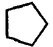 | Cl | I | F |
| 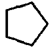 | F | Br | F |
| 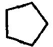 | Cl | Br | F |
| 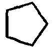 | F | CH₃ | F |
| 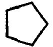 | Cl | CH₃ | F |
| 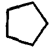 | F | CF₃ | F |
| 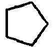 | Cl | CF₃ | F |
| 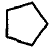 | Cl | 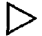 | F |
| 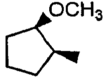 | F | (CH₂)₂CH₃ | F |
| 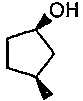 | Cl | C≡CH | F |
| 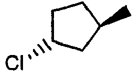 | CH₃ | SCH₃ | F |
| 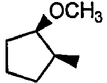 | Cl | CF₃ | F |
| 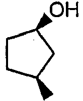 | CH₃ | CH₃ | F |
| 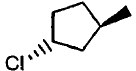 | F | CH₂OH | F |
| 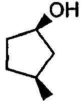 | Cl | (CH₂)₃OH | F |
| 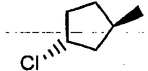 | F | OCH₂CH₃ | F |
| | | | |
| 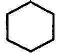 | F | I | F |
| 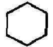 | Cl | I | F |
| 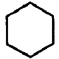 | F | Br | F |
| 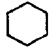 | Cl | Br | F |
| 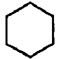 | F | CH₃ | F |
| 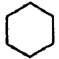 | Cl | CH₃ | F |
| 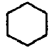 | F | CF₃ | F |
| 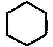 | Cl | CF₃ | F |

Table 3 shows embodiments according to formula IC

**Table 3 Embodiments According to Formula IC**

| **R_{1c}** | **X** | **Y** | **Z** |
|---|---|---|---|
| CH₂CH₃ | F | I | F |
| CH₂CH₃ | Cl | I | F |
| CH₂CH₃ | F | Br | F |
| CH₂CH₃ | Cl | Br | F |
| CH₂CH₃ | F | CH₃ | F |
| CH₂CH₃ | Cl | CH₃ | F |
| CH₂CH₃ | F | CF₃ | F |
| CH₂CH₃ | Cl | CF₃ | F |
| CH₂CH₃ | CH₃ | CH₃ | F |
| CH₂CH₃ | CH₃ | CH₃ | F |
| CH₂CH₃ | CH₃ | C≡CH | F |
| CH₂CH₃ | CH₃ | SCH₃ | F |
| CH₂CH₃ | F | C≡CH | F |
| CH₂CH₃ | Cl | SCH₃ | F |
| CH₂CH₃ | F | 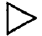 | F |
| CH₂CH₃ | Cl | 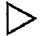 | F |
| CH₂CH₃ | CH₃ | 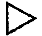 | F |
| | | | |
| CH(CH₃)₂ | F | OCH₃ | F |
| CH(CH₃)₂ | Cl | OCH₃ | F |
| CH(CH₃)₂ | F | I | F |
| CH(CH₃)₂ | Cl | I | F |
| CH(CH₃)₂ | F | Br | F |
| CH(CH₃)₂ | Cl | Br | F |
| CH(CH₃)₂ | F | CH₃ | F |
| CH(CH₃)₂ | Cl | CH₃ | F |
| CH(CH₃)₂ | F | CH₂CH₃ | F |
| CH(CH₃)₂ | Cl | CH₂CH₃ | F |
| CH(CH₃)₂ | CH₃ | CH₂CH₃ | F |
| CH(CH₃)₂ | Cl | CH₂CH₃ | F |
| CH(CH₃)₂ | Fl | CH(CH₃)₂ | F |
| CH(CH₃)₂ | Cl | CH(CH₃)₂ | F |
| | | | |
| CH(CH₃)₂ | F | CF₃ | F |
| CH(CH₃)₂ | Cl | CF₃ | F |
| CH(CH₃)₂ | CH₃ | Br | F |
| CH(CH₃)₂ | CH₃ | C≡CH | F |
| CH(CH₃)₂ | CH₃ | SCH₃ | F |
| CH(CH₃)₂ | CH₃ | 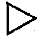 | F |
| CH(CH₃)₂ | F | CH₂OH | F |
| CH(CH₃)₂ | Cl | 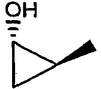 | F |
| | | | |
| *n*-butyl | F | I | F |
| *n*-butyl | Cl | I | F |
| *n*-butyl | F | Br | F |
| *n*-butyl | Cl | Br | F |
| *n*-butyl | F | CH₃ | F |
| *n*-butyl | Cl | CH₃ | F |
| *n*-butyl | F | OCH₃ | F |
| *n*-butyl | Cl | OCH₃ | F |
| *n*-butyl | CH₃ | OCH₃ | F |
| *n*-butyl | Cl | OCH₂CH₃ | F |
| *n*-butyl | F | OCH₂CH₃ | F |
| *n*-butyl | CH₃ | OCH₂CH₃ | F |
| *n*-butyl | F | OCH₂CH₂OH | F |
| | | | |
| *n*-butyl | F | CF₃ | F |
| *n*-butyl | Cl | CF₃ | F |
| | | | |
| *sec*-butyl | F | I | F |
| *sec*-butyl | Cl | I | F |
| *sec*-butyl | F | Br | F |
| *sec*-butyl | Cl | Br | F |
| *sec*-butyl | F | CH₃ | F |
| *sec*-butyl | Cl | CH₃ | F |
| *sec*-butyl | F | CF₃ | F |
| *sec*-butyl | Cl | CF₃ | F |
| | | | |
| CH₂CF₃ | F | I | F |
| CH₂CF₃ | Cl | I | F |
| CH₂CF₃ | F | Br | F |
| CH₂CF₃ | Cl | Br | F |
| CH₂CF₃ | F | CH₃ | F |
| CH₂CF₃ | Cl | CH₃ | F |
| CH₂CF₃ | F | CF₃ | F |
| CH₂CF₃ | Cl | CF₃ | F |
| | | | |
| CH₂CCl₃ | F | I | F |
| CH₂CCl₃ | Cl | I | F |
| CH₂CCl₃ | F | Br | F |
| CH₂CCl₃ | Cl | Br | F |
| CH₂CCl₃ | F | CH₃ | F |
| CH₂CCl₃ | Cl | CH₃ | F |
| CH₂CCl₃ | F | CF₃ | F |
| CH₂CCl₃ | Cl | CF₃ | F |
| | | | |
| CH₂-◁ | F | I | F |
| CH₂-◁ | Cl | I | F |
| CH₂-◁ | F | Br | F |
| CH₂-◁ | Cl | Br | F |
| CH₂-◁ | F | CH₃ | F |
| CH₂-◁ | Cl | CH₃ | F |
| CH₂-◁ | F | CF₃ | F |
| CH₂-◁ | Cl | CF₃ | F |
| | | | |
| CH₂CH₂F | F | I | F |
| CH₂CH₂F | Cl | I | F |
| CH₂CH₂F | F | Br | F |
| CH₂CH₂F | Cl | Br | F |
| CH₂CH₂F | F | CH₃ | F |
| CH₂CH₂F | Cl | CH₃ | F |
| CH₂CH₂F | F | CF₃ | F |
| CH₂CH₂F | Cl | CF₃ | F |
| | | | |
| CH₂CH₂Cl | F | I | F |
| CH₂CH₂Cl | Cl | I | F |
| CH₂CH₂Cl | F | Br | F |
| CH₂CH₂Cl | Cl | Br | F |
| CH₂CH₂Cl | F | CH₃ | F |
| CH₂CH₂Cl | Cl | CH₃ | F |
| CH₂CH₂Cl | F | CF₃ | F |
| CH₂CH₂Cl | Cl | CF₃ | F |
| | | | |
| CH₂CH₂CH₂Cl | F | I | F |
| CH₂CH₂CH₂Cl | Cl | I | F |
| CH₂CH₂CH₂Cl | F | Br | F |
| CH₂CH₂CH₂Cl | Cl | Br | F |
| CH₂CH₂CH₂Cl | F | CH₃ | F |
| CH₂CH₂CH₂Cl | Cl | CH₃ | F |
| CH₂CH₂CH₂Cl | F | CF₃ | F |
| CH₂CH₂CH₂Cl | Cl | CF₃ | F |
| | | | |
| CH₂CH₂OH | F | I | F |
| CH₂CH₂OH | Cl | I | F |
| CH₂CH₂OH | F | Br | F |
| CH₂CH₂OH | Cl | Br | F |
| CH₂CH₂OH | F | CH₃ | F |
| CH₂CH₂OH | Cl | CH₃ | F |
| CH₂CH₂OH | F | CF₃ | F |
| CH₂CH₂OH | Cl | CF₃ | F |
| | | | |
| CH₂CH₂CH₂OH | F | I | F |
| CH₂CH₂CH₂OH | Cl | I | F |
| CH₂CH₂CH₂OH | F | Br | F |
| CH₂CH₂CH₂OH | Cl | Br | F |
| CH₂CH₂CH₂OH | F | CH₃ | F |
| CH₂CH₂CH₂OH | Cl | CH₃ | F |
| CH₂CH₂CH₂OH | F | CF₃ | F |
| CH₂CH₂CH₂OH | Cl | CF₃ | F |
| | | | |
| (CH₂)₄OH | F | I | F |
| (CH₂)₄OH | Cl | I | F |
| (CH₂)₄OH | F | Br | F |
| (CH₂)₄OH | Cl | Br | F |
| (CH₂)₄OH | F | CH₃ | F |
| (CH₂)₄OH | Cl | CH₃ | F |
| (CH₂)₄OH | F | CF₃ | F |
| (CH₂)₄OH | Cl | CF₃ | F |
| | | | |
| CH₂CH₂OCH₃ | F | I | F |
| CH₂CH₂OCH₃ | Cl | I | F |
| CH₂CH₂OCH₃ | F | Br | F |
| CH₂CH₂OCH₃ | Cl | Br | F |
| CH₂CH₂OCH₃ | F | CH₃ | F |
| CH₂CH₂OCH₃ | Cl | CH₃ | F |
| CH₂CH₂OCH₃ | F | CF₃ | F |
| CH₂CH₂OCH₃ | Cl | CF₃ | F |
| | | | |
| (CH₂)₃OCH₃ | F | I | F |
| (CH₂)₃OCH₃ | Cl | I | F |
| (CH₂)₃OCH₃ | F | Br | F |
| (CH₂)₃OCH₃ | Cl | Br | F |
| (CH₂)₃OCH₃ | F | CH₃ | F |
| (CH₂)₃OCH₃ | Cl | CH₃ | F |
| (CH₂)₃OCH₃ | F | CF₃ | F |
| (CH₂)₃OCH₃ | Cl | CF₃ | F |
| | | | |
| CH₂CH₂OEt | F | I | F |
| CH₂CH₂OEt | Cl | I | F |
| CH₂CH₂OEt | F | Br | F |
| CH₂CH₂OEt | Cl | Br | F |
| CH₂CH₂OEt | F | CH₃ | F |
| CH₂CH₂OEt | Cl | CH₃ | F |
| CH₂CH₂OEt | F | CF₃ | F |
| CH₂CH₂OEt | Cl | CF₃ | F |
| | | | |
| 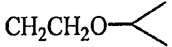 | F | I | F |
| 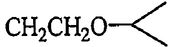 | Cl | I | F |
| 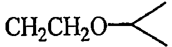 | F | Br | F |
| 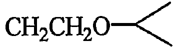 | Cl | Br | F |
| 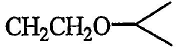 | F | CH₃ | F |
| 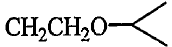 | Cl | CH₃ | F |
| 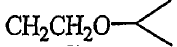 | F | CF₃ | F |
| 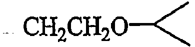 | Cl | CF₃ | F |
| 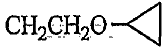 | F | I | F |
| 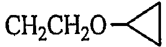 | Cl | I | F |
| 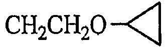 | F | Br | F |
| 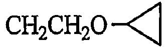 | Cl | Br | F |
| 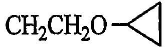 | F | CH₃ | F |
| 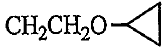 | Cl | CH₃ | F |
| 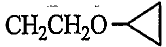 | F | CF₃ | F |
| 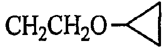 | Cl | CF₃ | F |
| | | | |
| CH₂CH₂CH₂OEt | F | I | F |
| CH₂CH₂CH₂OEt | Cl | I | F |
| CH₂CH₂CH₂OEt | F | Br | F |
| CH₂CH₂CH₂OEt | Cl | Br | F |
| CH₂CH₂CH₂OEt | F | CH₃ | F |
| CH₂CH₂CH₂OEt | Cl | CH₃ | F |
| CH₂CH₂CH₂OEt | F | CF₃ | F |
| CH₂CH₂CH₂OEt | Cl | CF₃ | F |
| | | | |
| 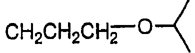 | F | I | F |
| 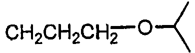 | Cl | I | F |
| 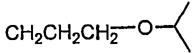 | F | Br | F |
| 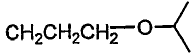 | Cl | Br | F |
| 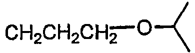 | F | CH₃ | F |
| 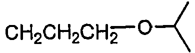 | Cl | CH₃ | F |
| 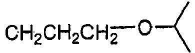 | F | CF₃ | F |
| 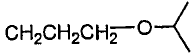 | Cl | CF₃ | F |
| | | | |
| 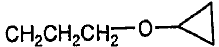 | F | I | F |
| 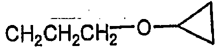 | Cl | I | F |
| 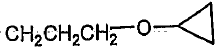 | F | Br | F |
| 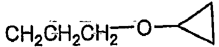 | Cl | Br | F |
| 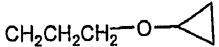 | F | CH₃ | F |
| 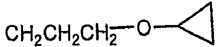 | Cl | CH₃ | F |
| 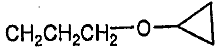 | F | CF₃ | F |
| 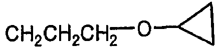 | Cl | CF₃ | F |
| | | | |
| 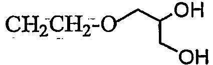 | F | I | F |
| 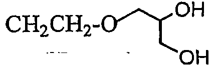 | Cl | I | F |
| 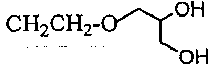 | F | Br | F |
| 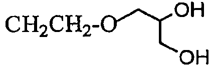 | Cl | Br | F |
| 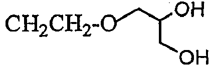 | F | CH₃ | F |
| 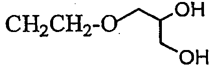 | Cl | CH₃ | F |
| 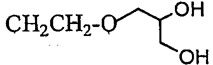 | F | CF₃ | F |
| 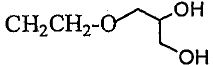 | Cl | CF₃ | F |
| | | | |
| 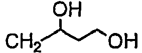 | F | I | F |
| 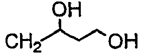 | Cl | I | F |
| 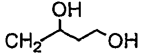 | F | Br | F |
| 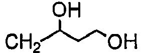 | Cl | Br | F |
| 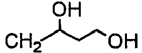 | F | CH₃ | F |
| 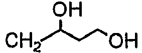 | Cl | CH₃ | F |
| 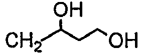 | F | CF₃ | F |
| 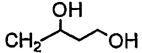 | Cl | CF₃ | F |
| | | | |
| 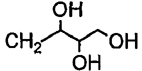 | F | I | F |
| 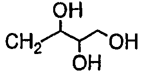 | Cl | I | F |
| 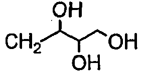 | CH₃ | I | F |
| 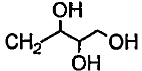 | F | Br | F |
| 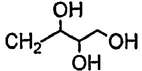 | Cl | Br | F |
| 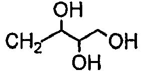 | CH₃ | Br | F |
| 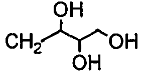 | F | CH₃ | F |
| 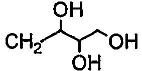 | Cl | CH₃ | F |
| 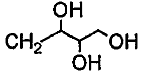 | CH₃ | CH₃ | F |
| 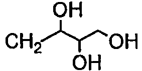 | F | C≡CH | F |
| 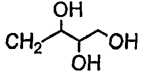 | F | SCH₃ | F |
| 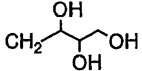 | F | CH₂CH₂CH₃ | F |
| 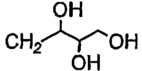 | Cl | CH₂CH(OH)CH₃ | F |
| 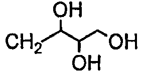 | F | CH(CH₃)₂ | F |
| 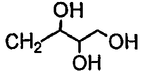 | Cl | CF₃ | F |

Tables 4a and 4b present species according to formula I, where A = R_{1d}, except where R_{1d} is heterocyclic; compounds of that type are presented in Table 5. Each line in the table corresponds to five species which differ only at position Y.

**TABLE 4a**

| **SPECIES CORRESPONDING TO A = R_{1d}** | | | |
|---|---|---|---|
| 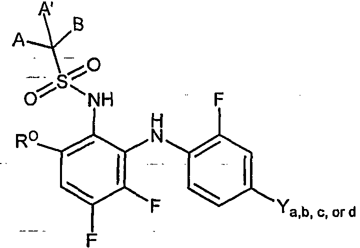 | | | |
| Yₐ**=** CH₃; Y_{b} = Br; Y_{c} = I; Y_{d} = Cl; | | | |

| **CMPD #** | **A, A'** | **B** | **R^{o}** |
|---|---|---|---|
| **1 (a-d)** | H, H | H | OCH₃ |
| **2 (a-d)** | H, H | H | NHCH₃ |
| **3 (a-d)** | H, H | H | CH₂CH₃ |
| **4 (a-d)** | H, H | H | CH₂CH=CH₂ |
| **5 (a-d)** | H, H | H | CN |
| **6 (a-d)** | H, H | H | CF₃ |
| **7(a-d)** | H, H | H | F |
| **8(a-d)** | H, H | H | C₆H₆ |
| **9(a-d)** | H, H | -CH₂CH(OH)CH₂OH | OCH₃ |
| **10(a-d)** | H, H | -CH₂CH(OH)CH₂OH | NHCH₃ |
| **11(a-d)** | H, H | -CH₂CH(OH)CH₂OH | CH₂CH₃ |
| **12(a-d)** | -((CH₂)₂- | -CH₂(C₃H₅) | OCH₃ |
| **13(a-d)** | -((CH₂)₂- | -CH₂(C₃H₅) | NHCH₃ |
| **14(a-d)** | -(CH₂)₂- | -CH₂(C₃H₅) | CH₂CH₃ |
| **15(a-d)** | -(CH₂)₂- | CH₃ | F |
| **16(a-d)** | -(CH₂)₂- | -CH₂CH₂OH | F |
| **17(a-d)** | -(CH₂)₂- | -(CH₂)₂CH(OH)CH₂OH | F |
| **18(a-d)** | CH₃, H | -(CH₂)₂CH(OH)CH₂OH | F |
| **19(a-d)** | -(CH₂)₂- | CH₃ | OCH₃ |
| **20(a-d)** | -(CH₂)₂- | -CH₂CH₂OH | OCH₃ |
| **21(a-d)** | -(CH₂)₂- | -(CH₂)₂CH(OH)CH₂OH | OCH₃ |
| **22(a-d)** | CH₃, H | (CH₂)₂CH(OH)CH₂OH | OCH₃ |
| **23(a-d)** | -(CH₂)₂- | CH₃ | H |
| **24(a-d)** | -(CH₂)₂- | CH₂CH₂OH | H |
| **25(a-d)** | -(CH₂)₂- | -(CH₂)₂CH(OH)CH₂OH | H |
| **26(a-d)** | CH₃, H | -(CH₂)₂CH(OH)CH₂OH | H |

**TABLE 2B**

| **CMPD #** | A, A' | B | R^{o} |
|---|---|---|---|
| **1(a-d)** | H, H | H | 2-furanyl |
| **2(a-d)** | H, H | H | 1,2,3 triazolyl-4-yl |
| **3(a-d)** | H, H | H | 4-imidazolyl |
| **4(a-d)** | H, H | H | 2-furanyl |
| **5(a-d)** | H, H | H | 1,2,3 triazolyl-4-yl |
| **6(a-d)** | H, H | H | 4-imidazolyl |
| **7(a-d)** | H, H | -CH₂CH(OH)CH₂OH | 2-furanyl |
| **8(a-d)** | H, H | -CH₂CH(OH)CH₂OH | 1,2,3 triazolyl-4-yl |
| **9(a-d)** | H, H | -CH₂CH(OH)CH₂OH | 4-imidazolyl |
| **10(a-d)** | -(CH₂)₂- | -CH₂(C₃H₅) | 2-furanyl |
| **11(a-d)** | -(CH₂)₂- | -CH₂(C₃H₅) | 1,2,3 triazolyl-4-yl |
| **12(a-d)** | -(CH₂)₂- | -CH₂(C₃H₅) | 4-imidazolyl |
| **13(a-d)** | -(CH₂)₂- | CH₃ | 4-thiazolyl |
| **14(a-d)** | -(CH₂)₂- | -CH₂CH₂OH | 4-thiazolyl |
| **15(a-d)** | -(CH₂)₂- | -(CH₂)₂CH(OH)CH₂OH | 4-thiazolyl |
| **16(a-d)** | CH3, H | -(CH₂)₂CH(OH)CH₂OH | 4-thiazolyl |
| **17(a-d)** | -(CH₂)₂- | CH₃ | 2-oxazolyl |
| **18(a-d)** | -((CH₂)₂- | -CH₂CH₂OH | 2-oxazolyl |
| **19(a-d)** | -(CH₂)₂- | -(CH₂)₂CH(OH)CH₂OH | 2-oxazolyl |
| **20(a-d)** | CH₃, H | -(CH₂)₂CH(OH)CH₂OH | 2-oxazolyl |

### Synthetic Procedures and Examples

Compounds of this invention can be prepared by a variety of methods. The procedures below are intended to illustrate those methods, and the examples given are intended to illustrate the scope of this invention. Neither the methods not the examples should be construed as limiting the invention in any way.

**I.** The preparation of compound of formula VI is outlined below

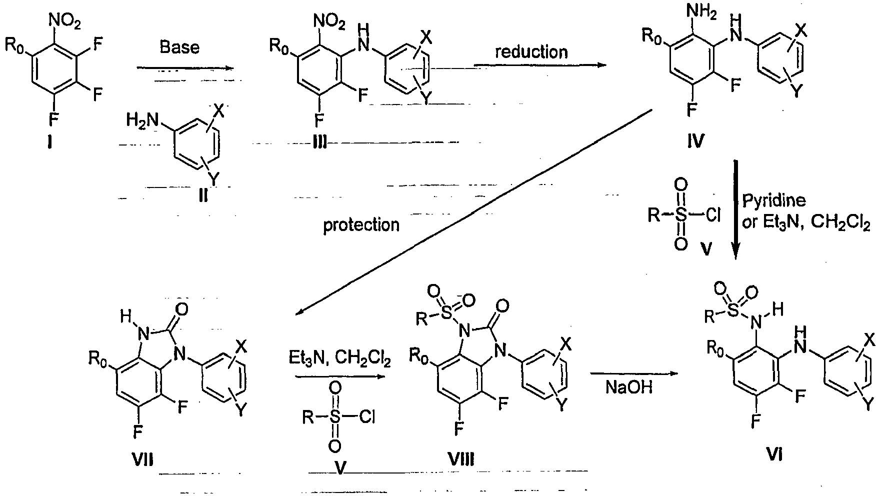

Scheme I above illustrates the preparation of sulfonamide derivatives of formula VI. 1,2 Diamine derivative (formula IV) can be easily prepared in two steps from the desired nitro derivatives (formula I). Compounds of formula IV can be reacted with the sulfonyl chloride derivatives (formula V, see next scheme) to form the desired sulfonamide. Alternatively, the 1,2 diamine derivatives IV can be protected to for an imidazolidone (formula VII), before being reacted with the corresponding sulfonyl chloride. Deprotection of the 1,2 diamine VIII under basic conditions provided the desired material VI.

**II.** The general route to synthesis compound of general formula V is outlined below

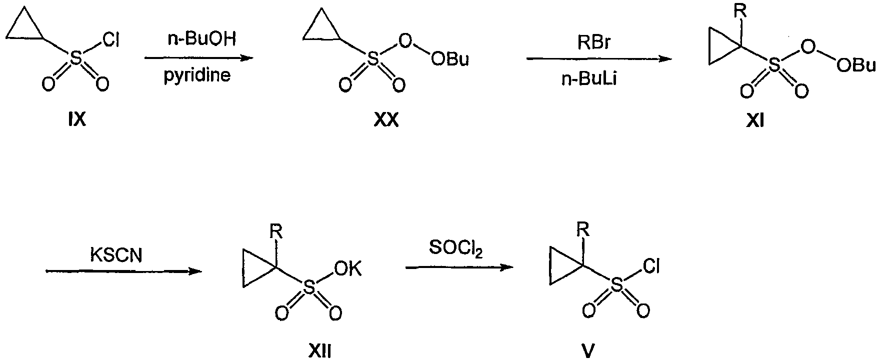

Scheme II above shows one example of the preparation of complex sulfonyl chloride. Compound XX can be synthesized from IX, alkylated, and converted to the potassium salt XII. Treatment of the salt with SOCl₂ or POCl₃ affords the desired compounds. Other more specific procedures to prepare unique sulfonyl chloride derivatives are reported in the experimental section.

The following examples are provided so that the invention might be more fully understood. These examples are illustration of the scope of the invention only and should not be construed as limiting the invention in any way.

### Typical procedures for the synthesis of sulfonamides:

**Procedure A:** To a solution of the amine (1 eq) in anhydrous dichloromethane (3 mL/ mmole) was added anhydrous triethylamine (5 eq). To this solution was added the sulfonyl chloride (1 eq) and the solution was stirred at room temperature for 16 h. The solvent was evaporated and the residue was purified by flash column chromatography on silica.

**Procedure B:** To a stirred solution of the amine (1 eq) in anhydrous pyridine (5ml/mmole) was added the sulfonyl chloride (1 - 5 eq). The reaction mixture was stirred at 40 °C for 48 hours. The reaction mixture was partitioned with water and EtOAc. The organic layer was washed with brine, dried (MgSO₄) and concentrated under reduced pressure. The residue was purified by flash column chromatography on silica.

### Example 1

### N-(3,4-difluoro-2-(2-fluoro-4-iodophenylamino)phenyl)methanesulfonamide:

### Step A: 2,3-Difluoro-N-(2-fluoro-4-iodophenyl)-6-nitroaniline:

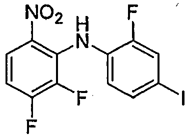

To a solution of 2-fluoro-4-iodoaniline (11.40 g, 47 mmol) in 100 ml anhydrous THF at 0 °C, 47 ml of a 1M solution of LHMDS in THF (47 mmol) was added dropwise. The color of the solution turned dark purple. The solution was transferred *via* cannula to a dripping funnel, and the solution (containing the amine free base) was added in small portions to a solution of 2,3,4-trifluoronitrobenzene (8.321 g, 47.0 mmol) in anhydrous THF (50 ml) at 0°C. After completion of addition the mixture was stirred under argon at room temperature for 15 hours. The volume of the solvent was reduced, followed by extraction using ethyl acetate and brine. The organic layer was dried over sodium sulfate, the solvent was removed, and the obtained dark oil was purified by flash chromatography (EtOAc / hexane 1:5, R_{f}= 0.58) yielding the crude product, which became a brown solid upon drying *in vacuo* (yield: 6.23 g, 33.6%). *m*/*z* = 393 [M-1]⁻.

### Step B: 5,6-Difluoro-N1-(2-fluoro-4-iodophenyl)benzene-1,2-diamine

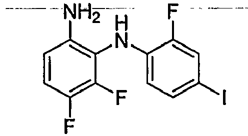

To a solution of nitro-diarylamine (6.23 g, 15.8 mmol) in 300 ml ethanol was added iron powder (13.74 g, 246 mmol) and ammonium chloride (13.59 g, 254 mmol) and the mixture was heated with stirring at 100 °C oil bath temperature for 14 hours. It was filtered and the residue washed two times with ethanol. The ethanol was removed *in vacuo,* and the residue was extracted using ethyl acetate / 1M NaOH solution. During the extraction, more precipitate was formed which was filtered and discarded. The combined organic layers were washed with brine and dried over sodium sulfate. The solvent was removed, and the crude product was recrystallized from CHCl₃ / hexane (1:50). The product was obtained as brown needles (2.094 g, 66%,). R_{f}= 0.44 (EtOAc / Hex 1:3). ¹H-NMR (500 MHz, CDCl₃): δ = 7.40-7.38 (dd, 1H, *J*= 11.3 Hz, *J=* 1.5 Hz). 7.25-7.23 (d, 1H, *J=* 8.5 Hz), 6.97-6.92 (q, 1H, *J= 9* Hz), 6.51-6.48 (m, 1H), 6.24-6.21 (t, 1H, *J =* 9 Hz), 5.3 (s, 1H, NH, br), 3.80 (s, 2H, NH₂, br); LRMS (ESI): *m*/*z* = 365 [M+H]⁺.

### Step C: N-(3,4-difluoro-2-(2-fluoro-4-iodophenylamino)phenyl) methanesulfonamide:

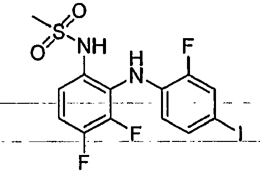

According to the general procedure A, 5,6-difluoro-N1-(2-fluoro-4-iodophenyl)benzene-1,2-diamine was reacted with methanesulfonyl chloride to obtain the desired product. ¹H NMR: (500 MHz, CDCl₃): δ = 7.38-7.37 (d, 1H), 7.35-7.34 (m, 1H), 7.27-7.26 (m, 1H), 7.20-7.0 (q, 1H), 6.68 (s, 1H, br), 6.15-6.12 (q, 1H), 5.65 (s, 1H, br), 2.95 (s, 3H); *m*/*z =* 441 [M-1]⁻.

### Example 2

### N-(3,4-difluoro-2-(2-fluoro-4-iodophenylamino)phenyl)cyclopropanesulfonamide:

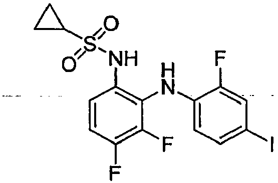

According to the general procedure A, 5,6-difluoro-N1-(2-fluoro-4-iodophenyl) benzene-1,2-diamine was reacted with cyclopropanesulfonyl chloride to obtain the desired product. ¹H NMR: (500 MHz, CDCl₃): δ = 7.38-7.37 (d, 1H), 7.35-7.34 (m, 1H), 7.27-7.26 (m, 1H), 7.20-7.0 (q, 1H), 6.68 (s, 1H, br), 6.15-6.12 (q, 1H), 5.65 (s, 1H, br), 3.25-3.20 (m, 1H), 2.4-2.3 (m, 2H), 2.0-1.8 (m, 2H); *m*/*z* = 467 [M-1]⁻.

### Example 3

### N-(3,4-difluoro-2-(2-fluoro-4-iodophenylamino)phenyl)propane-2-sulfonamide:

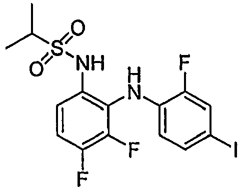

According to the general procedure A, 5,6-difluoro-N1-(2-fluoro-4-iodophenyl)benzene-1,2-diamine was reacted with isopropylsulfonyl chloride to obtain the desired product. Yield: 39%. ¹H-NMR (500 MHz, CDCl₃): δ = 7.50-7.43 (m, 1H), 7.35-7.34 (m, 1H), 7.27-7.26 (m, 1H), 7.15-7.09 (q, 1H, *J=* 1.6 Hz), 6.62 (s, 1H, br), 6.22-6.18 (q, 1H, *J=1.5* Hz), 5.65 (s, 1H, br), 3.30-3.28 (m, 1H), 1.38-1.37 (d, 6H, *J*=1.2 Hz); *m*/*z =* 469 [M-1]⁻.

### Example 4

### N-(3,4-difluoro-2-(2-fluoro-4-iodophenylamino)phenyl)butane-1-sulfonamide:

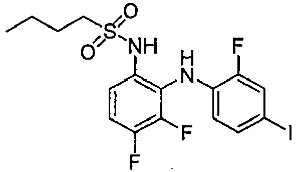

According to the general procedure A, 5,6-difluoro-N1-(2-fluoro-4-iodophenyl)benzene-1,2-diamine was reacted with n-butylsulfonyl chloride to obtain the desired product. Yield: 55%. ¹H-NMR (500 MHz, CDCl₃): δ = 7.50-7.43 (m, 1H), 7.35-7.34 (m, 1H), 7.27-7.26 (m, 1H), 7.15-7.09 (q, 1H, *J=* 1.6 Hz), 6.62 (s, 1H, br), 6.22-6.18 (q, 1H, *J=* 1.5 Hz), 5.65 (s, 1H, br), 3.06-3.031 (t, 2H, *J=* 1.4 Hz), 1.75-1.71 (m, 2H), 1.38-1.36 (m, 2H), 0.87-0.86 (t, 3H, *J* = 1.3 Hz); *m*/*z* = 483 [M-1]⁻.

### Example 5

### N-(3,4-difluoro-2-(2-fluoro-4-iodophenylamino)phenyl)-2,2,2-trifluoro ethane sulfonamide:

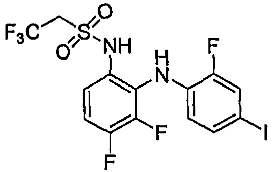

According to the general procedure A, 5,6-difluoro-N1-(2-fluoro-4-iodophenyl)benzene-1,2-diamine was reacted with 1,1,1-trifluoroethylsulfonyl chloride to obtain the desired product. Yield: 28%. *m*/*z =* 509 [M-1]⁻.

### Example 6

### N-(3,4-difluoro-2-(2-fluoro-4-iodophenylamino)phenyl)butane-2-sulfonamide:

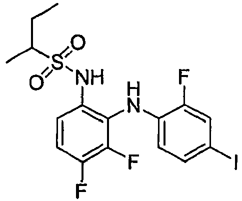

According to the general procedure A, 5,6-difluoro-N1-(2-fluoro-4-iodophenyl)benzene-1,2-diamine was reacted with sec-butylsulfonyl chloride to obtain the desired product. Yield: 22%. ¹H-NMR (500 MHz, MeOH[d4]): δ = 7.60-7.40 (m, 3H), 7.18-7.00 (q, 1H), 6.55-6.45 (m, 1H), 3.55-3.50 (m, 1H), 2.20-2.00 (m, 1H), 1.80-1.60 (m, 1H), 1.43-1.40 (d, 3H), 1.06-1.04 (t. 3H); *m*/*z =* 483 [M-1]⁻.

### Example 7

### N-(3,4-difluoro-2-(2-fluoro-4-iodophenylamino)phenyl)-N-methyl cyclopropane sulfonamide:

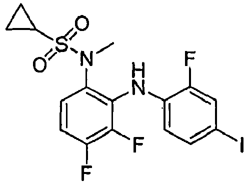

To a solution of N-(3,4-difluoro-2-(2-fluoro-4-iodophenylamino)phenyl)cyclopropane-sulfonamide (see Example 2) (283.9 mg, 0.61 mmol) in 3 ml anhydrous THF was added at -78 °C a 1M solution of LHMDS (0.6 ml, 0.6 mol) and the solution was stirred for 10 min at this temperature. Then, methyl iodide (0.8 ml, 1.824 g, 12.9 mmol) was added and the mixture was warmed to room temperature and stirred for 7 h. The solvent was removed and the residue extracted using EtAc and brine. The organic fractions were dried using Na₂SO₄ and the solvent was removed. The obtained crude product was purified using flash-column chromatography (Si, EtAc/Hexanes 1:2, R_{f}= 0.45). Yield: 205 mg, 70%). ¹H-NMR (500 MHz, CDCl₃): δ = 7.41-7.39 (d, 1H, *J=* 10 Hz), 7.30-7.29 (d, 1H, *J=* 8.0 Hz), 7.23-7.20 (m, 1H), 6.98-6.93 (q, 1H, *J=* 8.5 Hz), 6.60 (s, 1H, br), 6.51-6.47 (m, 1H), 3.23 (s, 3H), 2.46-2.42 (m, 1H), 1.19-1.16 (m, 2H), 1.04-1.02 (m, 2H); *m*/*z* = 481 [M-1]⁻.

### Example 8

### 1-Chloro-N-(3,4-difluoro-2-(2-fluoro-4-iodophenylamino)phenyl) methane sulfonamide:

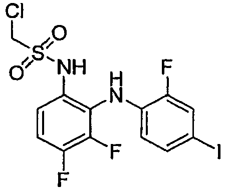

According to the general procedure A, 5,6-difluoro-N1-(2-fluoro-4-iodophenyl)benzene-1,2-diamine was reacted with chloromethanesulfonyl chloride to obtain the desired product. *m*/*z =* 475 [M-1]⁻.

### Example 9

### N-(3,4-difluoro-2-(2-fluoro-4-iodophenylamino)phenyl)-2-methylpropane-2-sulfonamide:

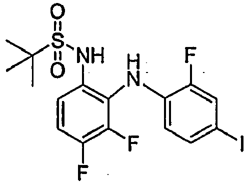

According to the general procedure B, 5,6-difluoro-N1-(2-fluoro-4-iodophenyl)benzene-1,2-diamine was reacted with 2-methylpropane-2-sulfonyl chloride (synthesized according to the literature procedure) to obtain the desired product. ¹H NMR (300 MHz, CDCl₃): δ 7.50 (m, 1H), 7.43 (dd, *J*= 1.8 & 10.5 Hz, 1H), 7.28 (br s, 1H), 7.10 (dd, *J*= 9.0 & 17.7 Hz, 1H), 6.48 (br s, D₂O exchangeable, 1H), 6.19 (t, *J=* 7.8 & 9.6 Hz, 1H), 5.58 (br s, D₂O exchangeable, 1H), 1.39 (s, 9H); *m*/*z =* 383 [M-1]⁻.

### Example 10

### N-(3,4-difluoro-2-(2-fluoro-4-iodophenylamino)phenyl)cyclopentanesulfonamide:

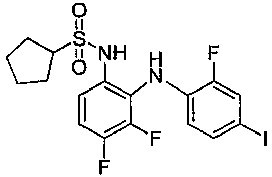

According to the general procedure B, 5,6-difluoro-N1-(2-fluoro-4-iodophenyl)benzene-1,2-diamine was reacted with cyclopentanesulfonyl chloride to obtain the desired product ¹H NMR (300 MHz, CDCl₃): δ 7.42 (dd, *J*= 2.1 & 10.5 Hz, 1H), 7.36 (ddd, *J=* 2.4, 4.8, & 9.3 Hz, 1H), 7.25 (m, 2H), 7.10 (dd, *J =* 9.6 & 17.7 Hz, 1H), 6.67 (br s, D₂O exchangeable, 1H), 6.20 (dt, *J*= 1.5, 8.4 & 17.4 Hz, 1H), 3.53 (p, 1H), 1.80 (m, 8H); *m*/*z* = 495 [M-1]⁻.

### Example 11

### N-(3,4-difluoro-2-(2-fluoro-4-iodophenylamino)phenyl)cyclohexanesulfonamide:

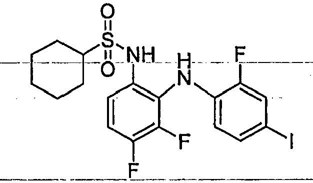

According to the general procedure B, 5,6-difluoro-N1-(2-fluoro-4-iodophenyl)benzene-1,2-diamine was reacted with cyclohexanesulfonyl chloride to obtain the desired product. ¹H NMR (300 MHz, CDCl₃): δ 7.43 (dd, *J* = 1.5 & 10.2 Hz, 1H), 7.37 (ddd, *J =* 2.4, 4.8 & 9.6 Hz, 1H), 7.27 (m, 1H), 7.11 (dd, *J =* 9.3 & 18.0 Hz, 1H), 6.64 (br s, 1H), 6.18 (dt, *J*= 1.5, 9.0 & 17.4 Hz, 1H), 5.63 (br s, 1H), 2.95 (triplet of triplet, 2.10-1.16 (m, 10H); *m*/*z =* 509 [M-1]⁻.

### Example 12

### N-(3,4-difluoro-2-(2-fluoro-4-iodophenylamino)phenyl)-1-methylcyclopropane-1-sulfonamide:

### Step A: n-Butyl 3-chloro-1-propanesulfonate:

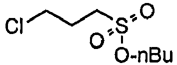

Triethylamine (28 ml, 200 mmol) in CH₂Cl₂ (50 ml) was slowly added to an ice-cooled solution of 3-chloro-1-propanesulfonyl chloride (36.6g, 200 mmol) and 1-butanol (18.4 g, 240 m mol) in CH₂Cl₂ (250 ml) and stirring was continued for 16h. The mixture was diluted with CH₂Cl₂ (200 ml), washed (aqueous HCl) and dried (MgSO₄) and the solvent was evaporated to obtain the titled product 1 (40.85 g, 95%) in crude form as slightly yellow oil which was used for the next reaction without further purification. ¹H NMR (CDCl₃₎) δ 0.94 (t, J = 7.5 Hz, 3H), 1.44 (sextet, 2H), 1.72 (quintet, 2H), 2.31 (quintet, 2H), 3.27(t, J = 6.9 Hz, 2H), 3.68 (t, J = 6.3 Hz), 4.23 (t, J = 6.6 Hz, 2H).

### Step B: 1-Butyl cyclopropanesulfonate:

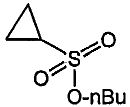

Solutions of 1-butyl 3-chloro-1-propanesulfonate (4.6 g, 21.39 mmol in 25 ml THF) and of butyllithium (14.7 ml, 23.53 mmol, 1.6M, THF) were simultaneously added to THF (150 ml) at -78 °C under nitrogen atmosphere. The solution was allowed to warm to 0 °C and then quenched with water (2 ml). The volatiles evaporated under reduced pressure and the residue extracted with CH₂Cl₂ (150 ml). The extract was washed with water and dried (MgSO₄) and evaporated to give crude desired product (3.23 g, 78.22%) in almost pure form as pale yellow oil which was used for next step without further purification. ¹H NMR (300 MHz, CDCl₃) δ 0.94 (t, J = 7.5 Hz, 3H), 1.07 (m, 2H), 1.25 (m, 2H), 1.45(sextet, 2H), 1.74(quintet, 2H), 2.45 (heptet, 1H), 4.23 (t, J = 6.6 Hz, 2H).

### Step C: Butyl 1-Methyl-cyclopropanesulfonate:

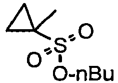

To a solution of 1-Butyl cyclopropanesulfonate (1 g, 5.58 mmol) in THF (15 ml) butyllithium solution (3.84 ml, 6,14 mmol, 1.6M, THF) was slowly added at -78 °C under nitrogen atmosphere. After 15 minutes MeI (0.72 ml, 11.16 mmol) was added and the solution was allowed to warm to 0 °C and quenched with water (1 ml). The volatiles evaporated under reduced pressure and the residue extracted with CH₂Cl₂ (100 ml). The extract was washed with water, dried (MgSO₄) and evaporated. The residue was purified over silica gel chromatography (eluants: hexane/ CH₂Cl₂) to obtain the titled product (0.59 g, 55.0%) as a colorless oil. ¹H NMR (300 MHz, CDCl₃₎) δ 0.84 (m, 2H), 0.95 (t, J = 7.2 Hz, 3H), 1.43 (m, 4H), 1.53 (s, 3H), 1.74(m, 2H), 4.21 ((t, J = 6.6 Hz, 2H).

### Step D: 1-Potassium 1-Methyl-cyclopropanesulfonate:

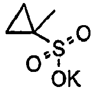

A mixture of 1- Butyl 1-Methyl-cyclopropanesulfonate (0.386 g, 2 mmol) and potassium thiocyanate (0.194 g, 2 mmol) in DME (5 ml) and water (5 ml) was refluxed for 16h. The volatiles were evaporated to obtain the crude sulfonate (0.348g, quantitative) which was dried under vacuum at 50°C for 16h. The crude product was used in the next reaction without further purification. ¹H NMR (300 MHz, D₂O) δ 0.56 (t, J = 6.3 Hz, 2H), 0.96 (t, J = 6.3 Hz, 2H), 1.26 (s, 3H).

### Step E: 1-Methyl-cyclopropanesulfonylchloride:

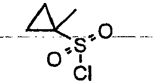

A solution of 1-potassium 1-methyl-cyclopropanesulfonate (0.348 g, 2 mmol), thionyl chloride (5 ml) and DMF (5 drops) was refluxed at 60 °C for 16h. The volatiles evaporated under reduced pressure and the residue extracted with CH₂Cl₂ (50 ml). The extract was washed with water, dried (MgSO₄) and evaporated to obtain the crude product as yellow gummy oil which was used in the next reaction without further purification.

### Step F: N-(3,4-difluoro-2-(2-fluoro-4-iodophenylamino)phenyl)-1-methylcyclopropane-1-sulfonamide:

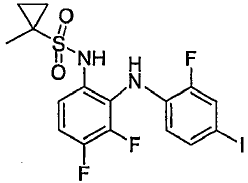

According to the general procedure B, 5,6-difluoro-Nl-(2-fluoro-4-iodophenyl)benzene-1,2-diamine was reacted with 1-methyl-cyclopropanesulfonylchloride to obtain the desired product. ¹H NMR (300 MHz, CDCl₃): δ 7.42 (dd, *J*= 1.8 & 10.5 Hz, 1H), 7.36 (ddd, *J=* 2.4, 4.5 & 9.0 Hz, 1H), 7.27 (d, *J*= 6.0 Hz, 1H), 7.07 (dd, *J=* 9.3 & 17.7 Hz, 1H), 6.24 (dt, *J*= 2.1, 8.7 & 17.4 Hz, 1H), 5.86 (br s, 1H), 1.43 (s, 3H), 1.33 (t, *J*= 5.4 Hz, 2H), 0.75 (dd, *J*= 5.1 & 6.3 Hz, 2H); *m*/*z* = 481 [M-1]⁻.

### Example 13

### N-(3,4-difluoro-2-(2-fluoro-4-iodophenylamino)phenyl)-1-(2,3-dihydroxypropyl) cyclopropane-1-sulfonamide:

### Step A: Butyl cyclopropanesulfonate:

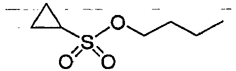

Cyclopropanesulfonyl chloride (5 g, 35 mmole, 1 eq) was dissolved in an excess BuOH (20 ml), the reaction mixture was cooled at -10°C and pyridine (5.8 mL, 70 mmole, 2 eq) was slowly added dropwise. The mixture was slowly warmed at room temperature and stirred overnight. The solvent was removed under reduced pressure and the resulting white solid was dissolved in CHCl₃. The organic phase was washed with water, brine and dried (MgSO4) and concentrated to give an oil (4.8 g, 24.9 mmole, 71%). ¹H NMR (300 MHz, CDCl₃): δ 4.25 (t, 2H), 2.46 (m, 1H), 1.74 (m, 2H), 1.45 (m, 2H), 1.25 (dd, 2H), 1.09 (dd, 2H), .93 (t, 3H).

### Step B: Butyl 1-allylcyclopropane-1-sulfonate:

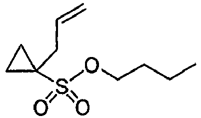

To a solution of 1-butyl cyclopropanesulfonate (4.8 g, 24.9 mmole) in THF at -78°C was added simultaneously butyllithium solution (15. 6 ml, 24.9 mmol, 1.6M, THF) and allyl iodide (24.9 mmole) under nitrogen atmosphere. The reaction mixture was stirred 2 hours at-78°C and 3 hours at room tempoerature. The volatiles were evaporated under reduced pressure and the residue extracted with CH₂Cl₂ (100 ml). The extract was washed with water, dried (MgSO₄) and evaporated. The residue was purified over silica gel chromatography (eluants: hexane/ CH₂Cl₂) to obtain the titled product (3.75 g, 69.0%) as a colorless oil. ¹H NMR (300 MHz, CDCl₃): δ 5.6 (m, 1H), 5.13-5.08 (t, 2H), 4.21 (t, 2H), 2.65 (d, 2H), 1.7 (m, 2H), 1.4 (m, 4H), 93(m, 5H).

### Step C: Potassium 1-allylcyclopropane-1-sulfonate:

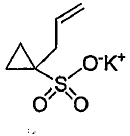

A mixture of 1- butyl 1-methyl-cyclopropanesulfonate (3.75 g, 17.2 mmol) and potassium thiocyanate (1.7 g, 17.2 mmol) in DME (20 ml) and water (20 ml) was refluxed for 16h. The volatiles were evaporated to obtain the crude sulfonate (3.44g, quantitative) which was dried under vacuum at 50°C for 16h. The crude product was used in the next reaction without further purification. ¹H NMR (CDCl₃): δ 5.6 (m, 1H), 4.91-4.85 (dd, 2H), 2.471-2.397 (d, 2H), 0.756 (m, 2H), 0.322 (m, 2H).

### Step D: 1-allylcyclopropane-1-sulfonyl chloride:

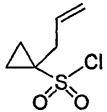

A solution of potassium 1-allylcyclopropane-1-sulfonate (3.44 g, 17.2 mmol), thionyl chloride (10 ml) and DMF (5 drops) was refluxed at 60 °C for 16h. The volatiles evaporated under reduced pressure and the residue extracted with CH₂Cl₂ (50 ml). The extract was washed with water, dried (MgSO₄) and evaporated to obtain the crude product as yellow gummy oil which was washed with hexane and used in the next reaction without further purification (2.7 g, 15 mmole, 87%). ¹H NMR (300 MHz, CDCl₃): δ 5.728 (m, 1H), 5.191 (t, 2H), 2.9 (d, 2H), 0.756 (m, 2H), 0.322 (m, 2H).

### Step E: 1-allyl-N-(3,4-difluoro-2-(2-fluoro-4-iodophenylamino)phenyl)cyclopropane-1-sulfonamide:

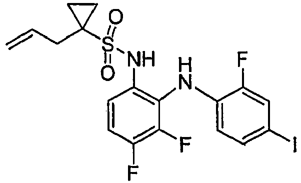

According to the general procedure B, 5,6-difluoro-N1-(2-fluoro-4-iodophenyl)benzene-1,2-diamine was reacted with 1-allylcyclopropane-1-sulfonyl chloride to obtain the desired product. *m*/*z* = 507 [M-1]⁻.

### Step F: N-(3,4-difluoro-2-(2-fluoro-4-iodophenylamino)phenyl)-1-(2,3-dihydroxypropyl)cyclopropane-1-sulfonamide:

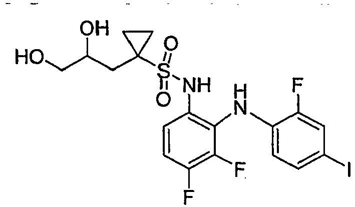

1-Allyl-N-(3,4-difluoro-2-(2-fluoro-4-iodophenylamino) phenyl)cyclopropane-1-sulfonamide (0,77 g, 1.52 mmole) and 4-methylmorpholine N-oxide (0,18 g, 1.52 mmole) were dissolved in THF (50 mL). Osmium tetroxide was added at room temperature (0.152 mmole, 0.965 mL, 4% in H₂O) and the reaction mixture was stirred at room temperature for 16 hours. EtOAc was added, the organic phase was washed with water, dried (MgSO₄) and concentrated under reduced pressure. The residue was purified over silica gel chromatography (eluants: EtOAc/MeOH) to obtain the titled product (0.65 g, 79%). ¹H NMR (300 MHz, CDCl₃ + D₂O): δ 7.38 (dd, *J*= 1.8 & 10.5 Hz, 1H), 7.36 (ddd, *J=* 2.4, 5.1 & 9.3 Hz, 1H), 7.25 (d, *J=* 8.7 Hz, 1H), 7.02 (dd, *J=* 9.0 & 17.7 Hz, 1H), 6.27 (dt, *J=* 3.0, 8.7 & 17.4 Hz, 1H), 3.92 (m, 1H), 3.54 (dd, *J=* 3.9 & 11.1 Hz, 1H), 3.39 (dd, *J*= 6.6 & 11.1 Hz, 1H), 2.16 (dd, *J* = 9.6 & 15.9 Hz, 1H), 1.59 (d, *J =* 14.1 Hz, 1H), 1.41 (m, 1H), 1.26 (m, 1H), 0.83 (m, 2H); *m*/*z* = 542 [M-1]⁻.

### Example 14

### (S)-N-(3,4-difluoro-2-(2-fluoro-4-iodophenylamino)phenyl)-1-(2,3-dihydroxypropyl)cyclopropane-1-sulfonamide:

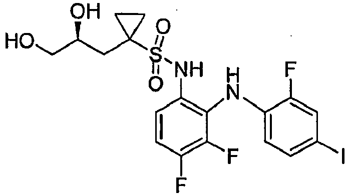

The pure S isomer was obtained by chiral HPLC separation of the racemic mixture (example 13). ¹H NMR (300 MHz, CDCl₃ + D₂O): δ 7.38 (dd, *J=* 1.8 & 10.5 Hz, 1H), 7.36 (ddd, *J* = 2.4, 5.1 & 9.3 Hz, 1H), 7.25 (d, *J* = 8.7 Hz, 1H), 7.02 (dd, *J* = 9.0 & 17.7 Hz, 1H), 6.27 (dt, *J=* 3.0, 8.7 & 17.4 Hz, 1H), 3.92 (m, 1H), 3.54 (dd, *J=* 3.9 & 11.1 Hz, 1H), 3.39 (dd, *J*= 6.6 & 11.1 Hz, 1H), 2.16 (dd, *J=* 9.6 & 15.9 Hz, 1H), 1.59 (d, *J=* 14.1 Hz, 1H), 1.41 (m, 1H), 1.26 (m, 1H), 0.83 (m, 2H); *m*/*z =* 542 [M-1]⁻.

### Example 15

### (R)-N-(3,4-difluoro-2-(2-fluoro-4-iodophenylamino)phenyl)-1-(2,3-dihydroxypropyl)cyclopropane-1-sulfonamide:

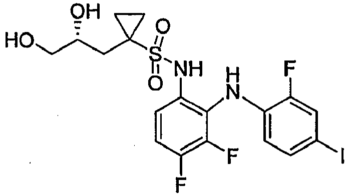

The pure R isomer was obtained by chiral HPLC separation of the racemic mixture (example 13). ¹H NMR (300 MHz, CDCl₃ + D₂O): δ 7.38 (dd, *J=* 1.8 & 10.5 Hz, 1H), 7.36 (ddd, *J*= 2.4, 5.1 & 9.3 Hz, 1H), 7.25 (d, *J*= 8.7 Hz, 1H), 7.02 (dd, *J=* 9.0 & 17.7 Hz, 1H), 6.27 (dt, *J=* 3.0, 8.7 & 17.4 Hz, 1H), 3.92 (m, 1H), 3.54 (dd, *J*= 3.9 & 11.1 Hz, 1H), 3.39 (dd, *J=* 6.6 & 11.1 Hz, 1H), 2.16 (dd, *J=* 9.6 & 15.9 Hz, 1H), 1.59 (d, *J* = 14.1 Hz, 1H), 1.41 (m, 1H), 1.26 (m, 1H), 0.83 (m, 2H); *m*/*z* = 542 [M-1]⁻.

### Example 16

### N-(3,4-difluoro-2-(2-fluoro-4-iodophenylamino)phenyl)-1-(2-hydroxyethyl)cyclopropane-1-sulfonamide:

### Step A: 2-(1-bromocyclopropyl)ethanol:

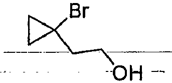

To a solution of neat diethyl zinc (3.3 ml, 3.977 g, 30 mmol) in 100 ml anhydrous DCM was added very slowly trifluoroacetic acid (2.31 ml, 3.4188 g, 30 mmol) dropwise at 0 °C. (Caution: Violent gas evolution, exothermic!). After completed addition of the TFA, the suspension was stirred for 20 min at the same temperature, followed by the addition of diiodo methane (2.45 ml, 8.134 g, 30.4 mmol). It was further stirred at 0 °C for 20 min, and then a solution of 3-bromobut-3-en-1-ol (1 ml, 1.523 g, 10.1 mmol) in 10 ml DCM was added at the same temperature. After complete addition, the mixture was warmed to room temperature and stirred for 4 hours. The mixture was quenched with 100 ml MeOH and 40 ml brine, and it was further stirred for 30 min. The solvents were reduced, and the residue extracted using CHCl₃ / aq. NH₄Cl. The organic layers were collected, washed with brine and water, and the solvent was removed to give 2-(1-bromocyclopropyl)-ethanol in sufficient purity (1.6564 g, 100%). ¹H-NMR (500 MHz, CDCl₃): δ = 3.90-3.83 (t, 2H), 1.91-1.87 (t, 2H), 1.71 (s, 1H, br), 1.14-1.09 (m, 2H), 0.83-0.79 (m, 2H).

### Step B: TBS protected 2-(1-bromocyclopropyl)ethanol:

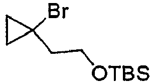

To a solution of the cyclopropyl alcohol (Step A) (1.303 g, 7.95 mmol) in 30 ml anhydrous DCM was added anhydrous pyridine (1.2 ml, 1,1736 g, 14.8 mmol) and TBSOTf (2.7 ml, 3.1077 g, 11.76 mol) and the solution was stirred at room temperature for 16 h. It was extracted with CHCl₃ / brine and the organic fraction was dried with MgSO₄. The solvent was reduced and the crude product purified using flash column chromatography (Si, CHCl₃ / hexanes 1:10, R_{f}= 0.4). Yield: 0.796 g, 36%. ¹H-NMR (500 MHz, CDCl₃): δ = 3.95-3.75 (t, 2H), 1.95-1.85 (t, 2H), 1.15-1.05 (m, 2H), 0.95-0.80 (m, 11H), 0.15-0.05 (s, 6H).

### Step C: TBS protected 2-(1-chlorosulfonylcyclopropyl)ethanol:

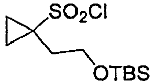

To a solution of the cyclopropyl bromide prepared in step B (1.1227 g, 4.04 mmol) in 15 ml anhydrous diethyl ether was added a 1.7 M solution oft-BuLi in pentane (4.8 ml, 8.16 mmol) at - 78 °C. The solution was stirred for 30 min at this temperature, and was then transferred via a transfer canola into a solution of freshly distilled sulfuryl chloride (0.65 ml, 1.029 g, 8.1 mmol) in 8 ml diethyl ether at - 78 °C. The yellow suspension was warmed to room temperature. The solvent was removed, and the residue was dried in vacuo to remove excessive sulfuryl chloride. Then, the residue was extracted two times with hexane, and after filtration the solvent was evaporated in vacuo to give the sulfonyl chloride in sufficient purity as a colorless oil. Yield: 870 mg (72%). ¹H-NMR (300 MHz, CDCl₃): δ = 3.95-3.85 (t, 2H), 2.35-2.25 (t, 2H), 1.80-1.70 (m, 2H), 1.45-1.38 (m, 2H), 0.90 (s, 9H), 0.10 (s, 6H).

### Step D: TBS-protected N-(3,4-difluoro-2-(2-fluoro-4-iodophenylamino)phenyl)-1-(2-hydroxyethyl)cyclopropane-1-sulfonamide:

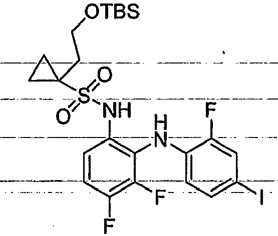

According to the general procedure B, 5,6-difluoro-N1-(2-fluoro-4-iodophenyl)benzene-1,2-diamine was reacted with the cyclpropylsulfonyl chloride prepared in step C to obtain the desired product. ¹H-NMR (300 MHz, CDCl₃): δ = 7.44-7.39 (dd, 1H), 7.32-7.24 (m, 2H), 7.1-6.98 (q, 1H), 6.34-6.24 (m, 1H), 6.16 (s, 1H, br), 3.85-3.75 (t, 2H), 2.15-2.00 (t, 2H), 1.35-1.20 (m, 2H), 0.95-0.75 (m, 11H), 0.10 (s, 6H); *m*/*z* = 625 [M-1]⁻.

### Step E: N-(3,4-difluoro-2-(2-fluoro-4-iodophenylamino)phenyl)-1-(2-hydroxyethyl)cyclopropane-1-sulfonamide:

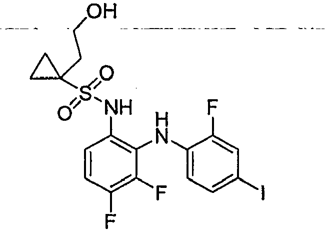

To a solution of the TBS-protected sulfonamide prepared in step D (21 mg, 0.033 mmol) in 1 ml THF was added 0.1 ml aq.1.2N HCl solution at 0 °C and the solution was stirred for 2 h. The solvents were reduced and the residue was extracted using aq. NaHCO₃ solution and EtAc. The organic fractions were dried with MgSO₄ and the volatiles were removed. The crude product was purified using flash-column chromatography (Si, CHCl₃ / MeOH 10:1, Rᵣ = 0.45) to give the pure product. Yield: 16.9 mg (100%). ¹H-NMR (300 MHz, CDCl₃): δ = 7.44-7.39 (dd, 1H), 7.32-7.24 (m, 2H), 7.1-6.98 (q, 1H), 6.34-6.24 (m, 1H), 6.16 (s, 1H, br), 3.85-3.75 (t, 2H), 2.15-2.00 (t, 2H), 1.35-1.20 (m, 2H), 0.95-0.85 (m, 2H); *m*/*z* = 511 [M-1]⁻.

### Example 17

### N-(3,4-difluoro-2-(2-fluoro-4-iodophenylamino)phenyl)-3-hydroxypropane-1-sulfonamide:

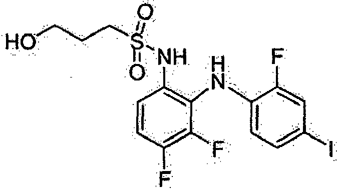

To a solution of 3-Chloro-N-(3,4-difluoro-2-(2-fluoro-4-iodophenylamino)phenyl)-propane-1-sulfonamide (69.4 mg, 0.138 mmol) in a mixture of 8 ml 1,4-dioxane and 2 ml H₂O was added KOH powder (0.674 g, 12.0 mmol) and the mixture was heated to the reflux temperature for 3 days. It was extracted using EtAc / brine, the organic fraction was dried with Na₂SO₄ and the volatiles were removed. The residue was purified using flash-column chromatography (Si, DCM / MeOH 5:1, R_{f} = 0.3). Yield: 41 mg(62%). ¹H-NMR (500 MHz, MeOH [d4]): δ = 7.38-7.21 (d, 1H), 7.23-7.21 (d, 1H), 7.06-7.00 (q, 1H), 6.52-6.50 (m, 1H). 6.17-6.13 (t, 1H), 3.30-3.27 (t, 2H), 2.86-2.83 (t, 2H), 2.05-2.00 (m, 2H); *m*/*z* = 485 [M-1]⁻.

### Example 18

### 3-Chloro-N-(3,4-difluoro-2-(2-fluoro-4-iodophenylamino)phenyl)propane-1-sulfonamide:

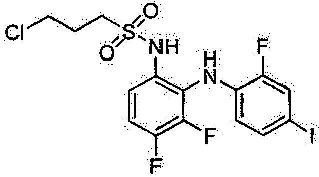

According to the general procedure A, 5,6-difluoro-N1-(2-fluoro-4-iodophenyl)benzene-1,2-diamine was reacted with 3-chloropropane-1-sulfonyl chloride to obtain the desired product. ¹H NMR (500 MHz, CDCl₃): δ = 7.39-7.38 (d, 1H), 7.35-7.34 (m, 1H), 7.27-7.26 (m, 1H), 7.10-7.0 (q, 1H), 6.63 (s, 1H, br), 6.1,5-6.11 (q, 1H), 5.60 (s, 1H, br), 3.60-3.56 (t, 2H), 3.22-3.20 (m, 2H), 2.22-2.16 (m, 2H).

### General procedure C: Synthesis of N-(3,4-difluoro-2-(2-fluoro-4-iodophenylamino)phenyl)-2-(alkylamino)ethanesulfonamide:

2-Chloro-ethanesulfonyl chloride (0.1 ml, 1 mmol) was added to a solution of 5,6-difluoro-*N*¹-(2-fluoro-4-iodophenyl)benzene-1,2-diamine (0.364 g, 1 mmol) and triethylamine (0.28 ml, 2 mmol) in CH₂Cl₂ (5 ml) and the reaction mixture was stirred at room temperature for 16h. Then it's treated with an excess amine (10 eq) either in solution or as a neat liquid. The reaction mixture stirred at room temperature for additional 6h. The reaction mixture diluted with CH₂Cl₂ (10 ml) and water (10 ml). The organic layer was sequentially washed with dil. HCl (2x20 ml, 2N) and saturated NaHCO₃ (2x10 ml) solution. Then the CH₂Cl₂ layer dried (MgSO₄) and evaporated to obtain the crude product. The impure product was purified under preparative HPLC conditions to obtain the pure products in 50-60% yield.

### Example 19

### N-(2-(4-chloro-2-fluorophenylamino)-3,4-difluorophenyl) cyclopropanesulfonamide:

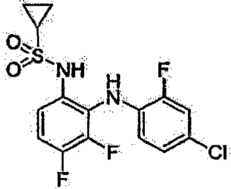

See example 1. ¹H NMR (300 MHz, CDCl₃) δ 0.85-0.95 (m, 2H), 1.05-1.15 (m, 2H), 2.2-2.4 (m, 1H), 5.8 (s, 1H), 6.3 (t, 1H), 6.6-7.4 (m, 5H); *mlz* = 375 [M-1]⁻.

### Example 20

### N-(3,4-difluoro-2-(4-iodo-2-methylphenylamino)phenyl)cyclopropanesulfonamide:

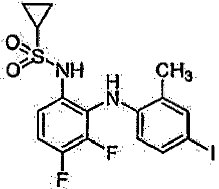

See example 1. ¹H NMR (CDCl₃) δ 0.80-1.0 (m, 2H), 1.05-1.20 (m, 2H), 1.55 (s, 3H), 2.4-2.5 (m, 1H), 5.6 (s, 1H), 6.2 (dd, 1H), 6.4 (s, 1H), 7.1 (q, 1H). 7.3-7.4 (m, 2H), 7.5 (s, 1H); *m*/*z* = 463 [M-1]⁻.

### Example 21

### N-(2-(4-tert-butyl-2-chlorophenylamino)-3,4-difluorophenyl) cyclopropanesulfonamide:

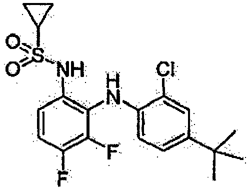

See example 1. ¹H NMR (300 MHz, CDCl₃) 8 0.9-1.0 (m, 2H), 1.05-1.20 (m, 2H), 1.3 (s, 9H), 2.4-2.5 (m, 1H), 5.8 (s, 1H), 6.3 (dd, 1H), 6.6 (s, 1H), 7.0-7.2 (m, 2H), 7.3-7.4 (m, 2H); *mlz* = 413 [M-1]⁻.

### Example 22

### N-(2-(2,4-dichlorophenylamino)-3,4-difluorophenyl)cyclopropanesulfonamide:

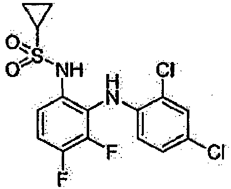

See example 1. ¹H NMR (300 MHz, CDCl₃) δ 0.9-1.0 (m, 2H), 1.05-1.20 (m, 2H), 2.4-2.5 (m, 1H), 6.0 (s, 1H), 6.3 (dd, 1H), 6.6 (s, 1H), 7.0-7.2 (m, 2H), 7.3-7.4 (m, 2H); *m*/*z* = 392 [M-1]⁻.

### Example 23

### 3-Chloro-N-(3,4-difluoro-2-(2-fluoro-4-trifluoromethyl)phenylamino)phenyl)propane-1-sulfonamide:

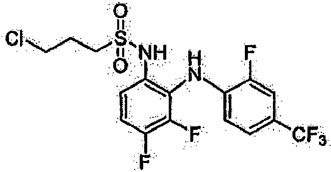

See example 1. ¹H NMR (300 MHz, CDCl₃): δ 7.39-7.26 (m, 2H), 7.25 (m, 1H), 7.18 (dd, *J* = 9.0 & 17.7 Hz, 1H), 6.78 (br s, D₂O exchangeable, 1H), 6.50 (t, *J* = 8.1 Hz, 1H), 6.00 (br d, D₂O exchangeable, *J* = 1.5 Hz, 1H), 3.63 (t, *J* = 6.0 & 6.3 Hz, 2H), 3.29 (t, *J* = 7.2 & 7.8 Hz, 2H), 2.26 (quintet, 2H); *mlz* = 445 [M-1]⁻.

### Example 24

### N-(3,4-difluoro-2-(2-chloro4-trifluoromethyl)phenylamino)methanesulfonamide:

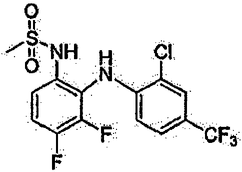

See example 1. ¹H NMR (300 MHz, CDCl₃): δ 7.65 (d, *J* = 7.8 Hz, 1H), 7.33 (m, 2H), 7.19 (dd, *J* = 9.3 & 17.4 Hz, 1H), 6.90 (br s, D₂O exchangeable, 1H), 6.45 (dd, *J* = 1.5 & 8.4 Hz, 1H), 6.39 (br s, D₂O exchangeable, 1H), 3.02 (s, 3H); *m*/*z* = 399 [M-1]⁻.

### Example 25

### 3-Chloro-N-(3,4-difluoro-2-(2-chloro-4-trifluoromethyl)phenylamino)phenyl)propane-1-sulfonamide:

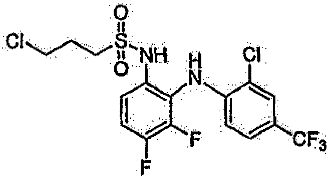

See example 1. ¹H NMR (300 MHz, CDCl₃): δ 7.66 (d, *J* = 1.5 Hz, 1H), 7.36 (m, 2H), 7.19 (dd, *J* = 9.0 & 17.4 Hz, 1H), 6.91 (br s, D₂O exchangeable, 1H), 6.50 (dd, *J* = 8.4 & 1.5 Hz, 1H), 6.37 (s, D₂O exchangeable, 1H), 3.62 (t, *J* = 6.0 Hz, 2H), 3.29 (t, *J* = 7.5 & 7.8 Hz, 2H), 2.27 (quintet, 2H); *m*/*z* = 462 [M-1]⁻.

### Example 26

### 3-Chloro-N-(3,4-difluoro-2-(2-bromo-4-trifluoromethyl) phenylamino)phenyl)propane-1-sulfonamide:

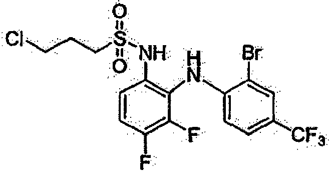

See example 1. ¹H NMR (300 MHz, CDCl₃): δ 7.82 (s, 1H), 7.38 (m, 2H), 7.20 (dd, *J* = 9.0 & 17.7 Hz, 1H), 6.62 (br s, D₂O exchangeable, 1H), 6.43 (d, *J* = 8.4 1H), 6.23 (s, D₂O exchangeable, 1H), 3.65 (t, *J* = 6.0 Hz, 2H), 3.30 (t, *J*=7.5 Hz, 2H), 2.28 (quintet, 2H); *m*/*z* = 506 [M*-*1]⁻.

### Example 27

### Cyclopropanesulfonic acid (3,4,6-trifluoro-2-(2-fluoro-4-iodo-phenylamino)-phenyl)-amide:

### Step A (2-Fluoro-4-iodo-phenyl)-(2,3,5-trifluoro-6-nitro-phenyl)-amine:

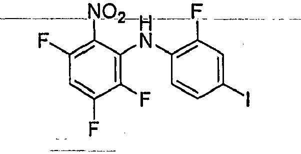

A stirred solution of 2-fluoro-4-iodoaniline (3.64 gm, 15.37 mmol) in dry THF (100 ml) under nitrogen was cooled to -78°C and a solution of 1.0 M lithium hexa methyl disilazide (LiN(SiMe₃)₂) "LHMDS" (15.37 ml, 15.37 mmol) was added slowly. This reaction mixture was kept stirring at -78°C for another hour and then 2,3,4,6-tetrafluoronitrobenzene was added. The reaction mixture was allowed to warm to room temperature and stirring continued for another 16 hours. Ethyl acetate (200 ml) was added to the reaction mixture and was washed with water. Organic layer was dried over sodium sulfate and further purified by column chromatography to provide yellow solid (3.75 gm, yield: 59.24%). M-H⁺: 410.9. ¹H NMR (DMSO, 300 MHz): 6.85 (t, 1H); 7.38 (d, 1H); 7.62 (m, 2H); 8.78 (s, 1H).

### Step B 3,4,6-Trifluoro-N²-(2-Fluoro-4-iodo-phenyl)-benzene-1,2-diamine:

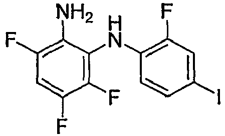

To the stirred solution of (2-fluoro-4-iodo-phenyl)-(2,3,5-trifluoro-6-nitro-phenyl)-amine 3 (5.2 gm, 12.62 mmol) in EtOH (200 ml), ammonium chloride (10.12 gm, 189.3 mmol) and iron powder (10.57 gm, 189.3 mmol) was added. This reaction mixture was kept stirring at reflux for 16 hours. Reaction mixture was allowed to cool and was filtered over celite and concentrated to dryness. The residue obtained was taken into EtOAc and was washed with water. The EtOAc layer was dried over sodium sulfate and further purified by crystallization from EtOH to provide off-white solid (3.2 gm, yield: 66.39%). M-H⁺: 381.1. ¹H NMR (DMSO, 300 MHz): 5.0 (s, 2H); 6.2 (t, 1H); 7,2 - 7.3 (m, 2H); 7.45 (s, 1H); 7.5 (d, 1H).

### Step C: 4,6,7-Trifluoro-1-(2-Fluoro-4-iodo-phenyl)-1,3,-dihydrobenzoimidazole-2-one:

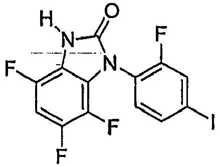

To the stirred solution of 3,4,6-trifluoro-N²-(2-Fluoro-4-iodo-phenyl)-benzene-1,2-diamine 3 (0.285 gm, 0.74 mmol) in CH₂Cl₂ (2 ml), 1,1'-carbonyldiimidazole (0.125 gm, 0.75 mmol) was added. This reaction mixture was kept stirring at room temperature for 16 hours when product precipitated out. The white solid was filtered and used further without any purification. (0.2 gm, yield: 65.85%); *m*/*z* = 407 [M-1]⁻.

### Step D/E: Cyclopropanesulfonic acid (3,4.6-trifluoro-2-(2-fluoro-4-iodo-phenylamino)-phenyl)-amide:

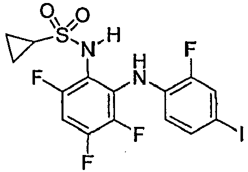

A stirred solution of 4,6,7-trifluoro-1-(2-fluoro-4-iodo-phenyl)-1,3,-dihydrobenzimidazol-2-one (0.2 gm, 0.41 mmol) in dry THF (4 ml) under nitrogen was cooled to -78°C and a solution of 1.0 M LiHMDS (0.41 ml, 0.41 mmol) was added slowly. (2 ml) followed by addition of cyclopropanesulfonyl chloride (0.050 ml, 0.49 mmol). This reaction mixture was kept stirring at room temperature for 16 hours, concentrated to dryness and was taken into EtOAc. The EtOAc was washed with water, dried over sodium sulfate and concentrated to dryness. The residue obtained 1-cyclopropanesulfonyl-4,5,7-trifluoro-3-(2-fluoro-4-iodo-phenyl)-1,3-dihydro-benzimidazol-2-one 5 was taken into dioxane (2 ml) and to this 1.0 N NaOH (0.5ml) was added and kept stirring at room 50°C for 16 hours. TLC indicated incomplete reaction, the product was purified by HPLC to provide off-white solid (4.4 mg) M+H⁺: 484.7, M-H⁺: 486.7. ¹H NMR (CDCl₃, 300 MHz): 0.9-1.1 (m, 2H); 1.1-1.2 (m, 2H); 2.45-2.55 (m, 1H); 6.05 (s, 1H); 6.44-6.54 (m, 1H); 7.1 (s, 1H); 7.4-7.7 (d, 1H); 7.38-7.44 (dd, 1H); *m*/*z* = 485 [M-1]⁻.

### Example 28

### N-(3,4-difluoro-2-(4-fluoro-2-iodophenylamino)-6-ethoxyphenyl) cyclopropane sulfonamide:

### Step A: (2,3-Difluoro-5-methoxy-6-nitro-phenyl)-(2-fluoro-4-iodo-phenyl)-amine:

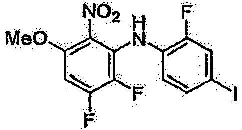

A stirred solution of (2-fluoro-4-iodo-phenyl)-(2,3,5-trifluoro-6-nitro-phenyl)-amine (1.23 gm, 3 mmol) in dry THF (25 ml) under nitrogen was cooled to -78°C and a solution of 25% NaOMe (0.68 ml, 0.3 mmol) was added slowly. Reaction mixture was allowed to warm to room temperature and stirring continued for another 16 hours. TLC indicated incomplete reaction. Ethyl acetate (100 ml) was added to the reaction mixture and was washed with water. Organic layer was dried over sodium sulfate and further purified by column chromatography to provide yellow solid (0.6 gm, yield: 47.6%). m/z = 424 [M=H]⁺.

### Step B: 5.6-Difluoro-N1-(4-fluoro-2-iodophenyl)-3-methoxybenzene-1,2-diamine:

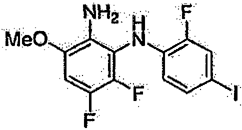

To the stirred solution of (2,3-difluoro-5-methoxy-6-nitro-phenyl)-(2-fluoro-4-iodophenyl)-amine (0.57 gm, 1.34 mmol) in EtOH (20 ml), ammonium chloride (1.18 gm, 20.16 mmol) and iron powder (1.15 gm, 21.44 mmol) was added. This reaction mixture was kept stirring at reflux for 16 hours. Reaction mixture was allowed to cool and was filtered over celite and concentrated to dryness. The residue obtained was taken into EtOAc and was washed with water. The EtOAc layer was dried over sodium sulfate and further purified by crystallization from EtOH to provide off-white solid (0.47 gm, yield: 90.3%). M-H⁺: 393.2. ¹H NMR (DMSO, 300 MHz): 3.76 (s, 3H); 6.1 (t, 1H); 6.8 - 7.0 (m, 1H); 7.2 (d, 1H); 7.35 (s, 1H); 7.42 (d, 1H).

### Step C: 6,7-Difluoro-1-(4-fluoro-2-iodophenyl)-4-methoxy-1H-benzo[d]imidazol-2(3H)-one:

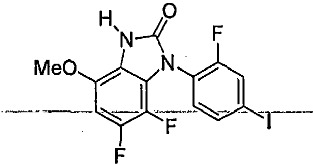

To the stirred solution of 5,6-difluoro-N1-(4-fluoro-2-iodophenyl)-3-methoxybenzene-1,2-diamine (0.17 gm, 0.43 mmol) in CH₂Cl₂ (2 ml), 1,1'-Carbonyldiimidazole (0.085 gm, 0.53 mmol) was added. This reaction mixture was kept stirring at room temperature for 16 hours when product precipitated out. The white solid was filtered and used further without any purification. (0.089 gm); *m*/*z* = 419 [M-1]⁻.

### Step D/F: N-(3,4-difluoro-2-(4-fluoro-2-iodophenylamino)-6-methoxyphenyl) cyclopropanesulfonamide:

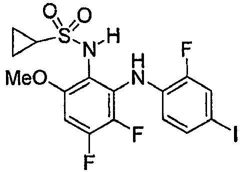

A stirred solution of 1-(cyclopropylsulfonyl)-4,5-difluoro-3-(2-fluoro-4-iodophenyl)-7-methoxy-1H-benzo[d]imidazol-2(3H)-one (0.89 gm, 0.17 mmol) in dry THF (4 ml) under nitrogen was cooled to -78°C and a solution of 1.0 M LiHMDS (0.17 ml, 0.17 mmol) was added slowly. (2 ml) followed by addition of Cyclopropanesulfonyl chloride (0.021 ml, 0.21 mmol). This reaction mixture was kept stirring at room temperature for 16 hours, concentrated to dryness and was taken into EtOAc. The EtOAc was washed with water, dried over sodium sulfate and concentrated to dryness. The resulting 1-(cyclopropylsulfonyl)-4,5-difluoro-3-(2-fluoro-4-iodophenyl)-7-methoxy-1H-benzo[d]imidazol-2(3H)-one was taken into dioxane (2 ml) and to this 1.0 N NaOH (0.5ml) was added and kept stirring at room 50°C for 16 hours. TLC indicated incomplete reaction, the product was purified by HPLC to provide off-white solid (2.5 mg) M+H⁺: 484.7, M-H⁺: 497.3. ¹H NMR (CDCl₃, 300 MHz): 0.85 - 0.95 (m, 2H); 1.05 - 1.15 (m, 2H); 2.4-2.5 (m, 1H); 3.9 (s, 3H); 6.1 (s, 1H); 6.4-6.6 (m, 2H); 7.3 (m, 1H); 7.35 - 7.4 (dd, 1H); *m*/*z* = 497 [M-1]⁻.

### Example 29

### Methylsulfonic acid (3,4-difluoro-2-(2-fluoro-4-iodo-phenylamino)-6-methoxy-phenyl)-amide:

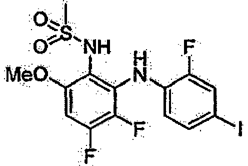

A stirred solution of 5,6-difluoro-N1-(4-fluoro-2-iodophenyl)-3-methoxybenzene-1,2-diamine (0.150 gm, 0.38 mmol) in dry CH₂Cl₂ (4 ml), TEA (.264 ml, 1.9 mmol) and Methanesulfonyl chloride was added slowly. This reaction mixture was kept stirring at room temperature for 16 hours, TLC indicated incomplete reaction along with starting material two products were observed. The reaction mixture was washed with water, organic layer was dried over sodium sulfate and concentrated to dryness, the product was purified by column chromatography. The minor product was found to be the expected compound (6.4 mg). M-H⁺: 471.5. ¹H NMR (CDCl₃, 300 MHz): 3.9 (s, 3H); 6.05 (s, 1H); 6.4-6.5 (m, 1H); 6.5-6.6 (m, 1H); 7.2 (s, 1H); 7.28 (d, 1H); 7.35-7.4 (d, 1H); *m*/*z* = 471 [M-1]⁻.

### Example 30

### 1-(2,3-Dihydroxy-propyl)-cyclopropanesulfonic acid [3,4,6-trifluoro-2-(4-fluoro-2-iodo-phenylamino)-phenyl]-amide:

### Step A: 1 -Allyl-cyclopropanesulfonic acid [3,4,6-trifluoro-2-(2-fluoro-4-iodo-phenylamino)-phenyl]-amide:

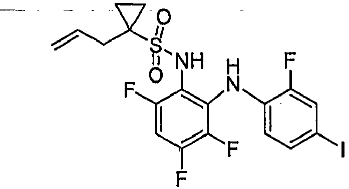

According to the general procedure B, 1-allyl-cyclopropanesulfonyl chloride was reacted with 3,5,6-trifluoro-N¹-(2-fluoro-4-iodophenyl)benzene-1,2-diamine to obtain the title product. ¹H NMR (CDCl₃, 300 MHz): δ 7.41 (dd, 1H), 7.38 (dd, 1H), 7.09 (s, 1H), 6.78 (m, 1H), 6.49 (m, 1H), 5.96 (s, 1H), 5.86 (m, 1H), 5.18 (d, 2H), 2.76 (d, 2H), 1.23 (m, 2H), 0.872 (m, 2H).

### Step B: 1-(2,3-Dihydroxypropyl)-N-(3,4,6-trifluoro-2-(2-fluoro-4-iodophenylamino) phenyl)cyclopropane-1-sulfonamide:

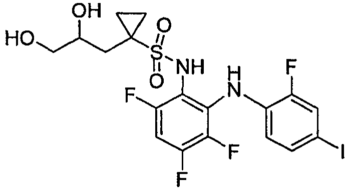

1-Allyl-cyclopropanesulfonic acid [3,4,6-trifluoro-2-(2-fluoro-4-iodo-phenyl amino)-phenyl]-amide (110 mg, 0.21 mmole) and 4-methylmorpholine N-oxide (24.6 mg, 0.21 mmole) was dissolved in THF (8 mL). Osmium tetroxide was added at room temperature (0.021 mmole, 0.153 mL, 4% in H₂O) and the reaction mixture was stirred at room temperature for 16 hours. EtOAc was added, the organic phase was washed with water, dried (MgSO₄) and concentrated under reduced pressure. The residue was purified over silica gel chromatography (eluants: EtOAc/ MeOH) to obtain the titled product (0. 89 g, 75 %). ¹H NMR (CDCl₃, 300 MHz): δ 7.39 (dd, J = 1.5 & 10.6 Hz, 1H), 7.29 (d, J = 8.8 Hz, 1H), 7.28 (s, 1H), 6.97 (s, 1H), 6.76 (m, 1H), 6.49 (m, 1H), 4.13 (m, 1H), 3.66 (dd, J = 3.7 & 11.4 Hz, 1H), 3.53 (dd. J = 6.7 & 11.2 Hz, 1H), 2.50(dd, J = 10.0 & 16.1 Hz, 1H), 1.6 (m, 1H), 1.46 (m, 1H), 1.28 (m, 1H), 1.20 (m, 2H), 0.92 (m, 2H); *mlz* = 559 [M-1]⁻.

### Example 31

### (S)-1-(2,3-dihydroxypropyl)-N-(3,4,6-trifluoro-2-(2-fluoro-4-iodophenylamino) phenyl)cyclopropane-1-sulfonamide:

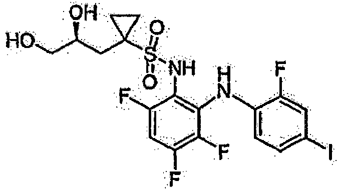

The pure S isomer was obtained by chiral HPLC separation of the racemic mixture (example 34). ¹H NMR (CDCl₃, 300 MHz): δ 7.39 (dd, J = 1.5 & 10.6 Hz, 1H), 7.29 (d, J = 8.8 Hz, 1H), 7.28 (s, 1H), 6.97 (s, 1H), 6.76 (m, 1H), 6.49 (m, 1H), 4.13 (m, 1H), 3.66 (dd, J = 3.7 & 11.4 Hz, 1H), 3.53 (dd. J = 6.7 & 11.2 Hz, 1H), 2.50(dd, J = 10.0 & 16.1 Hz, 1H), 1.6 (m,1H), 1.46 (m, 1H), 1.28 (m, 1H), 1.20 (m, 2H), 0.92 (m, 2H); *m*/*z* = 559 [M-1]⁻.

### Example 32

### (R)-1-(2,3-dihydroxypropyl)-N-(3,4,6-trifluoro-2-(2-fluoro-4-iodophenylamino) phenyl)cyclopropane-1-sulfonamide:

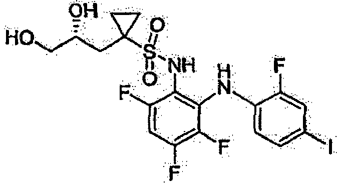

The pure R isomer was obtained by chiral HPLC separation of the racemic mixture (example 31). ¹H NMR (CDCl₃, 300 MHz): δ 7.39 (dd, J = 1.5 & 10.6 Hz, 1H), 7.29 (d, J = 8.8 Hz, 1H), 7.28 (s, 1H), 6.97 (s, 1H), 6.76 (m, 1H), 6.49 (m, 1H), 4.13 (m, 1H), 3.66 (dd, J = 3.7 & 11.4 Hz, 1H), 3.53 (dd. J = 6.7 & 11.2 Hz, 1H), 2.50(dd, J = 10.0 & 16.1 Hz, 1H), 1.6 (m,1H), 1.46 (m, 1H), 1.28 (m, 1H), 1.20 (m, 2H), 0.92 (m, 2H); *m*/*z* = 559 [M-1]⁻.

### Example 33

### N-(3,4-difluoro-2-(2-fluoro-4-iodophenylamino)phenyl-1-(2,3-dihydroxypropyl) cyclopropane-1-sulfonamide:

### Step A: 1-Allyl-N-(3,4-difluoro-2-(2-fluoro-4-iodophenylamino)-6-methoxyphenyl)cyclopropane-1-sulfonamide

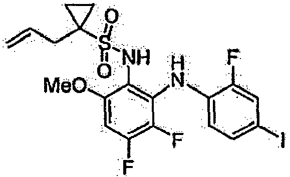

According to the general procedure B, 1-allyl-cyclopropanesulfonyl chloride was reacted with 5,6-difluoro-N1-(2-fluoro-4-iodophenyl)-3-methoxybenzene-1,2-diamine to obtain the title product. ¹H NMR (CDCl₃, 300 MHz): δ 7.417 (dd, 1H), 7.309(s, 1H), 7.25 (m, 1H), 6.89 (m, 1H), 6.32(m,1H), 6.427 (m, 1H), 6.03 (s,1H), 5.668 (m, 1H), 5.11 (t, 1H), 3.9 (s, 3H), 2.75 (d, 2H), 1.21 (m, 2H), 0.767 (m, 2H).

### Step B: N-(3,4-difluoro-2-(2-fluoro-4-iodophenylamino)phenyl)-1-(2,3-dihydroxypropyl) cyclopropane-1-sulfonamide

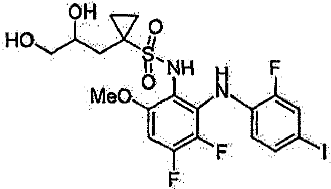

1-Allyl-N-(3,4-difluoro-2-(2-fluoro-4-iodophenylamino)-6-methoxyphenyl) cyclopropane-1-sulfonamide (97 mg, 0.18 mmole) and 4-methylmorpholine N-oxide (21 mg, 0.18 mmole) were dissolved in THF (8 mL). Osmium tetroxide was added at room temperature (0.018 mmole), 0.13 mL, 4% in H₂O) and the reaction mixture was stirred at room temperature for 16 hours. EtOAc was added, the organic phase was washed with water, dried (MgSO₄) and concentrated under reduced pressure. The residue was purified over silica gel chromatography (eluants: EtOAc/MeOH) to obtain the titled product (0. 80 g, 78 %). ¹H NMR (CDCl₃, 300 MHz): δ 7.38 (dd, J = 1.7 & 10.3 Hz, 1H), 7.26 (m, 1H), 7.14 (s, 1H), 6.87 (s, 1H), 6.53 (dd, J = 6.8 & 11.4 Hz, 1H), 6.43 (m, 1H), 4.06 (m, 1H), 3.89 (s, 3H), 3.63 (dd, J = 3.7 & 11.1 Hz, 1H), 3.49 (dd, J = 6.4 & 11.1 Hz, 1H), 2.3 (dd, J = 9.7 & 16.1 Hz, 1H), 1.77 (dd, J = 1.9 & 16.0 Hz, 1H), 1.37 (m, 1H), 1.25 (m, 1H), 1.21 (m, 2H), 0.86 (m, 2H); *mlz* = 571 [M-1]⁻.

### Example 34

### (S)-N-(3,4-difluoro-2-(2,-fluoro-4-iodophenylamino)-6-methoxyphenyl)-1-(2,3-dihydroxypropyl)cyclopropane-1-sulfonamide:

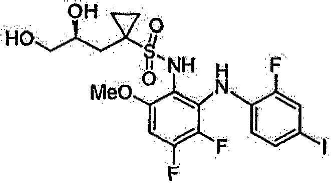

The pure S isomer was obtained by chiral HPLC separation of the racemic mixture (example 37). ¹H NMR (CDCl₃, 300 MHz): δ 7.38 (dd, J = 1.7 & 10.3 Hz,1H), 7.26 (m, 1H), 7.14 (s, 1H), 6.87 (s, 1H), 6.53 (dd, J = 6.8 & 11.4 Hz, 1H), 6.43 (m, 1H), 4.06 (m, 1H), 3.89 (s, 3H), 3.63 (dd, J = 3.7 & 11.1 Hz, 1H), 3.49 (dd, J = 6.4 & 11.1 Hz, 1H), 2.3 (dd, J = 9.7 & 16.1 Hz, 1H), 1.77 (dd, J = 1.9 & 16.0 Hz, 1H), 1.37 (m, 1H), 1.25 (m, 1H), 1.21 (m, 2H), 0.86 (m, 2H); *m*/*z* = 571 [M-1]⁻.

### Example 35

### (R)-N-(3,4-difluoro-2-(2-fluoro-4-iodophenylamino)-6-methoxyphenyl)-1-(2,3-dihydroxypropyl)cyclopropane-1-sulfonamide:

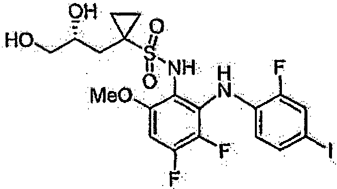

The pure R isomer was obtained by chiral HPLC separation of the racemic mixture (example 37). ¹H NMR (CDCl₃, 300 MHz): δ 7.38 (dd, J = 1.7 & 10.3 Hz,1H), 7.26 (m, 1H), 7.14 (s, 1H), 6.87 (s, 1H), 6.53 (dd, J = 6.8 & 11.4 Hz, 1H), 6.43 (m, 1H), 4.06 (m, 1H), 3.89 (s, 3H), 3.63 (dd, J = 3.7 & 11.1 Hz, 1H), 3.49 (dd, J = 6.4 & 11.1 Hz, 1H), 2.3 (dd, J = 9.7 & 16.1 Hz, 1H), 1.77 (dd, J = 1.9 *&* 16.0 Hz, 1H), 1.37 (m, 1H), 1.25 (m, 1H), 1.21 (m, 2H), 0.86 (m, 2H); *m*/*z* = 571 [M-1]⁻.

### Example 36

### 1-(2-hydroxyethyl)-N-(3,4,6-trifluoro-2-(2-fluoro-4-iodophenylamino)phenyl) cyclopropane-1-sulfonamide:

### Step A: TBS-protected 1-(2-hydroxyethyl)-N-(3,4,6-trifluoro-2-(2-fluoro-4-iodophenylamino)phenyl) cyclopropane-1-sulfonamide:

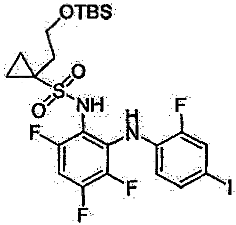

According to the general procedure B, the sulfonyl chloride prepared in step C of example 16 was reacted with 5,6-difluoro-N1-(2-fluoro-4-iodophenyl)-3-fluorobenzene-1,2-diamine to obtain the title product. Yield: 13%. ¹H-NMR (300 MHz, CDCl₃): δ = 7.51 (s, 1H, br), 7.37-7.35 (d, 1H), 7.27-7.25 (d, 1H), 6.94 (s, 1H, br), 6.78-6.68 (m, 1H), 6.46-6.44 (m, 1H), 3.90-3.88 (t, 2H), 2.12-2.10 (t, 2H), 1.31-1.28 (m, 2H), 0.91-0.89 (m, 2H), 0.86 (s, 9H), 0.05 (s, 6H); *m*/*z* = 643 [M-1]⁻.

### Step B: 1-(2-hydroxyethyl)-N-(3,4,6-trifluoro-2-(2-fluoro-4-iodophenylamino)-phenyl)cyclopropane-1-sulfonamide:

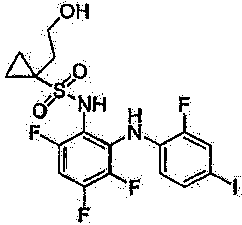

Same procedure as in step E, example: 16. Yield: 100%. ¹H-NMR (300 MHz, CDCl₃): δ = 7.51 (s, 1H, br), 7.37-7.35 (d, 1H), 7.27-7.25 (d, 1H), 6.94 (s, 1H, br), 6.78-6.68 (m, 1H), 6.46-6.44 (m, 1H), 3.90-3.88 (t, 2H), 2.12-2.10 (t, 2H), 1.31-1.28 (m, 2H), 0.91-0.89 (m, 2H); *m*/*z =* 529 [M-1]⁻.

### Example 37

### N-(3,4-difluoro-2-(2-fluoro-4-iodophenylamino)-6-methoxyphenyl)-1-(2-hydroxyethyl)cyclopropane-1-sulfonamide:

### Step A: TBS-protected N-(3,4-difluoro-2-(2-fluoro-4-iodophenylamino)-6-methoxyphenyl)-1-(2-hydroxyethy)cyclopropane-1-sulfonamide:

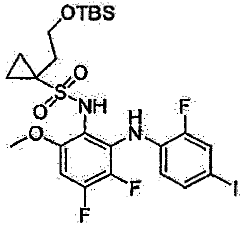

According to the general procedure B, the sulfonyl chloride prepared in step C of example 16 was reacted with 5,6-difluoro-N1-(2-fluoro-4-iodophenyl)-3-methoxy-benzene-1,2-diamine to obtain the title product. Yield: 37%. ¹H-NMR (300 MHz, CDCl₃): δ = 7.40-734 (dd, 1H), 7.23-7.21 (m,1H), 6.61 (s, 1H, br), 6.57-6.49 (dd, 1H), 6.48-6.39 (m, 1H), 3.9-3.7 (m, 5H), 2.15-2.05 (t, 2H), 1.30-1.20 (m, 2H), 0.95-0.80 (m, 11H), 0.05 (s, 6H); *m*/*z* = 655 [M-1]⁻.

### Step B: N-(3,4-difluoro-2-(2-fluoro-4-iodophenylamino)-6-methoxyphenyl)-1-(2-hydroxyethyl)cyclopropan-1-sulfonamide:

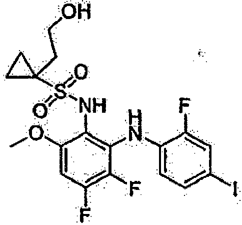

Same procedure as in step E, example 16. Yield: 100%. ¹H-NMR (300 MHz, CDCl₃): δ = 7.40-7.34 (dd, 1H), 7.23-7.21 (m, 1H), 6.61 (s, 1H, br), 6.57-6.49 (dd, 1H), 6.48-6.39 (m, 1H), 3.9-3.7 (m, 5H), 2.15-2.05 (t, 2H), 1.30-1.20 (m, 2H), 0:95-0.80 (m, 2H); *m*/*z* = 541 [M-1]⁻.

### Example 38

### N-(3,4-difluoro-2-(2-fluoro-4-iodophenylamino)-6-methoxyphenyl)-1-(3-hydroxy-2-(hydroxymethyl)propyl)cyclopropane-1-sulfonamide:

### Step A: Dimethyl 2-(2-bromoallyl)malonate:

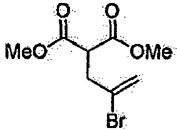

To a suspension of sodium hydride (5.0 g, 125 mmol) in HMPA (50 ml, distilled from calcium hydride) was added a solution of dimethyl malonate (11.7 ml, 100 mmol) in HMPA (5 ml) at 0°C under argon. The mixture was heated to 50°C and stirred 1 hour. Following this the solution was again cooled to 0°C, and a solution of 2,3-dibromopropene (12.2 ml, 100 mmol) in HMPA (5 ml) was added to the reaction mixture. Next, the solution was warmed to 40°C and stirred for 1 hour. The reaction mixture was quenched with aq. HCl (10%, 88 ml) and extracted with ether (3 x 45 ml). The organic fractions were collected, dried over MgSO-₄, and the solvent was removed *in vacuo.* The crude oil was purified via silica gel chromatography (eluants: chloroform/hexane) to obtain the titled product as a colorless oil (16.3 g, 65%). ¹H-NMR (300 MHz, CDCl₃) δ 5.70 (d, J = 1.8 Hz, 1 H), 5.48 (d, J = 1.8 Hz, 1H), 3.63 (t, J = 7.5 Hz, 1 H), 3.76 (s, 6 H), 3.04 (d, J = 7.5 Hz, 2 H).

### Step B: 2-(2-Bromoallyl)propane-1,3-diol:

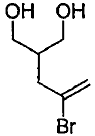

Lithium aluminum hydride (1.9 g, 7.65 mmol) was slurried in anhydrous diethyl ether (50 ml) and cooled to -78°C in a dry ice/acetone bath. A solution of the product from step A (0.639 g, 16.84 mmol) in dry ether (26 ml) was then added dropwise. After the malonate was added, the solution was allowed warm to room temperature and stirring was continued for 3 hours. The reaction was quenched with brine (50 ml), extracted with ethyl acetate (3 x 25 ml) and dried over MgSO₄. The solvent was removed *in vacuo* to give the desired product (1.3 g, 86%) which was used for the next step without further purification. ¹H-NMR (300 MHz, CDCl₃) δ 5.66 (d, J = 1.2 Hz, 1 H), 5.48 (d, J = 1.5 Hz, 1H), 3.86 (m, 2 H), 3.73 (m, 2 H), 2.51 (d, J = 7.5 Hz, 2 H), 2.40 (br s, 2 H), 2.15 (m, 1 H).

### Step C: Di-tert-butyldimethylsilyl protected 2-(2-bromoallyl)propane-1,3-diol:

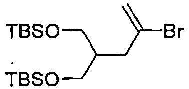

The product from step B (2.8 g, 14.20 mmol) was dissolved in anhydrous THF (140 ml). Anhydrous pyridine-(2.5-ml, 31.24 mmol) was added, and the solution was cooled to 0°C. tert-Butyldimethylsilyltriflate (7.2 ml, 31.24 mmol) was added dropwise, and upon completion, the reaction solution was heated to 35°C. After stirring for 6 days, the reaction was quenched with 100 ml brine, extracted with ethyl acetate (3 x 50 ml) and dried over MgSO₄. The combined organic phases were evaporated to obtain the crude product (5.5 g, 91%) as a yellow oil which was used in the next step without further purification. ¹H-NMR (300 MHz, CDCl₃) δ 5.54 (d, J = 0.9 Hz, 1H), 5.40 (d, J = 1.2 Hz, 1H), 3.55 (d, J = 5.4, 4 H), 2.40 (d, J = 6.9 Hz, 2 H), 1.97 (m, 1 H), 0.85 (s, 18 H), 0.02 (s, 9 H).

### Step D: Di-tert-butyldimethylsilyl protected 2-((1-bromocyclopropyl)methyl) propane-1,3-diol:

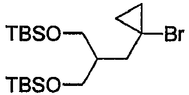

A reaction flask was charged with anhydrous CH₂Cl₂ (10 ml) and diethyl zinc (1.0 M in hexanes, 4.65 ml, 4.65 mmol) at 0°C. Trifluoroacetic acid (0.358 ml, 4.65 mmol) was added dropwise and the solution was allowed to stir for 20 minutes. Diiodomethane (0.375 ml, 4.65 mmol) was then added and the solution was stirred for another 20 minutes. Finally, the product from step C (0.492 g, 1.16 mmol) was added and the solution was allowed to warm to ambient temperature, stirring for 16 hours. The reaction was quenched with saturated aqueous NH₄Cl. The layers were partitioned and the aqueous phase was extracted with chloroform (3 x 5 ml). The combined organic phases were washed with brine (10 ml), dried over MgSO₄, and the volatiles were removed *in vacuo.* The resulting crude was purified via silica gel chromatography (eluants: chloroform/hexanes) to provide the product as a clear oil (0.280 g, 64%). ¹H-NMR (300 MHz, CDCl₃) δ 3.66 (d, J = 5.4, 4 H), 2.08 (m, 1 H), 1.64 (d, J = 6.9, 2 H), 1.13 (m, 2 H), 0.88 (s, 18 H), 0.81 (m, 2 H), 0.04 (s, 9 H).

### Step E: Di-tert-butyldimethylsilyl protected 1-(3-hydroxy-2-(hydroxymethyl)propyl) cyclopropane-1-sulfonyl chloride:

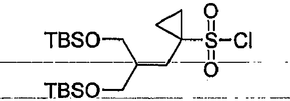

The product from **step D** (0.507 g, 1.16 mmol) was dissolved in anhydrous ether (6 ml) and the reaction solution was cooled to -78°C. Following this, tert-butyllithium (1.7 M in pentane, 1.50 ml, 2.55 mmol) was added dropwise over 5 minutes. After stirring for 0.5 hours, the lithiated product was transferred via cannula to a stirred solution of sulfuryl chloride (0.206 ml, 2.55 mmol) in dry ether (6 ml) at -78°C. Once the transfer is complete, the solution was allowed to warm to room temperature, the solvent was evaporated and the resulting white solid was slurried in dry hexanes. This slurry was immediately filtered through celite, and all volatiles were removed *in vacuo.* The resulting crude product (0.376 g, 71%) was isolated as a yellow oil and was used in the following step without further purification. ¹H-NMR (300 MHz, CDCl₃) δ 3.60 (m, 4 H), 2.16 (m, 1 H), 2.03 (d, 2 H), 0.88 (s, 18 H), 0.04 (s, 9 H).

### Step F: Di-tert-butyldimethylsilyl protected N-(3,4-difluoro-2-(2-fluoro-4-iodophenylamino)-6-methoxyphenyl)-1-(3-hydroxy-2-(hydroxymethyl)propyl) cyclopropane-1-sulfonamide:

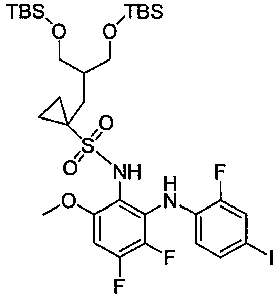

5,6-difluoro-N1-(2-fluoro-4-iodophenyl)-3-methoxybenzene-1,2-diamine (8.8 mg, 0.022 mmol) was dissolved in anhydrous pyridine (0.5 ml) under an argon atmosphere. The product from step E (20.5 mg, 0.045 mmol), dissolved in dry pyridine (0.5 ml), was added to the reaction flask and the mixture was heated at 80°C for 21 hours. The solvent was removed *in vacuo* and the resulting crude was purified via silica gel chromatography (eluents: ethyl acetate/hexanes) to provide the title compound (2.75 mg, 15%). *m*/*z* 813.5 (M-1).

### Step G: N-(3,4-difluoro-2-(2-fluoro-4-iodophenylamino)-6-methoxyphenyl)-1-(3-hydroxy-2-(hydroxymethyl)propyl)cyclopropane-1-sulfonamide:

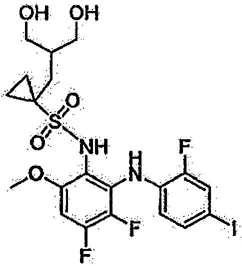

The product from step F (27.9 mg, 0.0342 mmol) was dissolved in THF (1 ml) and treated with aqueous HCl (1.2 N, 0.2 ml) at 0°C. The resulting solution was stirred for 4 hours. Following this, the reaction was quenched with saturated aqueous NaHCO₃, extracted with ethyl acetate, dried over MgSO₄ and the volatiles were removed *in vacuo.* The resulting crude was purified via silica gel chromatography (eluents: methanol/chloroform) followed by LC-MS purification to provide the title compound (11.8 mg, 59%). ¹H-NMR (300 MHz, CD₃OD) δ 7.32 (dd, 1 H), 7.21 (d, 1 H), 6.76 (dd, 1 H), 6.33 (m, 1 H), 3.82 (s, 3 H), 3.52 (d, 4 H), 2.01 (m, 1 H), 1.88 (d, 2 H), 1.07 (m, 2 H), 0.75 (m, 2 H), *m*/*z* 585.3 (M-1).

### Example 39

### N-(3,4-difluoro-2-(2-fluoro-4-iodophenylamino)-6-methoxyphenyl)cyclobutane sulfonamide:

### Step A: Cyclobutanesulfonyl chloride:

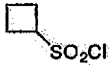

To a suspension of Mg turnings (0.790 g, 32.5 mmol) in 20 ml anhydrous diethyl ether was added a solution of cyclobutylbromide (1.8 ml, 2.5722 g, 19.1 mmol) in 20 ml diethyl ether in small portions with strong stirring. After the initial exothermic reaction had ceased, the mixture was further heated to the reflux temperature for 30 min. The suspension was cooled down to room temperature and the supernatant was added in small portions to an ice-cold solution of sulfuryl chloride (4.6 ml, 7.728 g, 57.2 mmol) in 30 ml anhydrous DCM. After complete addition, the suspension was warmed to room temperature and the volatiles were removed in vacuo. The residue was dried in oil-pump vacuo for 15 min, then it was extracted with hexane (150 ml). The hexane suspension was filtered and the hexane was removed in vacuo to give the crude product as dark purple oil which was used for the next step without further purification. There is still some unreacted cyclopropylbromide present. Crude yield: 1.1 g (38%).

### Step B: N-(3,4-difluoro-2-(2-fluoro-4-iodophenylamino)-6-methoxyphenyl) cyclobutanesulfonamide

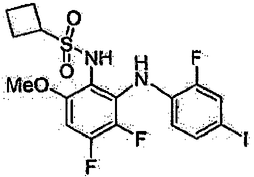

According to the general procedure B, the cyclobutylsulfonyl chloride prepared in the step above was reacted with 5,6-difluoro-N1-(2-fluoro-4-iodopheny)-3-methoxy-benzene-1,2-diamine to obtain the title product. Yield: 75%. ¹H-NMR (300 MHz, CDCl₃): δ = 7.44 (s, 1H, br), 7.41-7.36 (dd, 1H), 7.24-7.23 (m, 1H), 6.54-6.38 (m, 2H), 5.90 (s, 1H, br), 3.85-3.75 (m, 5H), 2.60-2.40 (m, 2H), 2.25-2.15 (m, 1H), 2.15-1.95 (m, 2H); *m*/*z* = 511 [M-1]⁻.

### Example 40

### N-(3,4-difluoro-2-(2-fluoro-4-iodophenylamino)-6-methylphenyl)-1-(2,3-dihydroxypropyl)cyclopropane-1-sulfonamide:

### Step A: (3,4,5-Trifluorophenyl)methanol:

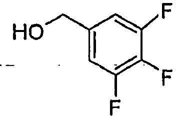

To a cooled (-5°C) solution of 3,4,5-trifluorobenzaldehyde (7.0 g, 43.75 mmol) in a mixture (50 ml, 9:1) of THF and water NaBH₄ (1.662 g, 43.75 mmol) was slowly added in portions over a period of 30 min. The reaction mixture was allowed to attain room temperature over a period of 2h and carefully poured into ice-cold dil HCl (200 ml, IN). The oily layer was extracted into CH₂Cl₂ (250 ml) and the organic layer washed with water (200 ml), dried (MgSO₄) and evaporated. The crude product (7.08 g, quantitative) obtained was taken forward without further purification.

### Step B: 5-(Bromomethyl)-1,2,3-trifluorobenzene:

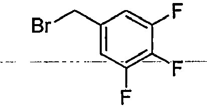

To a solution of the (3,4,5-Trifluorophenyl)methanol (40 mmol) in CH₂Cl₂ (150 ml), a solution of thionyl bromide (6.16 ml, 80 mmol) in CH₂Cl₂ (50 ml) was added slowly. The reaction mixture stirred at room temperature for 16h and poured into ice-water (200 ml). The organic layer was separated and washed with saturated NaHCO₃ (2x200 ml), water (200 ml), dried (MgSO₄) and evaporated to obtain the corresponding bromo compound as a pale yellow oil in quantitative yield. The crude product was carried forward for the next reaction without further purification.

### Step C: 1,2,3-Trifluoro-5-methylbenzene:

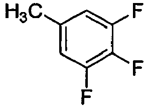

The above bromo compound (40 mmol) was mixed with triethylsilane (48 mmol) and the reaction mixture was treated with solid PdCl₂ (4 mmol) in small portions. After a few minutes a vigorous exothermic reaction was ensued and care was taken to reflux the contents of the flask by placing a reflux condenser. The reaction mixture was stirred at room temperature for additional 6h and the contents were allowed to settle over 16h. Then the crude liquid product was decanted carefully and carried forward for the next reaction without further purification. It was assumed tat the reaction proceeds in quantitative yield.

### Step D: 1,2,3-Trifluoro-5-methyl4-nitrobenzene:

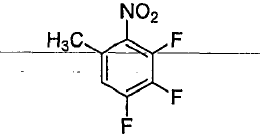

1,2,3-Trifluoro-5-methylbenzene (40 mmol) was added to conc. H₂SO₄ (50 ml) at 0-5° C. Then the reaction mixture was slowly treated with conc. HNO₃ (3.39 ml, 48.44 mmol, 90%) while maintaining the internal temperature below 20° C. The reaction mixture was stirred at room temperature for 16h and poured onto ice (300 g) and the oily layer was extracted with CH₂Cl₂ (2x125 ml). The organic layer was washed with water (2x200 ml), brine (200 ml) and dried (MgSO₄) and evaporated to obtain the crude product which was purified over flash silica gel chromatography to obtain the title product (6.5 g, 85%). ¹H NMR (300 MHz, CDCl₃): δ 6.96 (septet, 1H), 2.39 (s, 3H). ¹⁹F NMR (CDCl₃): δ -128.18,-141.50,-159.05.

### Step E: 2,3-Difluoro-N-(2-fluoro-4-iodophenyl)-5-methyl-6-nitroaniline:

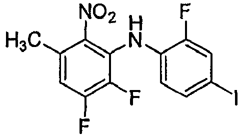

2-Fluoro-4-iodoaniline and 1,2,3-trifluoro-5-methyl-4-nitrobenzene were reacted using the condition described in Example 1 (Step A) to form the title compound. M-H⁺: 407.9

### Step F: 5,6-Difluoro-N1-(2-fluoro-4-iodophenyl)-3-methylbenzene-1,2-diamine:

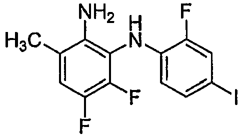

2,3-Difluoro-N-(2-fluoro-4-iodophenyl)-5-methyl-6-nitroaniline was reduced using the condition described in Example 1 (step B) to form the title compound. M-H⁺: 377.4

### Step G: 1-Allyl-N-(3,4-difluoro-2-(2-fluoro-4-iodophenylamino)-6-methylphenyl)cyclopropane-1-sulfonamide:

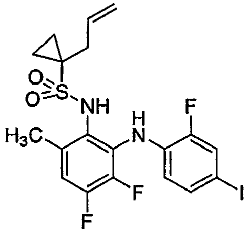

According to the general procedure B, 1-allyl-cyclopropanesulfonyl chloride (142 mg, 142 mg) was reacted with 5,6-difluoro-N1-(2-fluoro-4-iodophenyl)-3-methylbenzene-1,2-diamine (150 mg, 0.4 mmole) to obtain the title product (100 mg, 47%); *mlz* = 521 [M-1]⁻.

### Step H: N-(3,4-difluoro-2-(2-fluoro-4-iodophenylamino)-6-methylpheny)-1-(2,3-dihydroxypropyl)cyclopropane-1-sulfonamide:

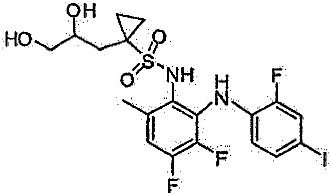

1-Allyl-N-(3,4-difluoro-2-(2-fluoro-4-iodophenylamino)-6-methylphenyl) cyclopropane-1-sulfonamide (150 mg, 0.29 mmole) and 4-methylmorpholine N-oxide (33 mg, 0.29 mmole) was dissolved in THF (5 mL). Osmium tetroxide was added at room temperature (0.029 mmole, 0.18 mL, 4% in H₂O) and the reaction mixture was stirred at room temperature for 16 hours. EtOAc was added, the organic phase was washed with water, dried (MgSO₄) and concentrated under reduced pressure. The residue was purified over silica gel chromatography (eluants: EtOAc/ MeOH) to obtain the titled product (0.110 g, 68 %). ¹H NMR (300 MHz, CDCl₃): δ 7.07 (m, 1H), 6.97 (br m, 2H), 6.84 (m, 2H), 6.60 (br m, 2H), 3.98 (br m, 1H), 3.58 (m, 1H), 3.43 (m, 1H), 3.20 (d, *J* = 3.9 Hz, 1H), 2.42 (s, 3H), 2.31 (dd, *J* = 9.9 & 15.6 Hz, 1H), 2.01 (br t, 1H), 2.31 (dd, *J* = 9.9 & 15.6 Hz, 1H), 1.66 (dd, *J* = 2.1 & 15.9 Hz, 1H), 1.52 (m, 1H), 1.40 (m, 1H), 0.91 (m, 2H).

### Example 41

### 1-(2,3-Dihydroxypropyl)-N-(6-ethyl-3,4-difluoro-2-(2-fluoro-4-iodophenylamino) phenyl) cyclopropane-1-sulfonamide:

### Step A: 1-(3,4,5-Trifluorophenyl)ethanol:

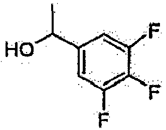

An ethereal solution (17.41 ml, 52.24 mmol, 3M) of MeMgBr was slowly added at - 78° C to a solution of 3,4,5-trifluorobenzaldehyde (6.96 g, 43.53 mmol) in THF (125 ml). The reaction mixture was stirred at room temperature for 16h and was cooled (0° C) and was quenched, sequentially, with excess ethyl acetate (10 ml) and water (5 ml). Excess anhydrous MgSO₄ (5 g) was added and stirred for 30 minutes at room temperature. The suspension was filtered over celite and the solids were washed with ethyl acetate (2x25 ml). The combined filtrate was evaporated to obtain the product in quantitative yield (7.65g).

### Step B: 5-(1-Bromoethyl)-1,2,3-trifluorobenzene:

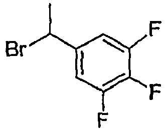

To a solution of the 1-(3,4,5-Trifluorophenyl)ethanol: (7.65 g, 43.5 mmol) in CH₂Cl₂ (250 ml), a solution of thionyl bromide (18.1 g, 87 mmol) in CH₂Cl₂ (50 ml) was added slowly. The reaction mixture stirred at room temperature for 16h and poured into ice-water (200 ml). The organic layer was separated and washed with saturated NaHCO₃ (2x200 ml), water (200 ml), dried (MgSO₄) and evaporated to obtain the corresponding bromo compound as a pale yellow oil in quantitative yield (10.4 g). The crude product was carried forward for the next reaction without further purification.

### Step C: 5-Ethyl-1,2,3-trifluorobenzene:

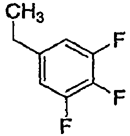

The above bromo compound (9.65 g, 40.4 mmol) was mixed with triethylsilane (41 mmol) and the reaction mixture was treated with solid PdCl₂ (177 mg, 4 mmol) in small portions. After a few minutes a vigorous exothermic reaction was ensued and care was taken to reflux the contents of the flask by placing a reflux condenser. The reaction mixture was stirred at room temperature for additional 6h and the contents were allowed to settle over 16h. Then the crude liquid product was decanted carefully and carried forward for the next reaction without further purification. It was assumed tat the reaction proceeds in quantitative yield.

### Step D: 1-Ethyl-3,4,5-trifluoro-2-nitrobenzene:

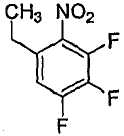

1,2,3-Trifluoro-5-methylbenzene (6.46 g, 40.4 mmol) was added to conc. H₂SO₄ (50 ml) at 0-5° C. Then the reaction mixture was slowly treated with conc. HNO₃ (3.39 ml, 48.44 mmol, 90%) while maintaining the internal temperature below 20° C. The reaction mixture was stirred at room temperature for 16h and poured onto ice (300 g) and the oily layer was extracted with CH₂Cl₂ (2x125 ml). The organic layer was washed with water (2x200 ml), brine (200 ml) and dried (MgSO₄) and evaporated to obtain the crude product which was purifed over flash silica gel chromatography to obtain the title product (6.6 g, 79%). ¹H NMR (CDCl₃): δ 6.98 (septet, 1H), 2.68 (q, 2H), 1.26 (t, *J* = 7.8 & 7.2 Hz, 3H).

### Step E: 3-Ethyl-5,6-difluoro-N-(2-fluoro-4-iodophenyl)-2nitroaniline:

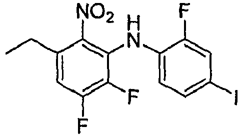

2-Fluoro-4-iodoaniline (2.05 g, 10 mmole) and 1-ethyl-3,4,5-trifluoro-2-nitrobenzene (2.37 g, 10 mmole) were reacted using the condition described in example 1 (Step A) to form the title compound (2.47 g, 60%); *m*/*z* = 407 [M-1]⁻.

### Step F: 3-Ethyl-5,6-difluoro-N1-(2-fluoro-4-iodophenyl)benzene-1,2-diamine:

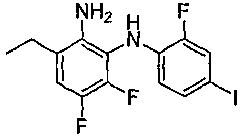

1,2,3-Trifluoro-5-methyl-4-nitrobenzene (2.47 g, 5.85 mmole) was reduced using the condition described in example 1 (Step B) to form the title compound. M-H⁺: 393

### Step-G: 1-Allyl-N-(6-ethyl-3,4-difluoro-2-(2-fluoro-4-iodophenylamino)phenyl)cyclopropane-1-sulfonamide:

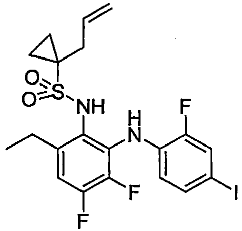

According to the general procedure B, 1-allyl-cyclopropanesulfonyl chloride (230 mg, 1.27 mmole) was reacted with 5,6-difluoro-N1-(2-fluoro-4-iodophenyl)-3-methylbenzene-1,2-diamine (100 mg, 0.255 mmole) to obtain the title product (72 mg, 53%); *m*/*z* = 535 [M-1]⁻.

### Step H: 1-(2,3-Dihydroxypropyl)-N-(6-ethyl-3,4-difluoro-2-(2-fluoro-4-iodophenylamino)phenyl)cyclopropane-1-sulfonamide:

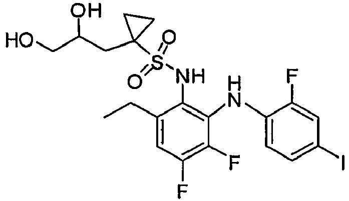

1-Allyl-N-(3,4-difluoro-2-(2-fluoro-4-iodophenylamino)-6-methylphenyl) cyclopropane-1-sulfonamide (70 mg, 0.13 mmole) and 4-methylmorpholine N-oxide (15 mg, 0.13 mmole) was dissolved in THF (2 mL). Osmium tetroxide was added at room temperature (0.013 mmole, 0.075 mL, 4% in H₂O) and the reaction mixture was stirred at room temperature for 16 hours. EtOAc was added, the organic phase was washed with water, dried (MgSO₄) and concentrated under reduced pressure. The residue was purified over silica gel chromatography (eluants: EtOAc/ MeOH) to obtain the titled product. ¹H NMR (300 MHz, CDCl₃): δ 7.38 (dd, *J* = 2.1 & 10.8 Hz, 1H), 7.27 (m, 2H), 7.12(br s, 1H), 6.91 (dd, *J* = 8.1 & 10.8 Hz, 1H), 6.69 (br s, 1H), 6.36 (dt, *J =* 4.8, 8.7 & 13.5 Hz, 1H), 4.00 (m, 1H), 3.62 (dd, *J* = 3.6 & 10.5 Hz, 1H), 3.47 (br m, 2H), 2.81 (q, 2H), 2.40 (dd, *J* = 10.2 & 15.9 Hz, 1H), 1.73 (br m, 2H), 1.58 (m, 1H), 1.43 (m, 1H), 0.94 (m, 2H).

### Example 42

### N-(3,4-difluoro-2-(2-fluoro-4-iodophenylamino)-6-(2-methoxyethoxy)phenyl)-1-(2,3-dihydroxypropyl)cyclopropane-1-sulfonamide:

### Step A: 1,2,3-Trifluoro-5-(2-methoxyethoxy)-4-nitrobenzene:

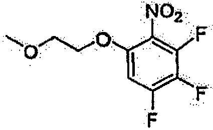

To a mixture of 3,4,5-trifluoro-2-nitrophenol (1.93, 10 mmol), Ph₃P (3.93 g, 15 mmol), and 2-methoxy-ethanol (1.18ml, 15 mmol) in anhydrous THF (25 ml) a solution of diisopropyl azodicarboxylate (2.91 ml, 15 mmol) in THF (5 ml) was added at 0° C and the reaction mixture was stirred at room temperature for 16h. The volatiles were evaporated and the residue was dissolved in CH₂Cl₂(100 ml) and the organic layer was washed with water (100 ml), brine (100 ml) dried (MgSO₄) and evaporated. The residue obtained was purified over flash silica gel chromatography to obtain the titled product in 68% (1.70 g) yield. ¹H NMR (300 MHz, CDCl₃): δ 6.78 (ddd, *J* = 2.4, 6.0, 11:7 Hz, 1H), 4.19 (t, *J* = 4.5 Hz, 2H), 3.72 (t, *J =* 4.5 Hz, 2H), 3.39 (s, 3H).

### Step B: 2,3-Difluoro-N-(2-fluoro-4-iodophenyl)-5-(2-methoxyethoxy)-6-nitroaniline:

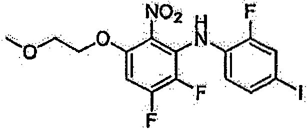

2-Fluoro-4-iodoaniline (1.6 g, 6.8 mmole) and 1,2,3-trifluoro-5-(2-methoxyethoxy)-4-nitrobenzene (1.7 g, 6.8 mmole) were reacted using the condition described in Example 1 (Step A) to form the title compound (1.02 g, 32 %); *mlz* = 467 [M-1]⁻.

### Step C: 5,6-Difluoro-N1-(2-fluoro-4-iodophenyl)-3-(2-methoxyethoxy)benzene-1,2-diamine:

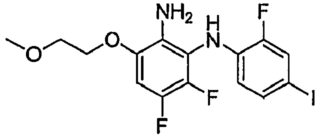

2,3-Difluoro-N-(2-fluoro-4-iodophenyl)-5-(2-methoxyethoxy)-6-nitroaniline (1.017 g, 2.17 mmole) was reduced using the condition described in Example 1 (Step B) to form the title compound; *mlz* = 337 [M-1]⁻.

### Step D: 1-Allyl-N-(3,4-difluoro-2-(2-fluoro-4-iodophenylaminol-6-(2-methoxyethoxy)phenyl)cyclopropane-1-sulfonamide:

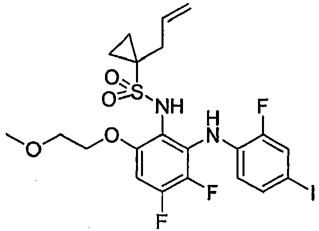

According to the general procedure B, 1-allyl-cyclopropanesulfonyl chloride (450 mg, 2.5 mmole) was reacted with 5,6-difluoro-N1-(2-fluoro-4-iodophenyl)-3-(2-methoxyethoxy)benzene-1,2-diamine (219 mg, 2.5 mmole) to obtain the title product (230 mg, 78%); *mlz* = 581 [M-1]⁻..

### Step E: N-(3,4-difluoro-2-(2-fluoro-4-iodophenylamino)-6-(2-methoxyethoxy)phenyl)-1-(2,3-dihydroxypropyl)cyclopropane-1-sulfonamide:

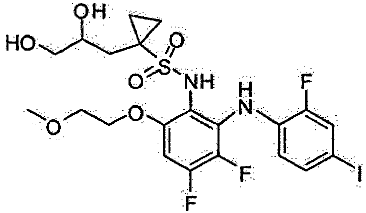

1-allyl-N-(3,4-difluoro-2-(2-fluoro-4-iodophenylamino)-6-(2-methoxyethoxy) phenyl) cyclopropane-1-sulfonamide (230 mg, 0.395 mmole) and 4-methylmorpholine-N-oxide (46 mg, 0.395 mmole) was dissolved in THF) (2 mL). Osmium tetroxide was added at room temperature (0.039 mmole, 0.25 mL, 4% in H₂O) and the reaction mixture was stirred at room temperature for 16 hours. EtOAc was added, the organic phase was washed with water, dried (MgSO₄) and concentrated under reduced pressure. The residue was purified over silica gel chromatography (eluants: EtOAc/ MeOH) to obtain the titled product. ¹H NMR (300 MHz, CDCl₃): δ 7.36 (dd, *J* =1.8 & 10.5 Hz, 1H), 7.27 (m, 2H), 6.56 (dd, *J* = 6.9 & 11.4 Hz, 1H), 6.40 (dt, *J* = 5.7, 7.5 & 12.9 Hz, 1H), 4.17 (m, 2H), 4.01 (m, 1H), 3.78 (m, 2H), 3.60 (dd, *J* = 3.6 & 11.1 Hz, 1H), 3.47 (m, 1H), 3.45 (s, 3H), 2.36 (dd, J= 9.6 & 15.9 Hz, 1H), 1.78 (dd, *J* = 2.4 & 15.6 Hz, 1H), 1.45-1.25 (m, 2H), 0.89 (m, 2H).

### Example 43

### N-(3,4-difluoro-2-(2-fluoro-4-methylphenylamino)phenyl)cyclopropanesulfonamide;

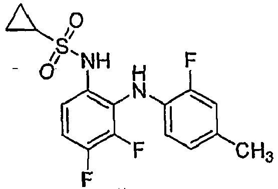

General procedure D : ¹H-NMR (500 MHz, CDCl₃): δ = 7.38-7.36 (m, 1H), 7.06-7.03 (q, 1H), 6.92-6.90 (1H), 6.73-6.72 (d, 1H), 6.63 (s, 1H, br), 6.37-6.33 (t, 1H), 5.54 (s, 1H, br), 2.42-2.39 (m, 1H), 2.25 (s, 3H), 1.14-1.11 (m, 2H), 0.94-0.90 (m, 2H); *mlz* = 355 [M-1]⁻.

Where racemic mixtures of chiral compounds have been resolved into separate enantiomers, the phrase "substantially free" of the epimer, as used herein, means an enantiomeric excess of at least 90%.

### Biological activity

### Generation of IC50 Data

Materials and preparation of reagents: Human GST-MEK1 and the constitutively active allele GST-MEK1^{CA} (harboring the mutations Ser218Asp and Ser222Asp) were subcloned into the yeast expression vector pGEM4Z (Promega, Madison, WI) from the wild type human MEK1 cDNA. GST-MEK1^{CA} was expressed in *Escherichia coli* and partially purified using Glutathione Sepharose 4B affinity resin (Amersham Pharmacia Biotech, Piscataway, NJ). The ERK2 allele was subcloned from MAPK2/Erk2 cDNA (wild type) in pUSEamp (Upstate Biotechnology, Inc., Waltham, MA) into the vector pET21a (Novagen, Madison, WI) resulting in an N-terminal histidine-tagged mouse ERK2 allele. ERK2 was expressed and purified to homogeneity [Zhang, 1993 #33]. Myelin basic protein (MBP) was purchased from Gibco BRL (Rockville, MD). EasyTides adenosine 5-triphosphate (ATP) ([γ-³³P]) (NEN Perkin Elmer, Wellesley, MA) was the source of radiolabel for all kinase reactions. Activated Raf-1 (truncated) and activated MAPKinase 2/ERK2 were purchased from Upstate, Inc. (Lake Placid, NY). 4-20% Criterion Precast gels were purchased from Bio-Rad (Hercules, CA).

Determination of enzymatic activity: Compounds were diluted from dimethylsulfoxide (DMSO) stocks into 1xHMNDE (20 mM HEPES pH 7.2, 1 mM MgCl₂, 100 mM NaCl, 1.25 mM DTT, 0.2 mM EDTA). A typical 25-microliter assay contained 0.002 nanomoles MEK1^{CA}, 0.02 nanomoles ERK2, 0.25 nanomoles MBP, 0.25 nanomoles unlabeled ATP, and 0.1 µCi [γ³³P] ATP. The screening assay essentially comprised four additions. Five µl of diluted compound were dispensed to 96-well assay plates. Ten µl of 2.5x enzyme cocktail (MEK1^{CA} and ERK2 only) were then added to each well followed by a preincubation for 30 minutes at ambient temperature. Ten µl of 2.5x substrate cocktail (labeled and unlabeled ATP plus MBP) were then added, followed by incubation for 60 minutes at ambient temperature. Finally, 100 µl of 10% trichloroacetic acid (TCA) were added and incubated for 30 minutes at room temperature to halt the reaction and precipitate radiolabeled protein products. Reaction products were harvested on glass fiber 96 well filter plates prewetted with water and 1% pyrophosphate. The filter plate was then washed 5 times with water. Water was displaced by absolute ethanol and the plate was allowed to air dry for 30 minutes at room temperature. A back seal was applied manually and 40 µl of scintillation cocktail were dispensed to each well. A top seal was applied and the plate was counted in the TopCount for two seconds per well.

For certain experiments a truncated version of MEK that requires activation by Raf kinase were used.

### Generation of EC50 Data

Effects of compounds in the cell were determined by Western blotting for phosphorylated ERK. MDA-MB-231 breast cancer cells were plated in a 48 well plate at 20,000 cells per well and grown in a 37° humidified CO₂ incubator. The following day, the growth media (DMEM + 10% fetal bovine serum) was removed and replaced with starve media (DMEM + 0.1% fetal bovine serum). Cells were incubated in the starve media for sixteen hours and then treated with a range of compound concentrations for thirty minutes. After incubation with compound, cells were stimulated with 100ng/ml EGF for five minutes. The cells were then lysed and analyzed by Western blot using a monoclonal antibody raised to phosphorylated ERK. The signal was amplified using a secondary antibody conjugated to a near -IR dye and detected on a Licor Odyssey scanner. The intensity of signal was quantitated and this data was used to generate dose response curves and EC50 calculations.

Legend: A, ECso = < 2.0nM; B, EC₅₀ = 2.0-15nM; C, EC₅₀ = 15nM-100nM;

| **Compound Number** | **Structure** | **ACTIVITY µM** |
|---|---|---|
| 1000 | 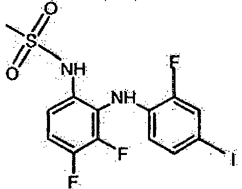 | A |
| 1001 | 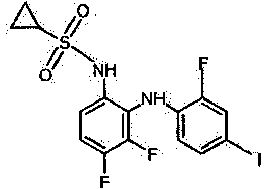 | A |
| 1002 | 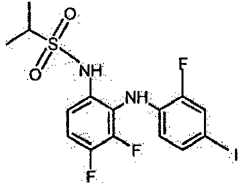 | B |
| 1003 | 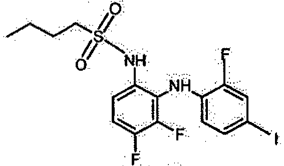 | C |
| 1004 | 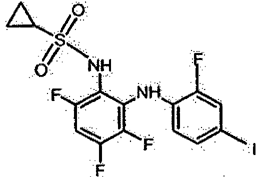 | C |
| 1007 | 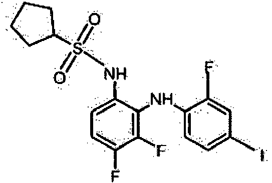 | C |
| 1008 | 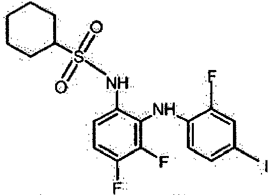 | C |
| 1009 | 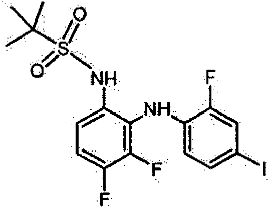 | C |
| 1010 | 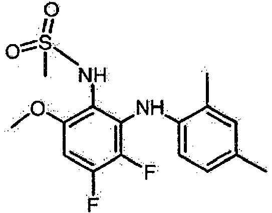 | A |
| 1012 | 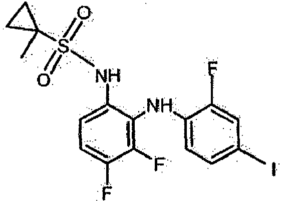 | B |
| 1013 | 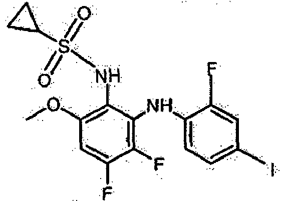 | B |
| 1017 | 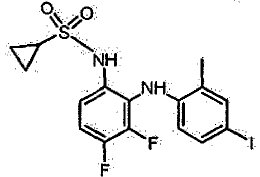 | B |
| 1018 (Racemic) | 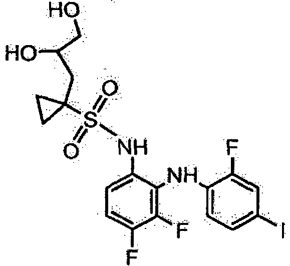 | A |
| 1019 (Racemic) | 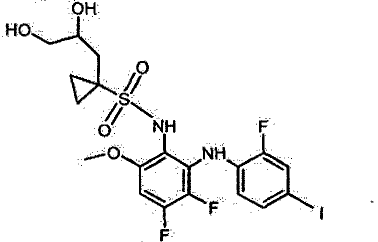 | A |
| 1020 (Racemic) | 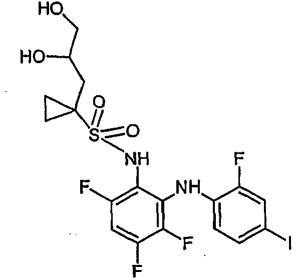 | A |
| 1021 (R isomer) | 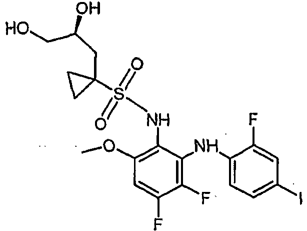 | A |
| 1022 (S isomer) | 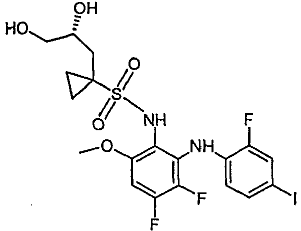 | B |
| 1023 (R isomer) | 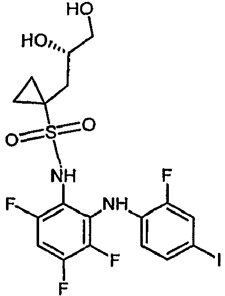 | B |
| 1024 (S isomer) | 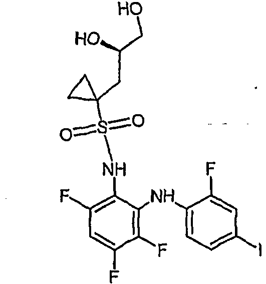 | B |
| 1025 | 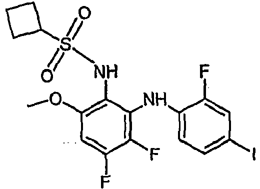 | B |
| 1026 | 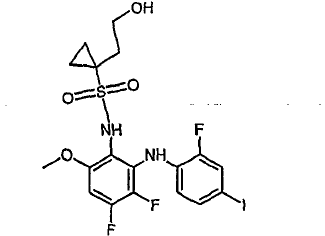 | A |
| 1027 | 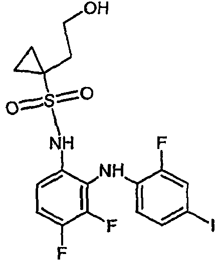 | A |
| 1028 | 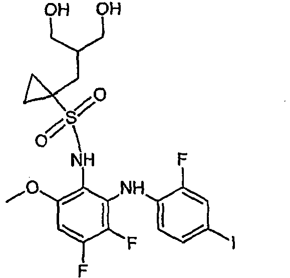 | A |
| 1029 | 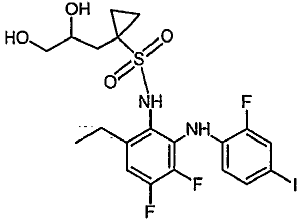 | C |
| 1030 | 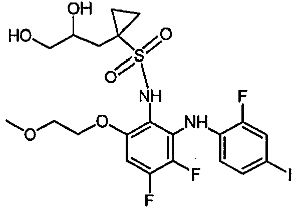 | C |
| 1031 | 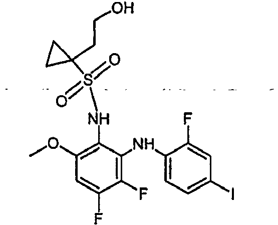 | A |

## Claims

1. A compound of formula I 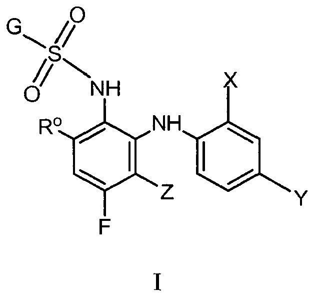 where G is R₁ₐ, R_{1b}, R_{1c}, R_{1d}, or R₁ₑ; R° is H, halogen, CH₃NH-, (CH₃)₂N-, C₁-C₆ alkyl, C₁-C₄ alkoxy, C₃-C₆ cycloalkyl, C₂-C₆ alkenyl, C₂-C₆ alkynyl, said alkyl, alkoxy, cycloalkyl, alkenyl, and alkynyl groups optionally substituted with 1-3 substituents selected independently from halogen, OH, CN, cyanomethyl, nitro, phenyl, and trifluoromethyl, and said C₁-C₆ alkyl and C₁-C₄ alkoxy groups also optionally substituted with OCH₃ or OCH₂CH₃; X is F, Cl, or methyl; Y is I, Br, Cl, CF₃, C₁-C₃ alkyl, C₂-C₃ alkenyl, C₂-C₃ alkynyl, cyclopropyl, phenyl, pyridyl, pyrazolyl, OMe, OEt, or SMe, where all said methyl, ethyl, C₁-C₃ alkyl, and cyclopropyl groups of X and Y are optionally substituted with OH, where all said phenyl, pyridyl, pyrazolyl groups of Y are optionally substituted with halogen, acetyl, methyl, and trifluoromethyl, and where all said methyl groups of X and Y are optionally substituted with one, two, or three F atoms; and Z is H or F;
where R₁ₐ is methyl, optionally substituted with 1-3 fluorine atoms or 1-3 chlorine atoms, or with OH, cyclopropoxy, or C₁- C₄ alkoxy, where said cyclopropoxy group or the C₁- C₄ alkyl moieties of said C₁-C₄ alkoxy groups are optionally substituted with one hydroxy or methoxy group, and where all C₂-C₄ alkyl groups within said C₁- C₄ alkoxy are optionally further substituted with a second OH group;
R_{1b} is CH(CH₃)-C₁₋₃ alkyl or C₃-C₆ cycloalkyl, said methyl, alkyl and cycloalkyl groups optionally substituted with 1-3 substituents selected independently from F, Cl, Br, I, OH, OCH₃, and CN;
R_{1c} is (CH₂)ₙOₘR', where m is 0 or 1; where, when m is 1, n is 2 or 3, and when m is 0, n is 1 or 2; and where R' is C₁-C₆ alkyl, optionally substituted with 1-3 substituents selected independently from F, Cl, OH, OCH₃, OCH₂CH₃, and C₃-C₆ cycloalkyl ;
R_{1d} is C(A)(A')(B)- where B, A, and A' are, independently, H or C₁₋₄ alkyl, optionally substituted with one or two OH groups or halogen atoms, or A and A', together with the carbon atom to which they are attached, form a 3- to 6- member saturated ring, said ring optionally containing one or two heteroatoms selected, independently, from O, N, and S and optionally substituted with one or two groups selected independently from methyl, ethyl, and halo; and
R₁ₑ is benzyl or 2-phenyl ethyl, in which the phenyl group is optionally substituted, as shown below : 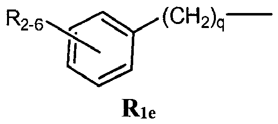 where q is 1 or 2, R₂, R₃ and R₄ are, independently, H, F, Cl, Br, CH₃, CH₂F, CHF₂, CF₃, OCH₃, OCH₂F, OCHF₂, OCF₃, ethyl, *n*-propyl, isopropyl, cyclopropyl, isobutyl, *sec*-butyl, *tert-butyl,* and methylsulfonyl, and R₄ may also be nitro, acetamido, amidinyl, cyano, carbamoyl, methylcarbamoyl, dimethylcarbamoyl, 1,3,4-oxadiazol-2-yl, 5-methyl-1,3,4-oxadiazol, 1,3,4-thiadiazol, 5-methyl-1,3,4-thiadiazol 1H-tetrazolyl, N-morpholyl carbonyl amino, N-morpholylsulfonyl and N-pyrrolidinylcarbonylamino; R₅ and R₆ are independently H, F, Cl or methyl.

2. The compound of claim 1, where X is F, Cl, or CH₃: Y is I, Br, Cl, CF₃, or C₁-C₃ alkyl; and Z is H or F.

3. The compound of claim 1, where R⁰ is F, Cl, C₁-C₄ alkyl or C₁-C₄ alkoxy, said C₁-C₄ alkyl group and the C₁-C₄ alkyl moiety of said C₁-C₄ alkoxy group optionally substituted with F, Cl, OCH₃, or OCH₂CH₃.

4. The compound of claim 2, where R⁰ is H, F, Cl, C₁-C₄ alkyl, methoxy, ethoxy, or 2-methoxy-ethoxy.

5. The compound of claim 4 where G is R₁ₐ and Z is F.

6. The compound of claim 5, where G is CH₃; R° is H; and Y is Br, I, CF₃, or CH₃.

7. The compound of any of claims 1 to 4, where G is R_{1b}.

8. The compound of claim 4 where G is R_{1b} and Z is F.

9. The compound of claim 8, where R° is H, F, or OCH₃, X is F or CH₃, and Y is Br, I, or CH₃.

10. The compound of claim 9, where G is unsubstituted C₃-C₆ cycloalkyl.

11. The compound of claim 10, where R⁰ is H.

12. The compound of claim 9, where G is isopropyl or cyclopropyl.

13. The compound of any of claims 1 to 4, where G is R_{1c}.

14. The compound of claim 4, where G is R_{1c}, Y is I, Br, CH₃, or CF₃; and Z is F.

15. The compound of claim 14, where m is zero.

16. The compound of any of claims 1 to 4, where G is R_{1d}.

17. The compound of claim 16, where R⁰ is fluoro, chloro, methyl, ethyl, propyl, isopropyl, sec-butyl, iso-butyl, tert-butyl, cyclopropyl, cyclobutyl, fluoromethyl, methoxy, fluoromethoxy, methylamino or dimethylamino; X is F, Cl, CH₃, or mono-, di- or trifluoromethyl; Y is I, Br, Cl, or mono-, di- or tri- fluoromethyl; and Z is H or F.

18. The compound of claim 16, where R⁰ is F, Cl, methyl, ethyl, methoxy, ethoxy, or 2-methoxy-ethoxy; X is F, Cl, or CH₃; Y is I, Br, Cl, or mono-, di- or tri- fluoromethyl; and Z is H or F.

19. The compound of claim 16, where R⁰ is H; X is F, Cl, CH₃, or mono-, di- or trifluoromethyl; Y is I, Br, Cl, or mono-, di- or tri- fluoromethyl; and Z is H or F.

20. The compound of any of claims 17-19, where C(A)(A') is C₁-C₆ cycloalkyl.

21. The compound of claim 20, where B is H.

22. The compound of claim 21, where C(A)(A') is cyclopropyl.

23. The compound of claim 20, where B is methyl, optionally substituted with one OH group, or C₂-C₄ alkyl, optionally substituted with one or two OH groups.

24. The compound of claim 23, where C(A)(A') is cyclopropyl.

25. The compound of claim 24, where B is methyl, ethyl, 2-hydroxyethyl, *n*-propyl, 3-hydroxypropyl, 2,3-dihydroxypropyl, 3,4-dihydroxybutyl, isopropyl, 1-methyl-2-hydroxy ethyl, n-butyl, sec-butyl, isobutyl, or 2-hydroxymethyl-3-hydroxy propyl.

26. The compound of claim 25, where B is 2,3-dihydroxypropyl or 3,4-dihydroxybutyl.

27. The compound of claim 26, in which the chiral carbon in B is in the R configuration.

28. A composition comprising a compound according to claim 27, which is substantially free of the S isomer.

29. The compound of any of claims 1 to 4, where G is R₁ₑ

30. The compound of claim 29, where q is 1.

31. The compound of claim 30, where R⁰ is H, R₄₋₆ are H; R₂ and R₃ are, independently, H, F, Cl, Br, CH₃, CH₂F, CHF₂, CF₃, OCH₃, OCH₂F, OCHF₂, OCF₃, ethyl, *n*-propyl, isopropyl, cyclopropyl, isobutyl, *sec*-butyl, *tert*-butyl, and methylsulfonyl; X is F and Y is I.

32. According to claim 1 compound which is selected from the compounds below

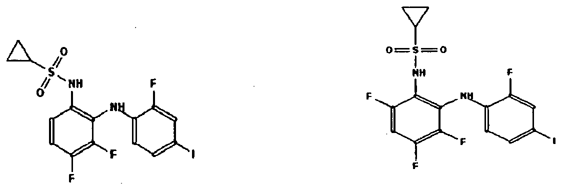

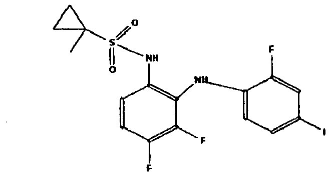

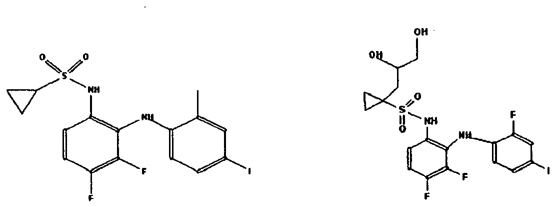

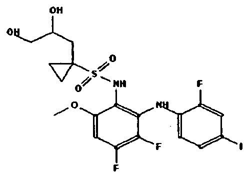

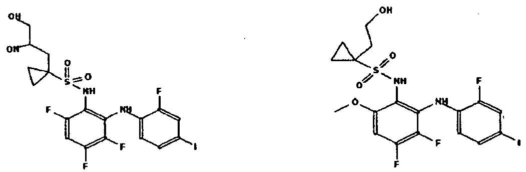

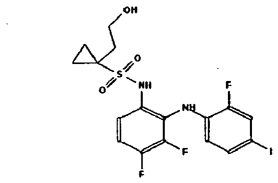

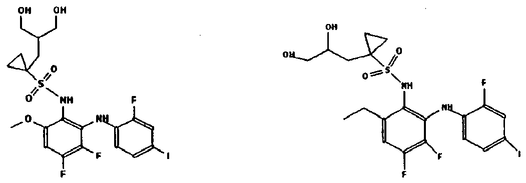

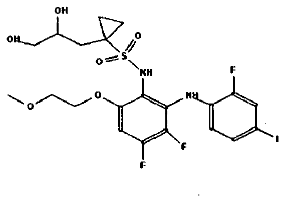

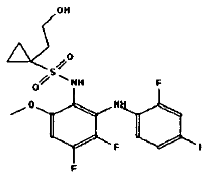

33. A compound according to claim 1 selected from the compounds below 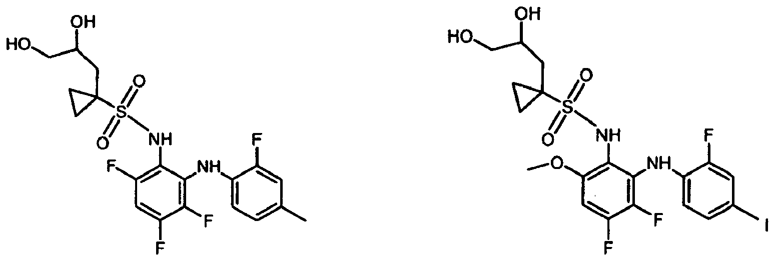 where the 2-OH carbon is in the R configuration.

34. A composition comprising the compound shown below, where the 2-OH carbon is in the R configuration, substantially free of the S-isomer.

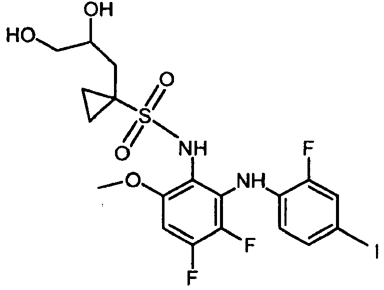

35. A compound according to claim 1 selected from those shown below 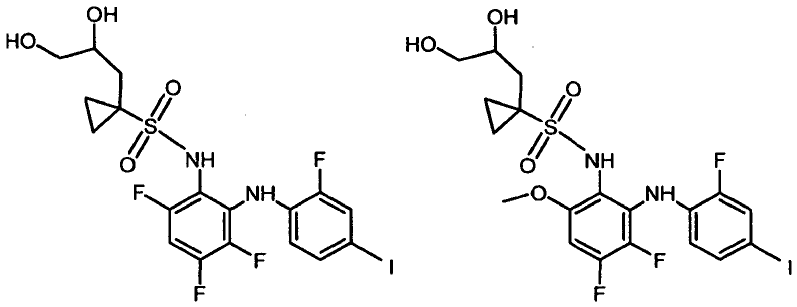 where the compound is the dextrorotatory configuration, substantially free of the levorotatory isomer.

36. (S)-N-(3,4-difluoro-2-(2-fluoro-4-iodophenylamino)-6-methoxyphenyl)-1-(2,3-dihydroxypropyl)cyclopropane-1-sulfonamide.

37. A pharmaceutical composition comprising a pharmaceutically effective amount of a compound of formula I according to any one of claims 1 to 36, or a pharmaceutically acceptable salt, ester, or hydrate thereof, and a pharmaceutically acceptable carrier.

38. A pharmaceutical composition comprising a pharmaceutically effective amount of a compound selected from those below, or a pharmaceutically acceptable salt, ester, or hydrate thereof, and a pharmaceutically acceptable carrier.

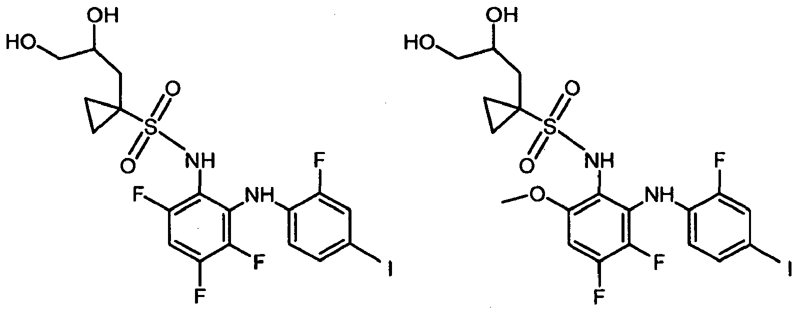

39. The composition of claim 38, where the compound is in the R or S configuration.

40. Use of a compound of formula I according to any one of claims 1 to 36, or a pharmaceutically acceptable salt, ester or hydrate thereof, for the manufacture of a medicament for treating a hypoproliferative disorder in a mammal, including a human.

41. Use of a compound of formula I according to any one of claims 1 to 36, or a pharmaceutically acceptable salt, ester or hydrate thereof, for the manufacture of a medicament for treating a disorder or condition which is modulated by the MEK cascade.

## Patentansprüche

1. Verbindung der Formel I 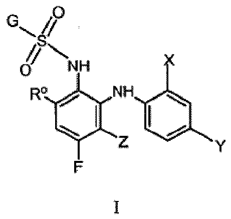 wobei G für R₁ₐ, R_{1b}, R_{1c}, R_{1d} oder R₁ₑ steht; R⁰ für H, Halogen, CH₃NH-, (CH₃)₂N-, C₁-C₆-Alkyl, C₁-C₄-Alkoxy, C₃-C₆-Cycloalkyl, C₂-C₆-Alkenyl, C₂-C₆-Alkinyl steht, wobei die Alkyl-, Alkoxy-, Cycloalkyl-, Alkenyl- und Alkinylgruppen gegebenenfalls durch 1-3 Substituenten, die unabhängig voneinander aus Halogen, OH, CN, Cyanomethyl, Nitro, Phenyl und Trifluormethyl ausgewählt sind, substituiert sind und die C₁-C₆-Alkyl- und C₁-C₄-Alkoxygruppen auch gegebenenfalls durch OCH₃ oder OCH₂CH₃ substituiert sind; X für F, C1 oder Methyl steht; Y für I, Br, Cl, CF₃, C₁-C₃-Alkyl, C₂-C₃-Alkenyl, C₂-C₃-Alkinyl, Cyclopropyl, Phenyl, Pyridyl, Pyrazolyl, OMe, OEt oder SMe steht, wobei alle Methyl-, Ethyl-, C₁-C₃-Alkyl- und Cyclopropylgruppen von X und Y gegebenenfalls durch OH substituiert sind, wobei alle Phenyl-, Pyridyl-, Pyrazolylgruppen von Y gegebenenfalls durch Halogen, Acetyl, Methyl und Trifluormethyl substituiert sind und wobei alle Methylgruppen von X und Y gegebenenfalls durch ein, zwei oder drei F-Atome substituiert sind; und Z für H oder F steht;
wobei R₁ₐ für Methyl, das gegebenenfalls durch 1-3 Fluoratome oder 1-3 Chloratome oder durch OH, Cyclopropoxy oder C₁-C₄-Alkoxy substituiert ist, steht, wobei die Cyclopropoxygruppe oder die C₁-C₄-Alkylteile der C₁-C₄-Alkoxygruppen gegebenenfalls durch eine Hydroxy- oder Methoxygruppe substituiert sind und wobei alle C₂-C₄-Alkylgruppen in dem C₁-C₄-Alkoxy gegebenenfalls ferner durch eine zweite OH-Gruppe substituiert sind;
R_{1b} für CH (CH₃) -C₁₋₃-Alkyl oder C₃-C₆-Cycloalkyl steht, wobei die Methyl-, Alkyl- und Cycloalkylgruppen gegebenenfalls durch 1-3 Substituenten, die unabhängig aus F, Cl, Br, I, OH, OCH₃ und CN ausgewählt sind, substituiert sind; R_{1c} für (CH₂)ₙOₘR' steht, wobei m für 0 oder 1 steht; wobei dann, wenn m für 1 steht, n für 2 oder 3 steht, und dann, wenn m für 0 steht, n für 1 oder 2 steht; und wobei R' für C₁-C₆-Alkyl steht, das gegebenenfalls durch 1-3 Substituenten, die unabhängig voneinander aus F, Cl, OH, OCH₃, OCH₂CH₃ und C₃-C₆-Cycloalkyl ausgewählt sind, substituiert ist;
R_{1d} für C(A)(A')(B)- steht, wobei B, A und A' unabhängig für H oder C₁₋₄-Alkyl, das gegebenenfalls durch eine oder zwei OH-Gruppen oder Halogenatome substituiert ist, stehen oder A und A' zusammen mit dem Kohlenstoffatom, an das sie gebunden sind, einen 3- bis 6-gliedrigen gesättigten Ring bilden, wobei der Ring gegebenenfalls ein oder zwei unabhängig aus 0, N und S ausgewählte Heteroatome enthält und gegebenenfalls durch eine oder zwei Gruppen, die unabhängig aus Methyl, Ethyl und Halogen ausgewählt sind, substituiert ist; und
R₁ₑ für Benzyl oder 2-Phenylethyl steht, worin die Phenylgruppe gegebenenfalls substituiert ist, wie nachstehend gezeigt: 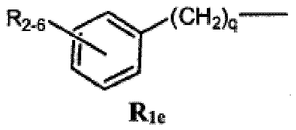 wobei q für 1 oder 2 steht, R₂, R₃ und R₄ unabhängig für H, F, Cl, Br, CH₃, CH₂F, CHF₂, CF₃, OCH₃, OCH₂F, OCHF₂, OCF₃, Ethyl, *n*-Propyl, Isopropyl, Cyclopropyl, Isobutyl, *sec*.-Butyl, *tert*.-Butyl und Methylsulfonyl stehen und R₄ auch für Nitro, Acetamido, Amidinyl, Cyano, Carbamoyl, Methylcarbamoyl, Dimethylcarbamoyl, 1,3,4-Oxadiazol-2-yl, 5-Methyl-1,3,4-oxadiazol, 1,3,4-Thiadiazol, 5-Methyl-1,3,4-thiadiazol, 1H-Tetrazolyl, N-Morpholylcarbonylamino, N-Morpholylsulfonyl und N-Pyrrolidinylcarbonylamino stehen kann; R₅ und R₆ unabhängig für H, F, Cl oder Methyl stehen.

2. Verbindung nach Anspruch 1, wobei X für F, Cl oder CH₃ steht; Y für I, Br, Cl, CF₃ oder C₁-C₃-Alkyl steht und Z für H oder F steht.

3. Verbindung nach Anspruch 1, wobei R⁰ für F, Cl, C₁-C₄-Alkyl oder C₁-C₄-Alkoxy steht, wobei die C₁-C₄-Alkylgruppe und der C₁-C₄-Alkylteil der C₁-C₄-Alkoxygruppe gegebenenfalls durch F, Cl, OCH₃ oder OCH₂CH₃ substituiert sind.

4. Verbindung nach Anspruch 2, wobei R⁰ für H, F, Cl, C₁-C₄-Alkyl, Methoxy, Ethoxy oder 2-Methoxyethoxy steht.

5. Verbindung nach Anspruch 4, wobei G für R₁ₐ steht und Z für F steht.

6. Verbindung nach Anspruch 5, wobei G für CH₃ steht; R⁰ für H steht und Y für Br, I, CF₃ oder CH₃ steht.

7. Verbindung nach einem der Ansprüche 1 bis 4, wobei G für R_{1b} steht.

8. Verbindung nach Anspruch 4, wobei G für R_{1b} steht und Z für F steht.

9. Verbindung nach Anspruch 8, wobei R⁰ für H, F oder OCH₃ steht, X für F oder CH₃ steht und Y für Br, I oder CH₃ steht.

10. Verbindung nach Anspruch 9, wobei G für unsubstituiertes C₃-C₆-Cycloalkyl steht.

11. Verbindung nach Anspruch 10, wobei R⁰ für H steht.

12. Verbindung nach Anspruch 9, wobei G für Isopropyl oder Cyclopropyl steht.

13. Verbindung nach einem der Ansprüche 1 bis 4, wobei G für R_{1c} steht.

14. Verbindung nach Anspruch 4, wobei G für R_{1c} steht, Y für I, Br, CH₃ oder CF₃ steht und Z für F steht.

15. Verbindung nach Anspruch 14, wobei m für null steht.

16. Verbindung nach einem der Ansprüche 1 bis 4, wobei G für R_{1d} steht.

17. Verbindung nach Anspruch 16, wobei R⁰ für Fluor, Chlor, Methyl, Ethyl, Propyl, Isopropyl, sec.-Butyl, Isobutyl, tert.-Butyl, Cyclopropyl, Cyclobutyl, Fluormethyl, Methoxy, Fluormethoxy, Methylamino oder Dimethylamino steht; X für F, Cl, CH₃ oder Mono-, Di- oder Trifluormethyl steht; Y für I, Br, Cl oder Mono-, Di- oder Trifluormethyl steht und Z für H oder F steht.

18. Verbindung nach Anspruch 16, wobei R⁰ für F, Cl, Methyl, Ethyl, Methoxy, Ethoxy oder 2-Methoxyethoxy steht; X für F, Cl oder CH₃ steht; Y für I, Br, Cl oder Mono-, Di- oder Trifluormethyl steht und Z für H oder F steht.

19. Verbindung nach Anspruch 16, wobei R⁰ für H steht; X für F, Cl, CH₃ oder Mono-, Di- oder Trifluormethyl steht; Y für I, Br, Cl oder Mono-, Di- oder Trifluormethyl steht und Z für H oder F steht.

20. Verbindung nach einem der Ansprüche 17-19, wobei C(A)(A') für C₁-C₆-Cycloalkyl steht.

21. Verbindung nach Anspruch 20, wobei B für H steht.

22. Verbindung nach Anspruch 21, wobei C(A)(A') für Cyclopropyl steht.

23. Verbindung nach Anspruch 20, wobei B für Methyl, das gegebenenfalls durch eine OH-Gruppe substituiert ist, oder C₂-C₄-Alkyl, das gegebenenfalls durch eine oder zwei OH-Gruppen substituiert ist, steht.

24. Verbindung nach Anspruch 23, wobei C(A)(A') für Cyclopropyl steht.

25. Verbindung nach Anspruch 24, wobei B für Methyl, Ethyl, 2-Hydroxyethyl, *n*-Propyl, 3-Hydroxypropyl, 2,3-Dihydroxypropyl, 3,4-Dihydroxybutyl, Isopropyl, 1-Methyl-2-hydroxyethyl, n-Butyl, sec.-Butyl, Isobutyl oder 2-Hydroxymethyl-3-hydroxypropyl steht.

26. Verbindung nach Anspruch 25, wobei B für 2,3-Dihydroxypropyl oder 3,4-Dihydroxybutyl steht.

27. Verbindung nach Anspruch 26, wobei der chirale Kohlenstoff in B in der R-Konfiguration vorliegt.

28. Zusammensetzung, umfassend eine Verbindung nach Anspruch 27, die weitgehend frei von dem S-Isomer ist.

29. Verbindung nach einem der Ansprüche 1 bis 4, wobei G für R₁ₑ steht.

30. Verbindung nach Anspruch 29, wobei q für 1 steht.

31. Verbindung nach Anspruch 30, wobei R⁰ für H steht, R₄₋₆ für H stehen; R₂ und R₃ unabhängig für H, F, Cl, Br, CH₃, CH₂F, CHF₂, CF₃, OCH₃, OCH₂F, OCHF₂, OCF₃, Ethyl, *n*-Propyl, Isopropyl, Cyclopropyl, Isobutyl, *sec*.-Butyl, *tert*.-Butyl und Methylsulfonyl stehen; X für F steht und Y für I steht.

32. Verbindung nach Anspruch 1, die aus den nachstehenden Verbindungen ausgewählt ist:

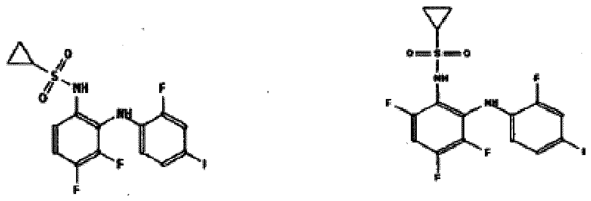

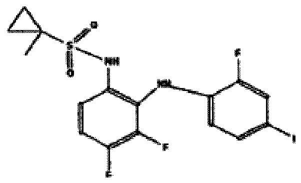

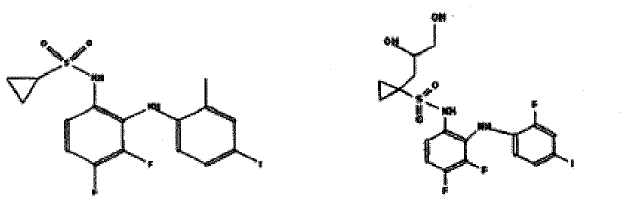

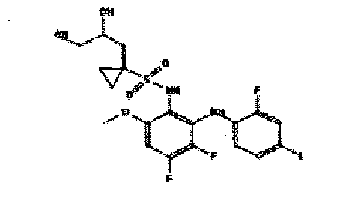

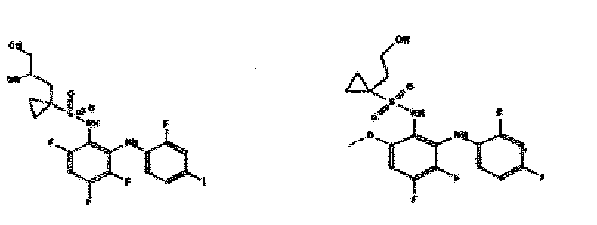

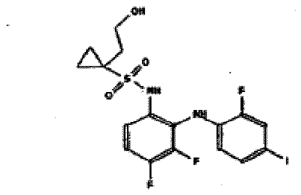

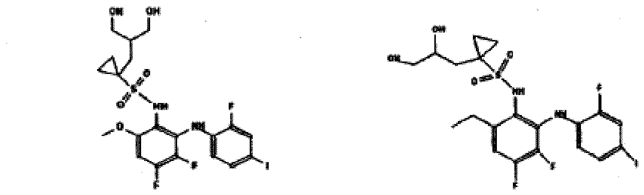

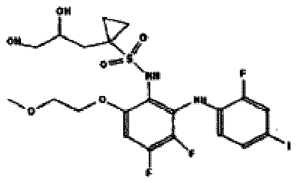

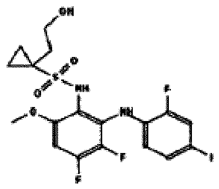

33. Verbindung nach Anspruch 1, die aus den nachstehenden Verbindungen ausgewählt ist: 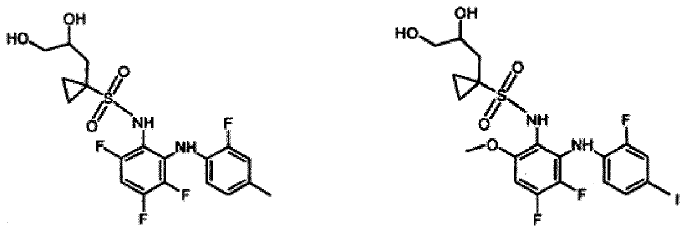 wobei der 2-OH-Kohlenstoff in der R-Konfiguration vorliegt.

34. Zusammensetzung, umfassend die nachstehend gezeigte Verbindung, wobei der 2-OH-Kohlenstoff in der R-Konfiguration vorliegt, die weitgehend frei von dem S-Isomer ist.

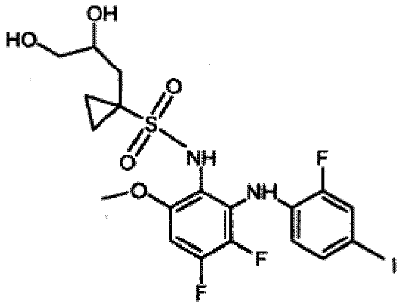

35. Verbindung nach Anspruch 1, die aus den nachstehenden Verbindungen ausgewählt ist: 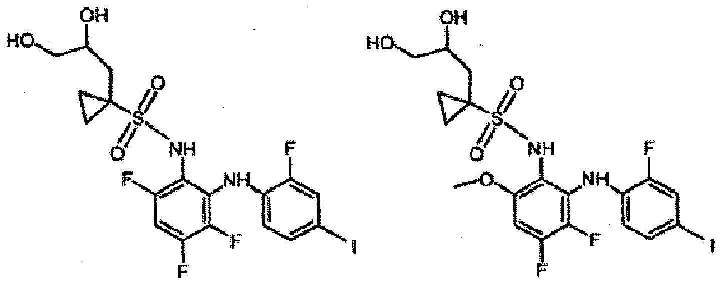 wobei die Verbindung in der rechtsdrehenden Konfiguration vorliegt und weitgehend frei von dem linksdrehenden Isomer ist.

36. (S)-N-(3,4-Difluor-2-(2-fluor-4-iodphenylamino)-6-methoxyphenyl)-1-(2,3-dihydroxypropyl)cyclopropan-1-sulfonamid.

37. Pharmazeutische Zusammensetzung, umfassend eine pharmazeutisch wirksame Menge einer Verbindung der Formel I nach einem der Ansprüche 1 bis 36 oder ein pharmazeutisch unbedenkliches Salz, einen pharmazeutisch unbedenklichen Ester oder ein pharmazeutisch unbedenkliches Hydrat davon und einen pharmazeutisch unbedenklichen Träger.

38. Pharmazeutische Zusammensetzung, umfassend eine pharmazeutisch wirksame Menge einer Verbindung, die aus den nachstehenden Verbindungen ausgewählt ist, oder ein pharmazeutisch unbedenkliches Salz, einen pharmazeutisch unbedenklichen Ester oder ein pharmazeutisch unbedenkliches Hydrat davon und einen pharmazeutisch unbedenklichen Träger.

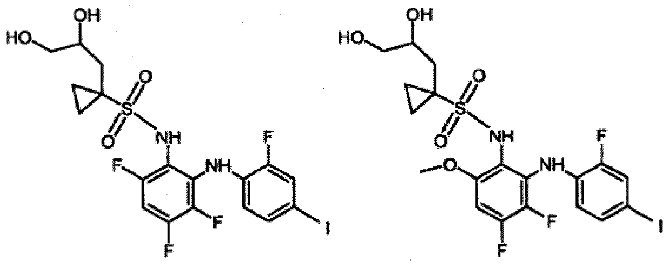

39. Zusammensetzung nach Anspruch 38, wobei die Verbindung in der R- oder S-Konfiguration vorliegt.

40. Verwendung einer Verbindung der Formel I nach einem der Ansprüche 1 bis 36 oder eines pharmazeutisch unbedenklichen Salzes, Esters oder Hydrats davon zur Herstellung eines Medikaments zur Behandlung einer hypoproliferativen Störung bei einem Säugetier einschließlich des Menschen.

41. Verwendung einer Verbindung der Formel I nach einem der Ansprüche 1 bis 36 oder eines pharmazeutisch unbedenklichen Salzes, Esters oder Hydrats davon zur Herstellung eines Medikaments zur Behandlung einer Störung oder eines Leidens, die bzw. das durch die MEK-Kaskade moduliert wird.

## Revendications

1. Composé de formule I 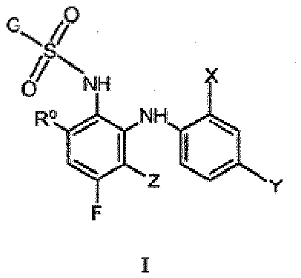 où G représente R₁ₐ, R_{1b}, R_{1c}, R_{1d} ou R₁ₑ ; R⁰ représente l'atome H, un atome d'halogène, le groupe CH₃NH-, (CH₃)₂N-, C₁-C₆-alkyle, C₁-C₄-alcoxy, C₃-C₆-cycloalkyle, C₂-C₆-alcényle, C₂-C₆-alcynyle, lesdits groupes alkyle, alcoxy, cycloalkyle, alcényle et alcynyle étant éventuellement substitués avec 1-3 substituants choisis indépendamment parmi le groupe composé d'un atome d'halogène, du groupe OH, CN, cyanométhyle, nitro, phényle et trifluorométhyle, et lesdits groupes C₁-C₆-alkyle et C₁-C₄-alcoxy étant également éventuellement substitués avec un groupe OCH₃ ou un groupe OCH₂CH₃ ; X représente l'atome F, Cl ou un groupe méthyle ; Y représente l'atome I, Br, Cl, le groupe CF₃, C₁-C₃-alkyle, C₂-C₃-alcényle, C₂-C₃-alcynyle, cyclopropyle, phényle, pyridyle, pyrazolyle, OMe, OEt ou SMe, où tous les groupes méthyle, éthyle, C₁-C₃-alkyle et cyclopropyle de X et de Y éventuellement substitués avec le groupe OH, où tous lesdits groupes phényle, pyridyle, pyrazolyle de Y étant éventuellement substitués avec un atome d'halogène, un groupe acétyle, méthyle et trifluorométhyle, et où tous lesdits groupes méthyle de X et de Y étant éventuellement substitués avec un, deux ou trois atomes F ; et Z représente l'atome H ou F ;
où R₁ₐ représente un groupe méthyle, éventuellement substitué avec 1-3 atomes de fluor ou avec 1-3 atomes de chlore, ou avec le groupe OH, cyclopropoxy ou C₁-C₄-alcoxy, où ledit groupe cyclopropoxy ou le motif C₁-C₄-alkyle desdits groupes C₁-C₄-alcoxy sont éventuellement substitués avec un groupe hydroxy ou un groupe méthoxy, et ou tous les groupes C₂-C₄-alkyle dans lesdits groupes C₁-C₄-alcoxy sont éventuellement en outre substitués avec un deuxième groupe OH ;
R_{1b} représente le groupe CH (CH₃) -C₁₋₃₋alkyle ou C₃-C₆ cycloalkyle, lesdits groupes méthyle, alkyle et cycloalkyle étant éventuellement substitués avec 1-3 substituants choisis indépendamment parmi le groupe composé de l'atome F, Cl, Br, I, du groupe OH, OCH₃ et CN;
R_{1c} représente le groupe (CH₂)ₙOₘR', m valant 0 ou 1 ; où, lorsque m vaut 1, n vaut 2 ou 3, et lorsque m vaut 0, n vaut 1 ou 2 ; et R' représentant le groupe C₁-C₆-alkyle, éventuellement substitué avec 1-3 substituants choisis indépendamment parmi le groupe composé de l'atome F, Cl, du groupe OH, OCH₃, OCH₂CH₃ et C₃-C₆-cycloalkyle ;
R_{1d} représente le groupe C(A)(A')(B)- où B, A, et A' représentent, indépendamment, l'atome H ou le groupe C₁₄-alkyle, éventuellement substitué avec un ou deux groupes OH ou atomes d'halogène, ou A et A', ensemble avec l'atome de carbone auquel ils sont liés, forment un cycle saturé de 3 à 6 chaînons, ledit cycle contenant éventuellement un ou deux hétéroatomes choisis indépendamment parmi le groupe composé de l'atome 0, N et S et éventuellement substitués avec un ou deux groupes choisis indépendamment parmi le groupe composé du groupe méthyle, éthyle et halogéno ; et
R₁ₑ représente le groupe benzyle ou le groupe 2-phényléthyle, dans lequel le groupe phényle est éventuellement substitué, comme indiqué ci-dessous : 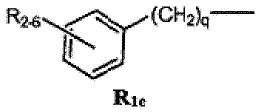 où q vaut 1 ou 2, R₂, R₃ et R₄ représentent, indépendamment, l'atome H, F, Cl, Br, le groupe CH₃, CH₂F, CHF₂, CF₃ , OCH₃, OCH₂F, OCHF₂, OCF₃, éthyle, *n-*propyle, isopropyle, cyclopropyle, isobutyle, *sec-*butyle, *tert*-butyle et méthylsulfonyle, et R₄ peut également représenter le groupe nitro, acétamido, amidinyle, cyano, carbamoyle, méthylcarbamoyle, diméthylcarbamoyle, 1,3,4-oxadiazol-2-yle, 5-méthyl-1,3,4-oxadiazole, 1,3,4-thiadiazole, 5-méthyl-l,3,4-thiadiazole-1H-tétrazolyle, N-morpholylcarbonylamino, N-morpholylsulfonyle et N-pyrrolidinylcarbonylamino ; R₅ et R₆ représentent, indépendamment, l'atome H, F, Cl ou le groupe méthyle.

2. Composé selon la revendication 1, où X représente l'atome F, Cl ou le groupe CH₃ ; Y représente l'atome I, Br, Cl, le groupe CF₃ ou C₁-C₃-alkyle ; et Z représente l'atome H ou F.

3. Composé selon la revendication 1, où R⁰ représente l'atome F, Cl, le groupe C₁-C₄-alkyle ou C₁-C₄-alcoxy, ledit groupe C₁-C₄-alkyle et le motif C₁-C₄-alkyle dudit groupe C₁-C₄-alcoxy éventuellement substitués avec l'atome F, Cl, le groupe OCH₃ ou OCH₂CH₃.

4. Composé selon la revendication 2, où R⁰ représente l'atome H, F, Cl, le groupe C₁-C₄-alkyle, méthoxy, éthoxy ou 2-méthoxy-éthoxy.

5. Composé selon la revendication 4 où G représente le groupe R₁ₐ et Z représente l'atome F.

6. Composé selon la revendication 5, où G représente le groupe CH₃; R⁰ représente l'atome H ; et Y représente l'atome Br, I, le groupe CF₃ ou CH₃.

7. Composé selon l'une quelconque des revendications 1 à 4, où G représente le groupe R_{1b}.

8. Composé selon la revendication 4, où G représente le groupe R_{1b} et Z représente l'atome F.

9. Composé selon la revendication 8, où R⁰ représente l'atome H, F ou le groupe OCH₃, X représente l'atome F ou le groupe CH₃, et Y représente l'atome Br, I ou le groupe CH₃.

10. Composé selon la revendication 9, où G représente le groupe C₃-C₆-cycloalkyle non substitué.

11. Composé selon la revendication 10, où R⁰ représente l'atome H.

12. Composé selon la revendication 9, où G représente le groupe isopropyle ou cyclopropyle.

13. Composé selon l'une quelconque des revendications 1 à 4, où G représente le groupe R_{1c}.

14. Composé selon la revendication 4, où G représente le groupe R_{1c}, Y représente l'atome I, Br, le groupe CH₃ ou CF₃ ; et Z représente l'atome F.

15. Composé selon la revendication 14, où m vaut zéro.

16. Composé selon l'une quelconque des revendications 1 à 4, où G représente le groupe R_{1d}.

17. Composé selon la revendication 16, où R⁰ représente l'atome de fluoro, de chloro, le groupe méthyle, éthyle, propyle, isopropyle, sec-butyle, iso-butyle, tert-butyle, cyclopropyle, cyclobutyle, fluorométhyle, méthoxy, fluorométhoxy, méthylamino ou diméthylamino ; X représente l'atome F, Cl, le groupe CH₃ ou mono-, di-ou tri- fluorométhyle ; Y représente l'atome I, Br, Cl ou le groupe mono-, di- ou tri- fluorométhyle ; et Z représente l'atome H ou F.

18. Composé selon la revendication 16, où R⁰ représente l'atome F, Cl, le groupe méthyle, éthyle, méthoxy, éthoxy ou 2-méthoxy-éthoxy ; X représente l'atome F, Cl ou le groupe CH₃ ; Y représente l'atome I, Br, Cl ou le groupe mono-, di- ou tri- fluorométhyle ; et Z représente l'atome H ou F.

19. Composé selon la revendication 16, où R⁰ représente l'atome H ; X représente l'atome F, Cl, le groupe CH₃ ou mono-, di- ou tri- fluorométhyle ; Y représente l'atome I, Br, Cl ou le groupe mono-, di- ou tri-fluorométhyle ; et Z représente l'atome H ou F.

20. Composé selon l'une quelconque des revendications 17 à 19, où C(A)(A') représente le groupe C₁-C₆-cycloalkyle.

21. Composé selon la revendication 20, où B représente l'atome H.

22. Composé selon la revendication 21, où C(A)(A') représente le groupe cyclopropyle.

23. Composé selon la revendication 20, où B représente le groupe méthyle, éventuellement substitué avec un groupe OH, ou C₂-C₄-alkyle, éventuellement substitué avec un ou deux groupes OH.

24. Composé selon la revendication 23, où C(A)(A') représente le groupe cyclopropyle.

25. Composé selon la revendication 24, où B représente le groupe méthyle, éthyle, 2-hydroxyéthyle, *n*-propyle, 3-hydroxypropyle, 2,3-dihydroxypropyle, 3,4-dihydroxybutyle, isopropyle, 1-méthyl-2-hydroxyéthyle, n-butyle, sec-butyle, isobutyle ou 2-hydroxyméthyl-3-hydroxypropyle.

26. Composé selon la revendication 25, où B représente le 2,3-dihydroxypropyle ou le 3,4-dihydroxybutyle.

27. Composé selon la revendication 26, dans lequel le carbone chiral de B est de configuration R.

28. Composition comprenant un composé selon la revendication 27, qui est essentiellement exempt de l'isomère S.

29. Composé selon l'une quelconque des revendications 1 à 4, où G représente le groupe R₁ₑ.

30. Composé selon la revendication 29, où q vaut 1.

31. Composé selon la revendication 30, où R⁰ représente l'atome H, R₄₋₆ représentent l'atome H ; R₂ et R₃ représentent, indépendamment, l'atome H, F, Cl, Br, le groupe CH₃, CH₂F, CHF₂, CF₃, OCH₃, OCH₂F, OCHF₂, OCF₃, éthyle, *n*-propyle, isopropyle, cyclopropyle, isobutyle, *sec*-butyle, *tert*-butyle et méthylsulfonyle ; X représente l'atome F et Y représente l'atome I.

32. Composé selon la revendication 1 qui est choisi parmi les composés ci-dessous

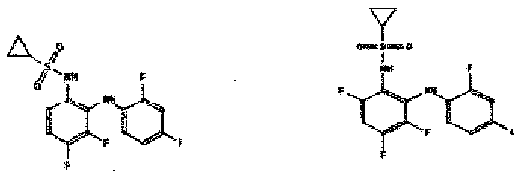

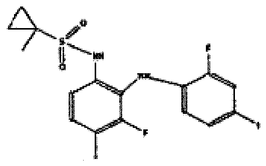

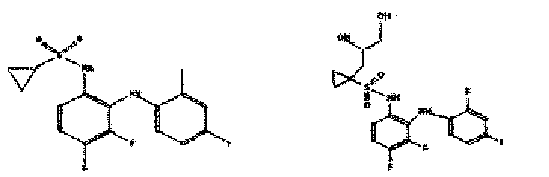

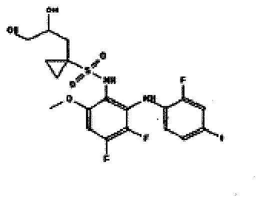

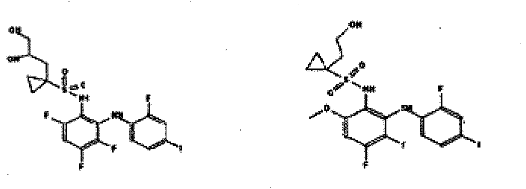

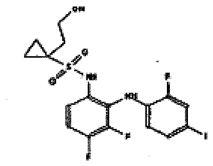

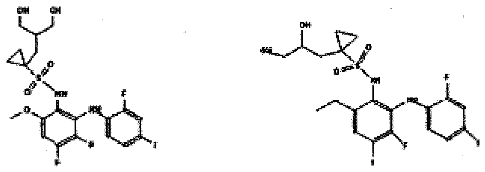

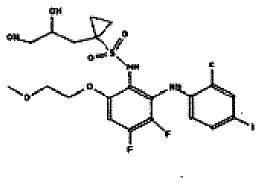

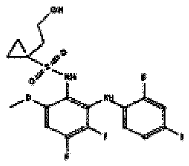

33. Composé selon la revendication 1 qui est choisi parmi les composés décrits ci-dessous 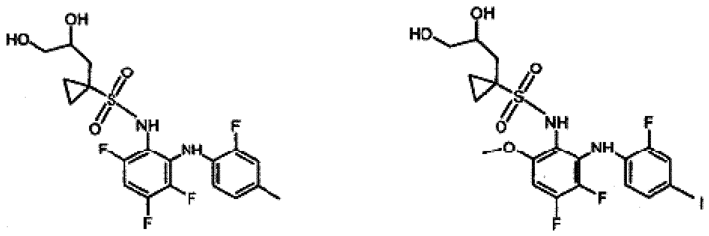 où le carbone 2 porteur du groupement OH est de configuration R.

34. Composition comprenant le composé décrit ci-dessous, où le carbone 2 porteur du groupement OH est de configuration R, essentiellement exempt de l'isomère S.

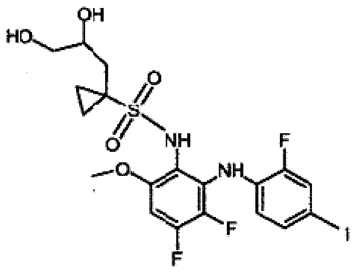

35. Composé selon la revendication 1 qui est choisi parmi les composés représentés ci-dessous 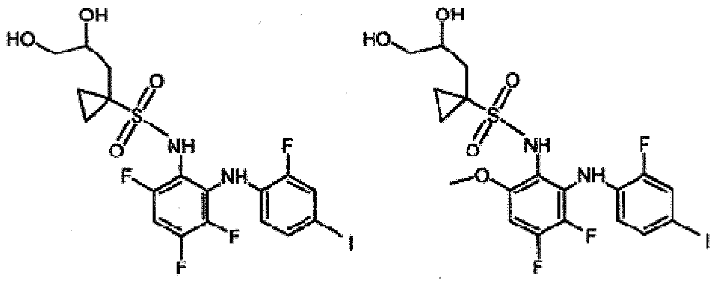 où le composé est de configuration dextrogyre, essentiellement exempt de l'isomère lévogyre.

36. (S)-N-(3,4-difluoro-2-(2-fluoro-4-iodophénylamino)-6-méthoxyphényl)-1-(2,3-dihydroxypropyl)cyclopropane-1-sulfonamide.

37. Composition pharmaceutique comprenant une quantité pharmaceutiquement efficace d'un composé de formule I selon l'une quelconque des revendications 1 à 36, ou d'un sel, d'un ester ou d'un hydrate pharmaceutiquement acceptable de celui-ci, et un support pharmaceutiquement acceptable.

38. Composition pharmaceutique comprenant une quantité pharmaceutiquement efficace d'un composé de formule I choisi parmi les composés décrits ci-dessous, ou d'un sel, d'un ester ou d'un hydrate pharmaceutiquement acceptable de celui-ci, et un support pharmaceutiquement acceptable.

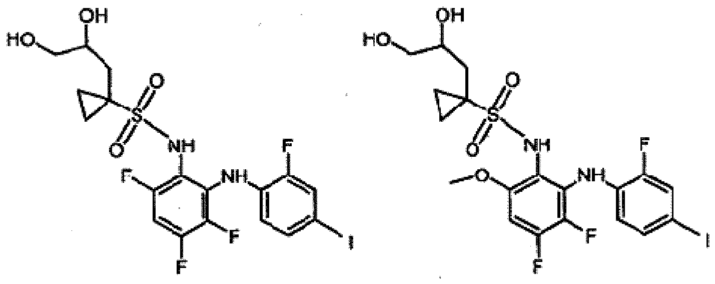

39. Composition selon la revendication 38, où le composé est de configuration R ou S.

40. Utilisation d'un composé de formule I selon l'une quelconque des revendications 1 à 36, ou d'un sel, d'un ester ou d'un hydrate pharmaceutiquement acceptable de celui-ci, pour préparer un médicament pour le traitement d'un trouble d'hypoprolifération chez les mammifères, y compris chez l'homme.

41. Utilisation d'un composé de formule I selon l'une quelconque des revendications 1 à 36, ou d'un sel, d'un ester ou d'un hydrate pharmaceutiquement acceptable de celui-ci, pour préparer un médicament pour le traitement d'un trouble ou d'un état pathologique qui est modulé par la cascade MEK.
